# EUROPEAN PATENT SPECIFICATION

(11) **EP 0 992 585 B1**
(45) Date of publication and mention of the grant of the patent: **12.01.2005**
(21) Application number: 99119092.7
(22) Date of filing: 14.08.1992
(51) Int. Cl.: C12N 15/12, C12N 15/70, C12N 5/10, G01N 33/68, C07K 14/00, C12Q 1/68

(54) **Human calcium channel compositions and methods**
Menschliche Calcium-Kanale und Verwendungen davon
Composition à canaux calciques humains et applications

(30) Priority: 15.08.1991 US 745206; 10.04.1992 US 868354
(43) Date of publication of application: 12.04.2000
(62) Divisional of application: 92918611.2
(73) Proprietor: Merck & Co., Inc., White House Station, New Jersey 08889 (US)
(72) Inventor: Harpold, Michael M., Santa FE, New Mexico 87505 (US); Ellis, Steven B., San Diego, California 92129 (US); Williams, Mark E., Carlsbad, California 92009 (US); McCue, Ann F., La Mesa, California 91942 (US); Feldman, Daniel H., Gainesville, Florida 32607 (US); Brenner, Robert, Austin, Texas 78703 (US)
(74) Representative: Horgan, James Michael Frederic

(56) References cited:
- WO-A-89/09834
- EDWARD PEREZ-REYES ET AL.: "Molecular diversity of L-type Calcium channels. Evidence for alternative splicing of the transcripts of three non-allelic genes" JOURNAL OF BIOLOGICAL CHEMISTRY, vol. 265, no. 33, 25 November 1990 (1990-11-25), pages 20430-20436, XP000653496 MD US
- ANNA HUI ET AL.: "Molecular cloning of multiple subtypes of a novel rat brain isoform of the alpha1 subunit of the voltage-dependent Calcium channel" NEURON, vol. 7, July 1991 (1991-07), pages 35-44, XP000653439
- MARTIN BIEL ET AL.: "Primary structure and functional expression of a high voltage activated calcium channel from rabbit lung" FEBS LETTERS, vol. 269, no. 2, 3 September 1990 (1990-09-03), pages 409-412, XP002147829 AMSTERDAM NL
- YASUO MORI ET AL.: "Primary structure and functional expression from complementary DNA of a brain Calcium channel" NATURE, vol. 350, no. 6317, 4 April 1991 (1991-04-04), pages 398-402, XP000652130 LONDON GB -& DATABASE EMBL [Online] Accession number X57688, 8 May 1991 (1991-05-08) XP002147832
- TERRY V.B. STARR ET AL.: "Primary structure of a Calcium channel that is highly expressed in the rat cerebellum" PROCEEDINGS OF THE NATIONAL ACADEMY OF SCIENCES OF USA, vol. 88, no. 13, 1 July 1991 (1991-07-01), pages 5621-5625, XP002147830 WASHINGTON US -& DATABASE EMBL [Online] Accession number M64373, 21 August 1991 (1991-08-21) XP002147833
- MARK E. WILLIAMS ET AL.: "Structure and functional expression of alpha1, alpha2, and beta subunits of a novel human neuronal Calcium channel subtype" NEURON, vol. 8, January 1992 (1992-01), pages 71-84, XP000886416
- SUSUMO SEIMO ET AL.: "Cloning of the alpha1 subunit of a voltage-dependent Calcium channel expressed in pancreatic beta cells" PROCEEDINGS OF THE NATIONAL ACADEMY OF SCIENCES OF USA, vol. 89, no. 2, 15 January 1992 (1992-01-15), pages 584-588, XP002142249 WASHINGTON US
- NIKOLAI M. SOLDATOV: "Molecular diversity of the L-type Ca2+ channel transcripts in human fibroblasts" PROCEEDINGS OF THE NATIONAL ACADEMY OF SCIENCES OF USA, vol. 89, no. 10, 15 May 1992 (1992-05-15), pages 4628-4632, XP002147831 WASHINGTON US
- MARK E. WILLIAMS: "Structure and functional expression of an omega-conotoxin-sensitive human N-type Calcium channel" SCIENCE, vol. 257, 17 July 1992 (1992-07-17), pages 389-394, XP002056227 LANCASTER, PA US

## Description

### TECHNICAL FIELD

The present invention relates to molecular biology and pharmacology. More particularly, the invention relates to calcium channel compositions and methods of making and using the same.

### BACKGROUND OF THE INVENTION

Calcium channels are membrane-spanning, multi-subunit proteins that allow controlled entry of Ca²⁺ ions into cells from the extracellular fluid. Cells throughout the animal kingdom, and at least some bacterial, fungal and plant cells, possess one or more types of calcium channel.

The most common type of calcium channel is voltage dependent. "Opening" of a voltage-dependent channel to allow an influx of Ca²⁺ ions into the cells requires a depolarization to a certain level of the potential difference between the inside of the cell bearing the channel and the extracellular medium bathing the cell. The rate of influx of Ca²⁺ into the cell depends on this potential difference. All "excitable" cells in animals, such as neurons of the central nervous system (CNS), peripheral nerve cells and muscle cells, including those of skeletal muscles, cardiac muscles, and venous and arterial smooth muscles, have voltage-dependent calcium channels. Multiple types of calcium channels have been identified in mammalian cells from various tissues, including skeletal muscle, cardiac muscle, lung, smooth muscle and brain, [see, *e.g*., Bean, B.P.(1989) *Ann. Rev*. *Physiol*. *51*:367-384 and Hess, P. (1990) *Ann. Rev*. *Neurosci*. 56:337). The different types of calcium channels have been broadly categorized into four classes, L-, T-, N-, and P-type, distinguished by current kinetics, holding potential sensitivity and sensitivity to calcium channel agonists and antagonists.

Calcium channels are multisubunit proteins. For example, rabbit skeletal muscle calcium channel contains two large subunits, designated α₁ and α₂, which have molecular weights between about 130 and about 200 kilodaltons ("kD"), and one to three different smaller subunits of less than about 60 kD in molecular weight. At least one of the larger subunits and possibly some of the smaller subunits are glycosylated. Some of the subunits are capable of being phosphorylated. The a, subunit has a molecular weight of about 150 to about 170 kD when analyzed by sodium dodecylsulfate (SDS)-polyacrylamide gel electrophoresis (PAGE) after isolation from mammalian muscle tissue and has specific binding sites for various 1,4-dihydropyridines (DHPs) and phenylalkylamines. Under non-reducing conditions (in the presence of N-ethylmaleimide), the α₂ subunit migrates in SDS-PAGE as a band corresponding to a molecular weight of about 160-190 kD. Upon reduction, a large fragment and smaller fragments are released. The β subunit of the rabbit skeletal muscle calcium channel is a phosphorylated protein that has a molecular weight of 52-65 kD as determined by SDS-PAGE analysis. This subunit is insensitive to reducing conditions. The γ subunit of the calcium channel, which is not observed in all purified preparations, appears to be a glycoprotein with an apparent molecular weight of 30-33 kD, as determined by SDS-PAGE analysis.

In order to study calcium channel structure and function, large amounts of pure channel protein are needed. Because of the complex nature of these multisubunit proteins, the varying concentrations of calcium channels in tissue sources of the protein, the presence of mixed populations of calcium channels in tissues, difficulties in obtaining tissues of interest, and the modifications of the native protein that can occur during the isolation procedure, it is extremely difficult to obtain large amounts of highly purified, completely intact calcium channel protein.

Characterization of a particular type of calcium channel by analysis of whole cells is severely restricted by the presence of mixed populations of different types of calcium channels in the majority of cells. Single-channel recording methods that are used to examine individual calcium channels do not reveal any information regarding the molecular structure or biochemical composition of the channel. Furthermore, in performing this type of analysis, the channel is isolated from other cellular constituents that might be important for natural functions and pharmacological interactions.

Characterization of the gene or genes encoding calcium channels provides another means of characterization of different types of calcium channels. The amino acid sequence determined from a complete nucleotide sequence of the coding region of a gene encoding a calcium channel protein represents the primary structure of the protein. Furthermore, secondary structure of the calcium channel protein and the relationship of the protein to the membrane may be predicted based on analysis of the primary structure. For instance, hydropathy plots of the a, subunit protein of the rabbit skeletal muscle calcium channel indicate that it contains four internal repeats, each containing six putative transmembrane regions [Tanabe, T. *et al*. (1987) *Nature 328*:313].

The cDNA and corresponding amino acid sequences of the α₁, α₂, β and γ subunits of the rabbit skeletal muscle calcium channel [see, Tanabe *et al.* (1987) *Nature 328*:313-318; International Application No. WO 89/09834, which is U.S. Application Serial No. 07/603,751, which is a continuation-in-part of U.S. Application Serial No. 07/176,899; Ruth *et al.* (1989) *Science 245*:1115-1118; and U.S. Patent Application Serial No. 482,384, filed February 20, 1990] have been determined. The cDNA and corresponding amino acid sequences of a, subunits of rabbit cardiac muscle [Mikami, A. *et al*. (1989) *Nature 340*:230-233] and lung [Biel, M. (1990) *FEBS Letters 269*:409-412] calcium channels have been determined.

In addition, a cDNA clone encoding a rabbit brain calcium channel (designated the BI channel) has been isolated [Mori, Y. et *al.* (1991) *Nature 350*:398-402]. Partial cDNA clones encoding portions of several different subtypes, referred to as rat brain class A, B, C and D, of the calcium channel α₁ subunit have been isolated from rat brain cDNA libraries [Snutch, T. *et al*. (1990) *Proc. Natl. Acad. Sci. USA 87*:3391-3395]. More recently full-length rat brain class A [Starr, T. *et al*. (1991) *Proc. Natl*. *Acad. Sci. USA 88*:5621-5625] and class C (Snutch, T. *et al*. (1991) *Neuron 7*:45-57] cDNA clones have been isolated. Although the amino acid sequence encoded by the rat brain class C DNA is approximately 95% identical to that encoded by the rabbit cardiac muscle calcium channel a, subunit-encoding DNA, the amino acid sequence encoded by the rat brain class A DNA shares only 33% sequence identity with the amino acid sequence encoded by the rabbit skeletal or cardiac muscle α₁ subunit-encoding DNA. A cDNA clone encoding another rat brain calcium channel α₁ subunit has also been obtained [Hui, A. *et al*. (1991*) Neuron 7*:35-44]. The amino acid sequence encoded by this clone is ~70% homologous to the proteins encoded by the rabbit skeletal and cardiac muscle calcium channel DNA. A cDNA clone closely related to the rat brain class C α₁ subunit-encoding cDNA and sequences of partial cDNA clones closely related to other partial cDNA clones encoding apparently different calcium channel a, subunits have also been isolated [see Snutch, T. *et al*. (1991) *Neuron 7*:45-57; Perez-Reyes, E. *et al.* (1990) *J. Biol. Chem. 265*:20430; and Hui, A. *et al.* (1991) *Neuron 7*:35-44]. DNA clones encoding other calcium channels have also been identified and isolated.

Expression of cDNA encoding calcium channel subunits has been achieved with several of the different rabbit or rat α₁ subunit cDNA clones discussed above. Voltage-dependent calcium currents have been detected in murine L cells transfected with DNA encoding the rabbit skeletal muscle calcium channel α₁ subunit [Perez-Reyes et *al.* (1989) *Nature 340*:233-236 (1989)]. These currents were enhanced in the presence of the calcium channel agonist Bay K 8644. Bay K 8644-sensitive Ba²⁺ currents have been detected in oöcytes injected with *in vitro* transcripts of the rabbit cardiac muscle calcium channel α₁ subunit cDNA [Mikami, A. *et al.* (1989) *Nature 340*:230-233]. These currents were substantially reduced in the presence of the calcium channel antagonist nifedipine. Barium currents of an oöcyte co-injected with RNA encoding the rabbit cardiac muscle calcium channel α₁ subunit and the RNA encoding the rabbit skeletal muscle calcium channel α₂ subunit were more than 2-fold larger than those of oöcytes injected with transcripts of the rabbit cardiac calcium channel α₁ subunit-encoding cDNA. Similar results were obtained when oöcytes were co-injected with RNA encoding the rabbit lung calcium channel α₁ subunit and the rabbit skeletal muscle calcium channel α₂ subunit. The barium current was greater than that detected in oöcytes injected only with RNA encoding the rabbit lung calcium channel a, subunit [Biel, M. *et al.* (1990) *FEBS Letters 269*:409-412]. Inward barium currents have been detected in oöcytes injected with *in vitro* RNA transcripts encoding the rabbit brain BI channel [Mori *et al*. (1991) *Nature 350*:398-402]. These currents were increased by two orders of magnitude when *in vitro* transcripts of the rabbit skeletal muscle calcium channel α₂, β, or α₂, β and γ subunits were co-injected with transcripts of the BI-encoding cDNA. Barium currents in oöcytes co-injected with transcripts encoding the BI channel and the rabbit skeletal muscle calcium channel α₂ and β were unaffected by the calcium channel antagonists nifedipine or ω-CgTx and inhibited by Bay K 8644 and crude venom from *Agelenopsis aperta*.

The results of studies of recombinant expression of rabbit calcium channel α₁ subunit-encoding cDNA clones and transcripts of the cDNA clones indicate that the α₁ subunit forms the pore through which calcium enters cells. The relevance of the barium currents generated in these recombinant cells to the actual current generated by calcium channels containing as one component the respective α₁ subunits *in vivo* is unclear. In order to completely and accurately characterize and evaluate different calcium channel types, however, it is essential to examine the functional properties of recombinant channels containing all of the subunits as found *in vivo*. Although there has been limited success in expressing DNA encoding rabbit and rat calcium channel subunits, far less has been achieved with respect to human calcium channels. Little is known about human calcium channel structure and function and gene expression. An understanding of the structure and function of human calcium channels would permit identification of substances that, in some manner, modulate the activity of calcium channels and that have potential for use in treating such disorders.

Because calcium channels are present in various tissues and have a central role in regulating intracellular calcium ion concentrations, they are implicated in a number of vital processes in animals, including neurotransmitter release, muscle contraction, pacemaker activity, and secretion of hormones and other substances. These processes appear to be involved in numerous human disorders, such as CNS and cardiovascular diseases. Calcium channels, thus, are also implicated in numerous disorders. A number of compounds useful for treating various cardiovascular diseases in animals, including humans, are thought to exert their beneficial effects by modulating functions of voltage-dependent calcium channels present in cardiac and/or vascular smooth muscle. Many of these compounds bind to calcium channels and block, or reduce the rate of, influx of ca²⁺ into the cells in response to depolarization of the cell membrane.

An understanding of the pharmacology of compounds that interact with calcium channels in other organ systems, such as the CNS, may aid in the rational design of compounds that specifically interact with subtypes of human calcium channels to have desired therapeutic effects, such as in the treatment of neurodegenerative and cardiovascular disorders. Such understanding and the ability to rationally design therapeutically effective compounds, however, have been hampered by an inability to independently determine the types of human calcium channels and the molecular nature of individual subtypes, particularly in the CNS, and by the unavailability of pure preparations of specific channel subtypes to use for evaluation of the specificity of calcium channel-effecting compounds. Thus, identification of DNA encoding human calcium channel subunits and the use of such DNA for expression of calcium channel subunits and functional calcium channels would aid in screening and designing therapeutically effective compounds.

Therefore, it is an object herein, to provide DNA encoding specific calcium channel subunits and to provide eukaryotic cells bearing recombinant tissue-specific or subtype- specific calcium channels. It is also an object to provide assays for identification of potentially therapeutic compounds that act as calcium channel antagonists and agonists.

### SUMMARY OF THE INVENTION

Eukaryotic cells containing heterologous DNA encoding one or more calcium channel α, β subunits, particularly human calcium channel subunits, or containing RNA transcripts of DNA clones encoding one or more of the subunits are provided. The cells contain DNA or RNA encoding a human α_{1β} subunit. In more preferred embodiments, the cells contain DNA or RNA encoding additional heterologous subunits, including at least one β, α₂ or γ subunits are included. In such embodiments, eukaryotic cells stably or transiently transfected with any combination of one, two, three or four of the subunit-encoding cDNA clones, such as α₁, α₁, + β, α₁ + β + α₁, are provided. In more preferred embodiments, the subunits encoded by the heterologous DNA are human subunits.

In preferred embodiments, the cells express such heterologous calcium channel subunits and include one or more of the subunits in membrane spanning heterologous calcium channels. In more preferred embodiments, the eukaryotic cells express functional, heterologous calcium channels that are capable of gating the passage of calcium channel selective ions and/or binding compounds that, at physiological concentrations, modulate the activity of the heterologous calcium channel. In certain embodiments, the heterologous calcium channels include at least one heterologous calcium channel subunit. In most preferred embodiments, the calcium channels that are expressed on the surface of the eukaryotic cells are composed substantially or entirely of subunits encoded by the heterologous DNA or RNA. In preferred embodiments, the heterologous calcium channels of such cells are distinguishable from any endogenous calcium channels of the host cell.

In certain embodiments the recombinant eukaryotic cells that contain the heterologous DNA encoding the calcium channel subunits are produced by transfection with DNA encoding one or more of the subunits or are injected with RNA transcripts of cDNA encoding one or more of the calcium channel subunits. The DNA may be introduced as a linear DNA fragment or may be included in an expression vector for stable or transient expression of the subunit-encoding DNA. Vectors containing DNA encoding human calcium channel subunits are also provided.

The eukaryotic cells that express heterologous calcium channels may be used in assays for calcium channel function or, in the case of cells transformed with fewer subunitencoding nucleic acids than necessary to constitute a functional recombinant human calcium channel, such cells may be used to assess the effects of additional subunits on calcium channel activity. The additional subunits can be provided by subsequently transfecting such a cell with one or more DNA clones or RNA transcripts encoding human calcium channel subunits.

The recombinant eukaryotic cells that express membrane spanning heterologous calcium channels may be used in methods for identifying compounds that modulate calcium channel activity. In particular, the cells are used in assays that identify agonists and antagonists of calcium channel activity in humans and/or assessing the contribution of the various calcium channel subunits to the transport and regulation of transport of calcium ions.

Assays using the eukaryotic cells for identifying compounds that modulate calcium channel activity are provided.

Although the present invention relates to the α_{1β} subunits identified in the claims, the following disclosure details other subunits which may be incorporated with the α_{1β} subunit to form functional heterologous calcium channels which are a feature of the present invention, together with uses of such calcium channels.

Isolated and purified DNA fragments that encode human calcium channel subunits are described. DNA encoding α₁ subunits of a human calcium channel, and RNA, encoding such subunits, made upon transcription of such DNA are described. In particular, DNA fragments encoding α₁ subunits of voltage-dependent human calcium channels (VDCCs) type A, type B (also referred to as VDCC IV), type C (also referred to as VDCC II) and type D (also referred to as VDCC III) are described.

In particular, DNA encoding an α_{1D} subunit that includes the amino acids substantially as set forth as residues 10-2161 of sequence ID No. 1 is described.
DNA encoding an α_{1D} subunit includes substantially the amino acids set forth as amino acids 1-34 in sequence ID No. 2 in place of amino acids 373-406 of SEQ ID No. 1 is also described. DNA encoding an α_{1C} subunit that includes the amino acids substantially as set forth in sequence ID No. 3 or sequence ID No. 6 is described and DNA encoding an α_{1B} subunit that includes an amino acid sequence substantially as set forth in sequence ID No. 7 or in sequence ID No. 8 is provided. A phage lysate of an *E*. *coli* host containing DNA encoding α_{1A} have been deposited in the American Type Culture Collection, 12301 Parklawn Drive, Rockville, Maryland 20852 U.S.A. under Accession No. in accord with the Budapest Treaty. The DNA in such phage includes a DNA fragment having the sequence set forth in SEQ ID No. 21. This fragment hybridizes to DNA encoding α_{1A} but not to DNA encoding α_{1B}.

DNA encoding α₂ subunits of a human calcium channel, and RNA encoding such subunits, made upon transcription of such a DNA are described. DNA encoding splice variants of the α₂ subunit, including tissue specific splice variants, are also described. In particular, DNA encoding the α₂ₐ-α_{2c} subunit subtypes is described. In particularly preferred embodiments, the DNA encoding the α₂ subunit is produced by alternative processing of a primary transcript that includes DNA encoding the amino acids set forth in SEQ ID 11 and the DNA of SEQ ID No. 13 inserted between nucleotides 1624 and 1625 of SEQ ID No. 11.

Isolated and purified DNA fragments encoding human calcium channel β subunits, including DNA encoding β₁ subunit splice variants and the β₃ subunit and is described.

In particular, DNA encoding the β₁ and β₃ subunits, including the β₁ subunit splice variants β₁₋₁-β₁₋₅, is described. RNA, encoding β subunits, made upon transcription of the DNA is also provided. *Escherichia coli (E. coli)* containing plasmids containing DNA encoding have been deposited in accord with the Budapest Treaty under Accession No. 69048 at the American Type Culture Collection. A partial sequence of the deposited clone is set forth in SEQ ID No. 19 (sequence from the 5' end) and SEQ ID No. 20 (sequence from the 3' end).

DNA encoding β subunits that are produced by alternative processing of a primary transcript encoding a β subunit, including a transcript that includes DNA encoding the amino acids set forth in SEQ ID No. 9 or including a primary transcript that encodes β₃ as deposited under ATCC Accession No. 69048, but lacking and including alternative exons are described or may be constructed from the DNA described herein. For example, DNA encoding a β subunit that is produced by alternative processing of a primary transcript that includes DNA encoding the amino acids set forth in SEQ ID No. 9, but including the DNA set forth in SEQ ID No. 12 inserted in place of nucleotides 615-781 of SEQ ID No. 9 is also described. DNA encoding β subunits that are encoded by transcripts that have the sequence set forth in SEQ ID No. 9 including the DNA set forth in SEQ ID No. 12 inserted in place of nucleotides 615-781 of SEQ ID No. 9, but that lack one or more of the following sequences of nucleotides: nucleotides 14-34 of SEQ ID No. 12, nucleotides 13-34 of SEQ ID No. 12, nucleotides 35-55 of SEQ ID No 12, nucleotides 56-190 of SEQ ID No. 12 and nucleotides 191-271 of SEQ ID No. 12 are also described.

DNA encoding γ subunits of human calcium channels is also described. RNA, encoding γ subunits, made upon transcription of the DNA are also provided. In particular, DNA containing the sequence of nucleotides set forth in SEQ ID No. 14 is described.

Full-length DNA clones and corresponding RNA transcripts, encoding the α₁, including α_{1D}, α_{1B}, α₂ and β subunits, including β₁₋₁-β₁₋₅, of human calcium channels are described. Also described are DNA clones encoding substantial portions of the α_{1A}, α_{1C}, β₃ and γ subunits of voltage-dependent human calcium channels for the preparation of full-length DNA clones encoding the full-length α_{1A}, α_{1C}, β₃ and γ subunits.

Nucleic acid probes containing at least about 14 contiguous nucleotides of α_{1D}, α_{1C}, α_{1B}, α_{1A}, α₂, β, including β₁ splice variants and β₁, and γ subunit-encoding DNA are described. Methods using the probes for the isolation and cloning of calcium channel subunit-encoding cDNA, including splice variants within tissues and inter-tissue variants are also described.

Purified human calcium channel subunits and purified human calcium channels are described. The subunits and channels can be isolated from a eukaryotic cell transfected with DNA that encodes the subunit.

Immunoglobulins or antibodies obtained from the serum of an animal immunized with a substantially pure preparation of a human calcium channel, human calcium channel subunit or epitope-containing fragment of a human calcium subunit are described Monoclonal antibodies produced using a human calcium channel, human calcium channel subunit or epitope-containing fragment thereof as an immunogen are also described. *E. coli* fusion proteins including a fragment of a human calcium channel subunit may also be used as immunogen. Such fusion proteins may contain a bacterial protein or portion thereof, such as the *E. coli* TrpE protein, fused to a calcium channel subunit peptide. The immunoglobulins that are produced using the calcium channel subunits or purified calcium channels as immunogens have, among other properties, the ability to specifically and preferentially bind to and/or cause the immunoprecipitation of a human calcium channel or a subunit thereof which may be present in a biological sample or a solution derived from such a biological sample.

A diagnostic method for determining the presence of Lambert Eaton Syndrome (LES) in a human based on immunological reactivity of LES immunoglobulin G (IgG) with a human calcium channel subunit or a eukaryotic cell which expresses a recombinant human calcium channel or a subunit thereof is also described. In particular, an immunoassay method for diagnosing Lambert-Eaton Syndrome in a person by combining serum or an IgG fraction from the person (test serum) with calcium channel proteins, including the α and β subunits, and ascertaining whether antibodies in the test serum react with one or more of the subunits, or a recombinant cell which expresses one or more of the subunits to a greater extent than antibodies in control serum, obtained from a person or group of persons known to be free of the Syndrome, is described. Any immunoassay procedure known in the art for detecting antibodies against a given antigen in serum can be employed in the method.

### DETAILED DESCRIPTION OF THE INVENTION

### Definitions:

Unless defined otherwise, all technical and scientific terms used herein have the same meaning as is commonly understood by one of skill in the art to which this invention belongs. All patents and publications referred to herein are incorporated by reference herein.

Reference to each of the calcium channel subunits includes the subunits that are specifically disclosed herein and human calcium channel subunits encoded by DNA that can be isolated by using the DNA disclosed as probes and screening an appropriate human cDNA or genomic library under at least low stringency. Such DNA also includes DNA that encodes proteins that have about 40% homology to any of the subunits proteins described herein or DNA that hybridizes under conditions of at least low stringency to the DNA provided herein and the protein encoded by such DNA exhibits additional identifying characteristics, such as function or molecular weight, except for DNA encoding the α_{1B} subunit

It is understood that subunits that are encoded by transcripts that represent splice variants of the disclosed subunits or other such subunits may exhibit less than 40% overall homology to any single subunit, but will include regions of such homology to one or more such subunits. It is also understood that 40% homology refers to proteins that share approximately 40% of their amino acids in common or that share somewhat less, but include conservative amino acid substitutions, whereby the activity of the protein is not substantially altered.

As used herein, the a, subunits types, encoded by different genes, are designated as type α_{1A}, α_{1B}, α_{1C}, and α₁. These types may also be also referred to as VDCC IV for α_{1B}, VDCC II for α_{1C} and VDCC III for α_{1D}. Subunit subtypes, which are splice variants, are referred to, for example as α_{1B-1}, α_{1B-2}, α_{1C-1} etc.

Thus, as used herein, DNA encoding the α₁ subunit refers to DNA that hybridizes to the DNA provided herein under conditions of at least low stringency or encodes a subunit that has roughly about 40% homology to protein encoded by DNA disclosed herein that encodes an α₁ subunit of a human calcium. An α₁ subunit may be identified by its ability to form a calcium channel. Typically, a, subunits have molecular weights greater than at least about 120 kD. The activity of a calcium channel may be assessed *in vitro* by methods known to those of skill in the art, including the electrophysiological and other methods described herein. Typically, α₁ subunits include regions to which one or more modulators of calcium channel activity, such as a 1,4 DHP or ω-CgTx, interact directly or indirectly. Types of α₁ subunits may be distinguished by any method known to those of skill in the art, including on the basis of binding specificity. For example, it has been found herein that α_{1B} subunits participate in the formation N-type channels, α_{1D} subunits participate in the formation of L-type channels, and α_{1A} subunits appear to participate in the formation of channels that exhibit characteristics typical of P-type channels. Thus, for example, the activity of channels that contain the α_{1B} subunit are insensitive to 1,4 DHPs; whereas the activity of channels that contain the α_{1D} subunit are modulated or altered by a 1,4 DHP. Types and subtypes of α₁ subunits may be characterized on the basis of the effects of such modulators on the subunit or a channel containing the subunit as well as differences in currents and current kinetics produced by calcium channels containing the subunit.

As used herein, an α₂ subunit is encoded by DNA that hybridizes to the DNA provided herein under conditions of low stringency or encodes a protein that has about 40% homology with that disclosed herein. Such DNA encodes a protein that typically has a molecular weight greater than about 120 kD, but does not form a calcium channel in the absence of an a, subunit, and may alter the activity of a calcium channel that contains an a, subunit. Subtypes of the α₂ subunit that arise as splice variants are designated by lower case letter, such as α₂ₐ, ... α₂ₐ. In addition, the α₂ subunit and the large fragment produced under reducing conditions appear to be glycosylated with at least N-linked sugars and do not specifically bind to the 1,4-DHPs and phenylalkylamines that specifically bind to the α₁ subunit. The smaller fragment, the C-terminal fragment, is referred to as the δ subunit and includes amino acids from about 946 (SEQ ID No. 11) through about the C-terminus. This fragment may dissociate from the remaining portion of α₂ when the α₂ subunit is exposed to reducing conditions.

As used herein, a β subunit is encoded by DNA that hybridizes to the DNA provided herein under conditions of low stringency or encodes a protein that has about 40% homology with that disclosed herein and is a protein that typically has a molecular weight lower than the a subunits and on the order of about 50-80 kD, does not form a detectable calcium channel in the absence of an α₁ subunit, but may alter the activity of a calcium channel that contains an α₁ subunit or that contains an α₁ and α₂ subunit.

Types of the β subunit that are encoded by different genes are designated with subscripts, such as β₁ and β₃. Subtypes of β subunits that arise as splice variants of a particular type are designated with a numerical subscript referring to the subtype and to the variant. Such subtypes include, but are not limited to the β₁ splice variants, including β₁₋₁-β₁₋₅.

As used herein, a γ subunit is a subunit encoded by DNA disclosed herein as encoding the γ subunit and may be isolated and identified using the DNA disclosed herein as a probe by hybridization or other such method known to those of skill in the art, whereby full-length clones encoding a γ subunit may be isolated or constructed. A γ subunit will be encoded by DNA that hybridizes to the DNA provided herein under conditions of low stringency or exhibits sufficient sequence homology to encode a protein that has about 40% homology with the γ subunit described herein.

Thus, one of skill in the art, in light of the disclosure herein, can identify DNA encoding α₁, α₂, β, δ and calcium channel subunits, including types encoded by different genes and subtypes that represent splice variants. For example, DNA probes based on the DNA disclosed herein may be used to screen an appropriate library, including a genomic or cDNA library, and obtain DNA in one or more clones that includes an open reading fragment that encodes an entire protein. Subsequent to screening an appropriate library with the DNA disclosed herein, the isolated DNA can be examined for the presence of an open reading frame from which the sequence of the encoded protein may be deduced. Determination of the molecular weight and comparison with the sequences herein should reveal the identity of the subunit as an α₁, α₂ etc. subunit. Functional assays may, if necessary, be used to determine whether the subunit is an α₁, α₂ subunit or subunit.

For example, DNA encoding α_{1A} may be isolated by screening an appropriate library with DNA, encoding all or a portion of the human α_{1A} subunit, isolated from the phage deposited under ATCC Accession No. , including screening with an oligonucleotide having the sequence set forth in SEQ ID No. 21. Similarly, DNA encoding β₃ may be isolated by screening a human cDNA library with DNA probes prepared from the plasmid β1.42 deposited under ATCC Accession No. 69048 or probes having sequences prepared according to the sequences set forth in SEQ ID Nos. 19 and 20. Any method known to those of skill in the art for isolation and identification of DNA and preparation of full-length genomic or cDNA clones, including methods exemplified herein, may be used.

The subunit encoded by isolated DNA may be identified by comparison with the DNA and amino acid sequences of the subunits provided herein. Splice variants share extensive regions of homology, but include non-homologous regions, subunits encoded by different genes share a uniform distribution of non-homologous sequences.

As used herein, a splice variant refers to a variant produced by differential processing of a primary transcript of genomic DNA that results in more than one type of mRNA. Splice variants may occur within a single tissue type or among tissues (tissue-specific variants). Thus, cDNA clones that encode calcium channel subunit subtypes that have regions of identical amino acids and regions of different amino acid sequences are referred to herein as "splice variants".

As used herein, a "calcium channel selective ion" is an ion that is capable of flowing through, or being blocked from flowing through, a calcium channel which spans a cellular membrane under conditions which would substantially similarly permit or block the flow of Ca²⁺. Ba²⁺ is an example of an ion which is a calcium channel selective ion.

As used herein, a compound that modulates calcium channel activity is one that affects the ability of the calcium channel to pass calcium channel selective ions or affects other detectable calcium channel features, such as current kinetics. Such compounds include calcium channel antagonists and agonists and compounds that exert their effect on the activity of the calcium channel directly or indirectly.

As used herein, a "substantially pure" subunit or protein is a subunit or protein that is sufficiently free of other polypeptide contaminants to appear homogeneous by SDS-PAGE or to be unambiguously sequenced.

As used herein, heterologous or foreign DNA and RNA are used interchangeably and refer to DNA or RNA that does not occur naturally as part of the genome in which it is present or which is found in a location or locations in the genome that differ from that in which it occurs in nature. It is DNA or RNA that is not endogenous to the cell and has been artificially introduced into the cell. Examples of heterologous DNA include, but are not limited to, DNA that encodes a calcium channel subunit and DNA that encodes RNA or proteins that mediate or alter expression of endogenous DNA by affecting transcription, translation, or other regulatable biochemical processes. The cell that expresses the heterologous DNA, such as DNA encoding the calcium channel subunit, may contain DNA encoding the same or different calcium channel subunits. The heterologous DNA need not be expressed and may be introduced in a manner such that it is integrated into the host cell genome or is maintained episomally.

As used herein, operative linkage of heterologous DNA to regulatory and effector sequences of nucleotides, such as promoters, enhancers, transcriptional and translational stop sites, and other signal sequences, refers to the functional relationship between such DNA and such sequences of nucleotides. For example, operative linkage of heterologous DNA to a promoter refers to the physical and functional relationship between the DNA and the promoter such that the transcription of such DNA is initiated from the promoter by an RNA polymerase that specifically recognizes, binds to and transcribes the DNA in reading frame.

As used herein, isolated, substantially pure DNA refers to DNA fragments purified according to standard techniques employed by those skilled in the art [see, *e.g.*, Maniatis *et al.* (1982) *Molecular Cloning: A Laboratory Manual*, Cold Spring Harbor Laboratory Press, Cold Spring Harbor, NY].

As used herein, expression refers to the process by which nucleic acid is transcribed into mRNA and translated into peptides, polypeptides, or proteins. If the nucleic acid is derived from genomic DNA, expression may, if an appropriate eukaryotic host cell or organism is selected, include splicing of the mRNA.

As used herein, vector or plasmid refers to discrete elements that are used to introduce heterologous DNA into cells for either expression of the heterologous DNA or for replication of the cloned heterologous DNA. Selection and use of such vectors and plasmids are well within the level of skill of the art.

As used herein, expression vector includes vectors capable of expressing DNA fragments that are in operative linkage with regulatory sequences, such as promoter regions, that are capable of effecting expression of such DNA fragments. Thus, an expression vector refers to a recombinant DNA or RNA construct, such as a plasmid, a phage, recombinant virus or other vector that, upon introduction into an appropriate host cell, results in expression of the cloned DNA. Appropriate expression vectors are well known to those of skill in the art and include those that are replicable in eukaryotic cells and/or prokaryotic cells and those that remain episomal or may integrate into the host cell genome.

As used herein, a promoter region refers to the portion of DNA of a gene that controls transcription of DNA to which it is operatively linked. The promoter region includes specific sequences of DNA that are sufficient for RNA polymerase recognition, binding and transcription initiation. This portion of the promoter region is referred to as the promoter. In addition, the promoter region includes sequences that modulate this recognition, binding and transcription initiation activity of the RNA polymerase. These sequences may be *cis* acting or may be responsive to *trans* acting factors. Promoters, depending upon the nature of the regulation, may be constitutive or regulated.

As used herein, a recombinant eukaryotic cell is a eukaryotic cell that contains heterologous DNA or RNA.

As used herein, a recombinant or heterologous calcium channel refers to a calcium channel that contains one or more subunits that are encoded by heterologous DNA that has been introduced into and expressed in a eukaryotic cells that expresses the recombinant calcium channel. A recombinant calcium channel may also include subunits that are produced by DNA endogenous to the cell. In certain embodiments, the recombinant or heterologous calcium channel may contain only subunits that are encoded by heterologous DNA.

As used herein, "functional" with respect to a recombinant or heterologous calcium channel means that the channel is able to provide for and regulate entry of calcium channel selective ions, including, but not limited to, Ca²⁺ or Ba²⁺, in response to a stimulus and/or bind ligands with affinity for the channel. Preferably such calcium channel activity is distinguishable, such as electrophysiological, pharmacological and other means known to those of skill in the art, from any endogenous calcium channel activity that in the host cell.

As used herein, a peptide having an amino acid sequence substantially as set forth in a particular SEQ ID No. includes peptides that have the same function but may include minor variations in sequence, such as conservative amino acid changes or minor deletions or insertions that do not alter the activity of the peptide. The activity of a calcium channel receptor subunit peptide refers to its ability to form functional calcium channels with other such subunits.

As used herein, a physiological concentration of a compound is that which is necessary and sufficient for a biological process to occur. For example, a physiological concentration of a calcium channel selective ion is a concentration of the calcium channel selective ion necessary and sufficient to provide an inward current when the channels open.

As used herein, activity of a calcium channel refers to the movement of a calcium selective ion through a calcium channel. Such activity may be measured by any method known to those of skill in the art, including, but not limited to, measurement of the amount of current which flows through the recombinant channel in response to a stimulus.

As used herein, a "functional assay" refers to an assay that identifies functional calcium channels. A functional assay, thus, is an assay to assess function.

As understood by those skilled in the art, assay methods for identifying compounds, such as antagonists and agonists, that modulate calcium channel activity, generally requires comparison to a control. One type of a "control" cell or "control" culture is a cell or culture that is treated substantially the same as the cell or culture exposed to the test compound except that the control culture is not exposed to the test compound. Another type of a "control" cell or "control" culture may be a cell or a culture of cells which are identical to the transfected cells except the cells employed for the control culture do not express functional calcium channels. In this situation, the response of test cell to the test compound compared to the response (or lack of response) of the receptor-negative cell to the test compound, when cells or cultures of each type of cell are exposed to substantially the same reaction conditions in the presence of the compound being assayed. For example, in methods that use patch clamp electrophysiological procedures, the same cell can be tested in the presence and absence of the test compound, by changing the external solution bathing the cell as known in the art.

### Assays

### Assays for identifying compounds that modulate calcium channel activity

*In vitro* methods for identifying compounds, such as calcium channel agonist and antagonists, that modulate the activity of calcium channels using eukaryotic cells that express heterologous human calcium channels are provided.

In particular, the assays use eukaryotic cells that express heterologous human calcium channel subunits encoded by heterologous DNA provided herein, for screening potential calcium channel agonists and antagonists which are specific for human calcium channels and particularly for screening for compounds that are specific for particular human calcium channel subtypes. Such assays may be used in conjunction with methods of rational drug design to select among agonists and antagonists, which differ slightly in structure, those particularly useful for modulating the activity of human calcium channels, and to design or select compounds that exhibit subtype- or tissue-specific calcium channel antagonist and agonist activities.

These assays should accurately predict the relative therapeutic efficacy of a compound for the treatment of certain disorders in humans. In addition, since subtype- and tissue-specific calcium channel subunits are provided, cells with tissue- specific or subtype-specific recombinant calcium channels may be prepared and used in assays for identification of human calcium channel tissue- or subtype-specific drugs.

The assays involve contacting the cell membrane of a recombinant eukaryotic cell which expresses at least one subunit of a human calcium channel, preferably at least an α₁ subunit of a human calcium channel, with a test compound and measuring the ability of the test compound to specifically bind to the membrane or alter or modulate the activity of a heterologous calcium channel on the membrane.

In preferred embodiments, the assay uses a recombinant cell which has a calcium channel containing an α₁ subunit of a human calcium channel in combination with a β-subunit of a human calcium channel and/or an α₂ subunit of a human calcium channel. Recombinant cells expressing heterologous calcium channels containing each of the α₁, β and α₂ human subunits, and, optionally, a γ subunit of a human calcium channel are especially preferred for use in such assays.

In certain embodiments, the assays for identifying compounds that modulate calcium channel activity are practiced by measuring the calcium channel activity of a eukaryotic cell having a heterologous, functional calcium channel when such cell is exposed to a solution containing the test compound and a calcium channel selective ion and comparing the measured calcium channel activity to the calcium channel activity of the same cell or a substantially identical control cell in a solution not containing the test compound. The cell is maintained in a solution having a concentration of calcium channel selective ions sufficient to provide an inward current when the channels open. Especially preferred for use, is a recombinant cell expressing calcium channels that include each of the α₁, β and α₂ human subunits, and, optionally, a γ subunit of a human calcium channel. Methods for practicing such assays are known to those of skill in the art. For example, for similar methods applied with *Xenopus* *laevis* oöcytes and acetylcholine receptors, see, Mishina *et al.* [(1985) *Nature 313*:364] and, with such oöcytes and sodium channels [see, Noda *et al.* (1986) *Nature 322*:826-828]. For similar studies which have been carried out with the acetylcholine receptor, see, *e.g.,* Claudio *et al*. [(1987) *Science 238*:1688-1694].

The assays thus use cells, provided herein, that express heterologous functional calcium channels and measure functionally, such as electrophysiologically, the ability of a test compound to potentiate, antagonize or otherwise modulate the magnitude and duration of the flow of calcium channel selective ions, such as ca⁺⁺ or Ba⁺⁺, through the heterologous functional channel. The amount of current which flows through the recombinant calcium channels of a cell may be determined directly, such as electrophysiologically, or by monitoring an independent reaction which occurs intracellularly and which is directly influenced in a calcium (or other) ion dependent manner.

Any method for assessing the activity of a calcium channel may be used in conjunction with the cells and assays provided herein. For example, in one embodiment of the method for testing a compound for its ability to modulate calcium channel activity, the amount of current is measured by its modulation of a reaction which is sensitive to calcium channel selective ions and uses a eukaryotic cell which expresses a heterologous calcium channel and also contains a transcriptional control element operatively linked for expression to a structural gene that encodes an indicator protein. The transcriptional control element used for transcription of the indicator gene is responsive in the cell to a calcium channel selective ion, such as Ca²⁺ and Ba⁺. The details of such transcriptional based assays are described in commonly owned PCT International Patent Application No. PCT/US91/5625, filed August 7, 1991, which claims priority to copending commonly owned U.S. Application Serial No. 07/ 563,751, filed August 7, 1990, the contents of which applications are herein incorporated by reference thereto.

### Assays for diagnosis of LES

LES is an autoimmune disease characterized by an insufficient release of acetylcholine from motor nerve terminals which normally are responsive to nerve impulses. Immunoglobulins (IgG) from LES patients block individual voltage-dependent calcium channels and thus inhibit calcium channel activity [Kim and Neher, *Science 239*:405-408 (1988)]. A diagnostic assay for Lambert Eaton Syndrome (LES) is provided herein. The diagnostic assay for LES relies on the immunological reactivity of LES IgG with the human calcium channels or particular subunits alone or in combination or expressed on the surface of recombinant cells. For example, such an assay may be based on immunoprecipitation of LES IgG by the human calcium channel subunits and cells that express such subunits provided herein.

### Identification and isolation of DNA encoding human calcium channel subunits

Methods for identifying and isolating DNA encoding α₁, α₂, β and γ subunits of human calcium channels are provided.

Identification and isolation of such DNA may be accomplished by hybridizing, under appropriate conditions, at least low stringency whereby DNA that encodes the desired subunit is isolated, restriction enzyme-digested human DNA with a labeled probe having at least 14 nucleotides and derived from any contiguous portion of DNA having a sequence of nucleotides set forth herein by sequence identification number. Once a hybridizing fragment is identified in the hybridization reaction, it can be cloned employing standard cloning techniques known to those of skill in the art. Full-length clones may be identified by the presence of a complete open reading frame and the identity of the encoded protein verified by sequence comparison with the subunits provided herein and by functional assays to assess calcium channel forming ability or other function. This method can be used to identify genomic DNA encoding the subunit or cDNA encoding splice variants of human calcium channel subunits generated by alternative splicing of the primary transcript of genomic subunit DNA. For instance, DNA, cDNA or genomic DNA, encoding a calcium channel subunit may be identified by hybridization to a DNA probe and characterized by methods known to those of skill in the art, such as restriction mapping and DNA sequencing, and compared to the DNA provided herein in order to identify heterogeneity or divergence in the sequences the DNA. Such sequence differences may indicate that the transcripts from which the cDNA was produced result from alternative splicing of a primary transcript, if the non-homologous and homologous regions are clustered, or from a different gene if the non-homologous regions are distributed throughout the cloned DNA.

Any suitable method for isolating genes using the DNA provided herein may be used. For example, oligonucleotides corresponding to regions of sequence differences have been used to isolate, by hybridization, DNA encoding the full-length splice variant and can be used to isolate genomic clones. A probe, based on a nucleotide sequence disclosed herein, which encodes at least a portion of a subunit of a human calcium channel, such as a tissue-specific exon, may be used as a probe to clone related DNA, to clone a full-length cDNA clone or genomic clone encoding the human calcium channel subunit.

Labeled, including, but not limited to, radioactively or enzymatically labeled, RNA or single-stranded DNA of at least 14 substantially contiguous bases, preferably at least 30 contiguous bases of a nucleic acid which encodes at least a portion of a human calcium channel subunit, the sequence of which nucleic acid corresponds to a segment of a nucleic acid sequence disclosed herein by reference to a SEQ ID No. are provided. Such nucleic acid segments may be used as probes in the methods provided herein for cloning DNA encoding calcium channel subunits. See, generally, Sambrook *et al*. (1989) *Molecular Cloning:* A *Laboratory Manual*, 2nd Edition, Cold Spring Harbor Laboratory Press.

In addition, nucleic acid amplification techniques, which are well known in the art, can be used to locate splice variants of calcium channel subunits by employing oligonucleotides based on DNA sequences surrounding the divergent sequence primers for amplifying human RNA or genomic DNA. Size and sequence determinations of the amplification products can reveal splice variants. Furthermore, isolation of human genomic DNA sequences by hybridization can yield DNA containing multiple exons, separated by introns, that correspond to different splice variants of transcripts encoding human calcium channel subunits.

DNA encoding types and subtypes of each of the α₁, α₂ β and γ subunit of voltage-dependent human calcium channels has been cloned herein by screening human cDNA libraries prepared from isolated poly A+ mRNA from cell lines or tissue of human origin having such calcium channels. Among the sources of such cells or tissue for obtaining mRNA are human brain tissue or a human cell line of neural origin, such as a neuroblastoma cell line, human skeletal muscle or smooth muscle cells, and the like. Methods of preparing cDNA libraries are well known in the art [see generally Ausubel *et al.* (1987) *Current Protocols in Molecular Biology,* Wiley-Interscience, New York; and Davis *et al.* (1986) *Basic Methods in Molecular Biology,* Elsevier Science Publishing Co., New York].

With respect to each of the respective subunits of a human calcium channel (α₁, α₂, β or γ), once the DNA encoding the channel subunit was identified by a nucleic acid screening method, the isolated clone was used for further screening to identify overlapping clones. Some of the cloned DNA fragments can and have been subcloned into an appropriate vector such as pIBI24/25 (IBI, New Haven, CT), M13mp18/19, pGEM4, pGEM3, pGEM7Z, pSP72 and other such vectors known to those of skill in this art, and characterized by DNA sequencing and restriction enzyme mapping. A sequential series of overlapping clones may thus be generated for each of the subunits until a full-length clone can be prepared by methods, known to those of skill in the art, that include identification of translation initiation (start) and translation termination (stop) codons. For expression of the cloned DNA, the 5' noncoding region and other transcriptional and translational control regions of such a clone may be replaced with an efficient ribosome binding site and other regulatory regions as known in the art. Examples II-VI, below, describe in detail the cloning of each of the various subunits of a human calcium channel as well as subtypes and splice variants, including tissue-specific variants thereof. In the instances in which partial sequences of a subunit are disclosed, it is well within the skill of the art, in view of the teaching herein, to obtain the corresponding full-length nucleotide sequence encoding the subunit, subtype or splice variant thereof.

### Identification and isolation of DNA encoding α₁ subunits

A number of voltage-dependent calcium channel a, subunit genes, which are expressed in the human CNS, have been identified and have been designated as α_{1A}, α_{1B} (or VDCC IV), α_{1C} (or VDCC II) and α_{1D} (or VDCC III). DNA, isolated from a human neuronal cDNA library, that encodes each of the subunit types has been isolated. DNA encoding subtypes of each of the types, which arise as splice variants are also provided. Subtypes are herein designated, for example, as α_{1B-1}, α_{1B-2}.

The α₁ subunits types A B, C, and D of voltage-dependent calcium channels, and subtypes thereof, differ with respect to sensitivity to known classes of calcium channel agonists and antagonists, such as DHPs, phenylalkylamines, omega conotoxin (ω-CgTx) and pyrazonoylguanidines. They also appear to differ in the holding potential and ion the kinetics of currents produced upon depolarization of cell membranes containing calcium channels that include different types of a, subunits.

DNA that encodes an α₁-subunit that binds to at least one compound selected from among dihydropyridines, phenylalkylamines, ω-CgTx, components of funnel web spider toxin, and pyrazonoylguanidines is provided. For example, the α_{1B} subunit provided herein appears to specifically interact with ω-CgTx in N-type channels, and the α_{1D} subunit provided herein specifically interacts with DHPs in L-type channels.

### Identification and isolation of DNA encoding the α_{1D} human calcium channel subunit

The α_{1D} subunit cDNA has been isolated using fragments of the rabbit skeletal muscle calcium channel α₁ subunit cDNA as a probe to screen a cDNA library of a human neuroblastoma cell line, IMR32, to obtain clone α1.36. This clone was used as a probe to screen additional IMR32 cell cDNA libraries to obtain overlapping clones, which were then employed for screening until a sufficient series of clones to span the length of the nucleotide sequence encoding the human α_{1D} subunit were obtained. Full-length clones encoding α_{1D} were constructed by ligating portions of partial α_{1D} clones as described in Example II. SEQ ID No. 1 shows the 7,635 nucleotide sequence of the cDNA encoding the α_{1D} subunit. There is a 6,483 nucleotide sequence reading frame which encodes a sequence of 2,161 amino acids (as set forth in SEQ ID No. 1).

SEQ ID No. 2 provides the sequence of an alternative exon encoding the IS6 transmembrane domain [see Tanabe, T., *et al. (*1987) *Nature 328*:313-318 for a description of transmembrane domain terminology of the α_{1D} subunit.

SEQ ID No. 1 also shows the 2,161 amino acid sequence deduced from the human neuronal calcium channel α_{1D} subunit DNA. Based on the amino acid sequence, the α_{1D} protein has a calculated Mr of 245,163. The α_{1D} subunit of the calcium channel contains four putative internal repeated sequence regions. Four internally repeated regions represent 24 putative transmembrane segments, and the amino- and carboxyl-termini extend intracellularly.

The α_{1D} subunit has been shown to mediate DHP-sensitive, high-voltage-activated, long-lasting calcium channel activity. This calcium channel activity was detected when oöcytes were co-injected with RNA transcripts encoding an α_{1D} and β₁ or α_{1D}, α₂ and β₁ subunits. This activity was distinguished from Ba²⁺ currents detected when oöcytes were injected with RNA transcripts encoding the β₁ ± α₂ subunits. These currents pharmacologically and biophysically resembled Ca²⁺ currents reported for uninjected oöcytes.

### Identification and isolation DNA encoding the α_{1A} human calcium channel subunit

Biological material containing DNA encoding the α_{1A} subunit had been deposited in the American Type Culture Collection, 12301 Parklawn Drive, Rockville, Maryland 20852 U.S.A. under the terms of the Budapest Treaty on the International Recognition of Deposits of Microorganisms for Purposes of Patent Procedure and the Regulations promulgated under this Treaty. Samples of the deposited material are and will be available to industrial property offices and other persons legally entitled to receive them under the terms of said Treaty and Regulations and otherwise in compliance with the patent laws and regulations of the United States of America and all other nations or international organizations in which this application, or an application claiming priority of this application, is filed or in which any patent granted on any such application is granted.

The α_{1A} subunit is encoded by an approximately 3 kb insert in λgt10 phage designated α1.254 in *E. coli* host strain NM514. A phage lysate of this material has been deposited as at the American Type Culture Collection under ATCC Accession No. , as described above. DNA encoding α_{1A} may also be identified by screening with a probe prepared from DNA that has SEQ ID No. 21:

### Identification and isolation of DNA encoding the α_{1B} human calcium channel subunit

DNA encoding the α_{1B} subunit was isolated by screening a human basal ganglia cDNA library with fragments of the rabbit skeletal muscle calcium channel α₁ subunit-encoding cDNA. A portion of one of the positive clones was used to screen an IMR32 cell cDNA library. Clones that hybridized to the basal ganglia DNA probe were used to further screen an IMR32 cell cDNA library to identify overlapping clones that in turn were used to screen a human hippocampus cDNA library. In this way, a sufficient series of clones to span nearly the entire length of the nucleotide sequence encoding the human α_{1B} subunit was obtained. PCR amplification of specific regions of the IMR32 cell α_{1B} mRNA yielded additional segments of the α_{1B} coding sequence.

A full-length α_{1B} DNA clone was constructed by ligating portions of the partial cDNA clones as described in Example II.C. SEQ ID Nos. 7 and 8 show the nucleotide sequences of DNA clones encoding the α_{1B} subunit as well as the deduced amino acid sequences. The α_{1B} subunit encoded by SEQ ID No. 7 is referred to as the α_{1B-1} subunit to distinguish it from another α_{1B} subunit, α_{1B-2}, encoded by the nucleotide sequence shown as SEQ ID No. 8, which is derived from alternative splicing of the α_{1B} subunit transcript.

PCR amplification of IMR32 cell mRNA using oligonucleotide primers designed according to nucleotide sequences within the α_{1B-1}-encoding DNA has identified variants of the α_{1B} transcript that appear to be splice variants because they contain divergent coding sequences.

### Identification and isolation of DNA encoding the α_{1C} human calcium channel subunit

Numerous α_{1C}-specific DNA clones were isolated. Characterization of the sequence revealed the α_{1C} coding sequence, the α_{1C} initiation of translation sequence, and an alternatively spliced region of α_{1C}. Alternatively spliced variants of the α_{1C} subunit have been identified. SEQ ID No. 3 sets forth DNA encoding an α_{1C} subunit. The DNA sequences set forth in SEQ ID No. 4 and No. 5 encode two possible amino terminal ends of the α_{1C} protein. SEQ ID No. 6 encodes an alternative exon for the IV S3 transmembrane domain.

The isolation and identification of DNA clones encoding portions of the α_{1C} subunit is described in detail in Example II.

DNA encoding other α₁ subunits, including α_{1A}, has also been isolated. Additional such subunits may also be isolated and identified using the DNA provided herein as described for the α_{1B}, α_{1C} and α_{1D} subunits or using other methods known to those of skill in the art.

### Identification and isolation DNA encoding β human calcium channel subunits DNA encoding β₁

To isolate DNA encoding the β₁ subunit, a human hippocampus cDNA library was screened by hybridization to a DNA fragment encoding a rabbit skeletal muscle calcium channel β subunit. A hybridizing clone was selected and was in turn used to isolate overlapping clones until the overlapping clones encompassing DNA encoding the entire the human calcium channel β subunit were isolated and sequenced.

Five alternatively spliced forms of the human calcium channel β₁ subunit have been identified and DNA encoding a number of forms have been isolated. These forms are designated β₁₋₁, expressed in skeletal muscle, β₁₋₂, expressed in the CNS, β₁₋₃, also expressed in the in the CNS, β₁₋₄, expressed in aorta tissue and HEK 293 cells, and β₁₋₅, expressed in HEK 293 cells. A full-length DNA clone encoding the β₁₋₂ subunit has been constructed. The subunits β₁₋₁, β₁₋₂, β₁₋₄ and β₁₋₅ have been identified by PCR analysis as alternatively spliced forms of the β subunit.

The alternatively spliced variants were identified by comparison of amino acid sequences encoded by the human neuronal and rabbit skeletal muscle calcium channel β subunit-encoding DNA. This comparison revealed a 45-amino acid deletion in the human β subunit compared to the rabbit β subunit. Using DNA from the region as a probe for DNA cloning, as well as PCR analysis and DNA sequencing of this area of sequence divergence, alternatively spliced forms of the human calcium channel β subunit transcript were identified. For example, the sequence of DNA encoding one splice variant β₁₋₂ is set forth in SEQ ID No. 9. SEQ ID No. 10 sets forth the sequence of the β₁₋₃ subunit (nt 1-1851, including 3' untranslated sequence nt 1795-1851), which is another splice variant of the β subunit primary transcript. β₁₋₂ and β₁₋₃ are human neuronal β subunits. DNA distinctive for a portion of a β subunit (β₁₋₄) of a human aortic calcium channel and also human embryonic kidney (HEK) cells is set forth in SEQ ID No. 12 (nt 1-13 and 191-271). The sequence of DNA encoding a portion of a human calcium channel β subunit expressed in skeletal muscle (β₁₋₁) is shown in SEQ ID No. 12 (nt 1-12 and 35-271).

### DNA encoding β₃

DNA encoding the β₃ subunit and any splice variants thereof may be isolated by screening a library, as described above for the β₁ subunit, using DNA probes prepared according to SEQ ID Nos. 19 and 20 or using all or a portion of the deposited β₃ clone plasmid β1.42 (ATCC Accession No. 69048).

The *E. coli* host containing plasmid β1.42 that includes DNA encoding the β₃ subunit have been deposited as ATCC Accession No. 69048 in the American Type Culture Collection, 12301 Parklawn Drive, Rockville, Maryland 20852 U.S.A. under the terms of the Budapest Treaty on the International Recognition of Deposits of Microorganisms for Purposes of Patent Procedure and the Regulations promulgated under this Treaty. Samples of the deposited material are and will be available to industrial property offices and other persons legally entitled to receive them under the terms of said Treaty and Regulations and otherwise in compliance with the patent laws and regulations of the United States of America and all other nations or international organizations in which this application, or an application claiming priority of this application, is filed or in which any patent granted on any such application is granted.

The β₃ encoding plasmid is designated β1.42. The plasmid contains a 2.5 kb EcoRI fragment encoding β₃ inserted into vector pGem® 7zF (+) and has been deposited i *E. coli* host strain DN5α. A partial DNA sequence of the 5' and 3' ends of β₃ are set forth in SEQ ID Nos. 19 and 20, respectively.

### Identification and isolation DNA encoding the α2 human calcium channel subunit

DNA encoding a human neuronal calcium channel α₂ subunit was isolated in a manner substantially similar to that used for isolating DNA encoding an α₁ subunit, except that a human genomic DNA library was probed under low and high stringency conditions with a fragment of DNA encoding the rabbit skeletal muscle calcium channel α₂ subunit. The fragment included nucleotides having a sequence corresponding to the nucleotide sequence between nucleotides 43 and 272 inclusive of rabbit back skeletal muscle calcium channel α₂ subunit cDNA as disclosed in PCT International Patent Application Publication No. WO 89/09834, which corresponds to U.S. Application Serial No. 07/620,520, which is a continuation-in-part of United States Serial No. 176,899, filed April 4, 1988, which applications have been incorporated herein by reference.

Example IV describes the isolation of DNA clones encoding α₂ subunits of a human calcium channel from a human DNA library using genomic DNA and cDNA clones, identified by hybridization to the genomic DNA, as probes.

SEQ ID No. 11 shows the sequence of DNA encoding an α₂ subunit. As described in Example V, PCR analysis of RNA from human skeletal muscle, brain tissue and aorta using oligonucleotide primers specific for a region of the human neuronal α₂ subunit cDNA that diverges from the rabbit skeletal muscle calcium channel α₂ subunit cDNA identified splice variants of the human calcium channel α₂ subunit transcript.

### Identification and isolation of DNA encoding γ human calcium channel subunits

DNA encoding a human neuronal calcium channel γ subunit has been isolated as described in detail in Example VI. SEQ ID No. 14 shows the nucleotide sequence at the 3'-end of this DNA which includes a reading frame encoding a sequence of 43 amino acid residues.

### Preparation of recombinant eukaryotic cells containing DNA encoding heterologous calcium channel subunits

DNA encoding one or more of the calcium channel subunits or a portion of a calcium channel subunit may be introduced into a host cell for expression or replication of the DNA. Such DNA may be introduced using methods described in the following examples or using other procedures well known to those skilled in the art. Incorporation of cloned DNA into a suitable expression vector, transfection of eukaryotic cells with a plasmid vector or a combination of plasmid vectors, each encoding one or more distinct genes or with linear DNA, and selection of transfected cells are also well known in the art [see, *e.g.*, Sambrook *et al.* (1989) *Molecular Cloning: A Laboratory Manual, Second Edition*, Cold Spring Harbor Laboratory Press].

Cloned full-length DNA encoding any of the subunits of a human calcium channel may be introduced into a plasmid vector for expression in a eukaryotic cell. Such DNA may be genomic DNA or cDNA. Host cells may be transfected with one or a combination of said plasmids, each of which encodes at least one calcium channel subunit. Alternatively, host cells may be transfected with linear DNA using methods well known to those of skill in the art.

While the DNA provided herein may be expressed in any eukaryotic cell, including yeast cells such as *P. pastoris* [see, *e.g.*, Cregg *et al*. (1987) *Bio*/*Technology 5*:479], mammalian expression systems for expression of the DNA encoding the human calcium channel subunits provided herein are preferred.

The heterologous DNA may be introduced by any method known to those of skill in the art, such as transfection with a vector encoding the heterologous DNA. Particularly preferred vectors for transfection of mammalian cells are the pSV2dhfr expression vectors, which contain the SV40 early promoter, mouse dhfr gene, SV40 polyadenylation and splice sites and sequences necessary for maintaining the vector in bacteria, cytomegalovirus (CMV) promoter-based vectors such as pCMV or pCDNA1, and MMTV promoter-based vectors. DNA encoding the human calcium channel subunits has been inserted in the vector pCDNA1 at a position immediately following the CMV promoter.

Stably or transiently transfected mammalian cells may be prepared by methods known in the art by transfecting cells with an expression vector having a selectable marker gene such as the gene for thymidine kinase, dihydrofolate reductase, neomycin resistance or the like, and, for transient transfection, growing the transfected cells under conditions selective for cells expressing the marker gene. Functional voltage-dependent calcium channels have been produced in HEK 293 cells transfected with a derivative of the vector pCDNA1 that contains DNA encoding a human calcium channel subunit.

The heterologous DNA may be maintained in the cell as an episomal element or may be integrated into chromosomal DNA of the cell. The resulting recombinant cells may then be cultured or subcultured (or passaged, in the case of mammalian cells) from such a culture or a subculture thereof. Methods for transfection, injection and culturing recombinant cells are known to the skilled artisan. Eukaryotic cells in which DNA or RNA may be introduced, include any cells that are transfectable by such DNA or RNA or into which such DNA may be injected. Virtually any eukaryotic cell can serve as a vehicle for heterologous DNA. Preferred cells are those that can also express the DNA and RNA and most preferred cells are those that can form recombinant or heterologous calcium channels that include one or more subunits encoded by the heterologous DNA. Such cells may be identified empirically or selected from among those known to be readily transfected or injected. Preferred cells for introducing DNA include those that can be transiently or stably transfected and include, but are not limited to cells of mammalian origin, such as COS cells, mouse L cells, CHO cells, human embryonic kidney cells, African green monkey cells and other such cells known to those of skill in the art, amphibian cells, such as *Xenopus laevis* oöcytes, or those of yeast such as *Saccharomyces cerevisiae* or *Pichia pastoris*. Preferred cells for expressing injected RNA transcripts include Xenopus laevis cöcytes. Cells that are preferred for transfection of DNA are those that can be readily and efficiently transfected. Such cells are known to those of skill in the art or may be empirically identified. Preferred cells include DG44 cells and HEK 293 cells, particularly HEK 293 cells that have been adapted for growth in suspension and that can be frozen in liquid nitrogen and then thawed and regrown. Such HEK 293 cells are described, for example in U.S. Patent No. 5,024,939 to Gorman [see, also Stillman *et al*. (1985) *Mol. Cell.Biol. 5*:2051-2060].

The cells may be used as vehicles for replicating heterologous DNA introduced therein or for expressing the heterologous DNA introduced therein. In certain embodiments, the cells are used as vehicles for expressing the heterologous DNA as a means to produce substantially pure human calcium channel subunits or heterologous calcium channels. Host cells containing the heterologous DNA may be cultured under conditions whereby the calcium channels are expressed. The calcium channel subunits may be purified using protein purification methods known to those of skill in the art. For example, antibodies, such as those provided herein, that specifically bind to one or more of the subunits may be used for affinity purification of the subunit or calcium channels containing the subunits.

Substantially pure subunits of a human calcium channel α₁ subunits of a human calcium channel, α₂ subunits of a human calcium channel β subunits of a human calcium channel and γ subunits of a human calcium channel are provided. Substantially pure isolated calcium channels that contain at least one of the human calcium channel subunits are also provided. Substantially pure calcium channels that contain a mixture of one or more subunits encoded by the host cell and one or more subunits encoded by heterologous DNA or RNA that has been introduced into the cell are also provided. Substantially pure subtype- or tissue-type specific calcium channels are also provided.

In other embodiments, eukaryotic cells that contain heterologous DNA encoding at least one of an α₁ subunit of a human calcium channel , an α₂ subunit of a human calcium channel, a β subunit of a human calcium channel and a γ subunit of a human calcium channel are provided. In accordance with one preferred embodiment, the heterologous DNA is expressed in the eukaryotic cell and preferably encodes a human calcium channel a, subunit.

In particularly preferred aspects, the eukaryotic cell which contains the heterologous DNA expresses it and forms a recombinant functional calcium channel activity. In more preferred aspects, the recombinant calcium channel activity is readily detectable because it is a type that is absent from the untransfected host cell or is of a magnitude not exhibited in the untransfected cell.

Preferred among such cells is a recombinant eukaryotic cell with a functional heterologous calcium channel. The recombinant cell can be produced by introduction of and expression of heterologous DNA or RNA transcripts encoding an α₁ subunit of a human calcium channel, more preferably also expressing, a heterologous DNA encoding a β subunit of a human calcium channel and/or heterologous DNA encoding an α₂ subunit of a human calcium channel. Especially preferred is the expression in such a recombinant cell of each of the α₁, β and α₂ subunits encoded by such heterologous DNA or RNA transcripts, and optionally expression of heterologous DNA or an RNA transcript encoding a γ subunit of a human calcium channel.

In certain embodiments, the eukaryotic cell with a heterologous calcium channel is produced by introducing into the cell a first composition, which contains at least one RNA transcript that is translated in the cell into a subunit of a human calcium channel. In preferred embodiments, the subunits that are translated include an α₁ subunit of a human calcium channel. More preferably, the composition that is introduced contains an RNA transcript which encodes an α₁ subunit of a human calcium channel and also contains (1) an RNA transcript which encodes a β subunit of a human calcium channel and/or (2) an RNA transcript which encodes an α₂ subunit of a human calcium channel. Especially preferred is the introduction of RNA encoding an α₁, a β and an α₂ human calcium channel subunit, and, optionally, a γ subunit of a human calcium channel.

Methods for *in vitro* transcription of a cloned DNA and injection of the resulting RNA into eukaryotic cells are well known in the art. Transcripts of any of the full-length DNA encoding any of the subunits of a human calcium channel may be injected alone or in combination with other transcripts into eukaryotic cells for expression in the cells. Amphibian oöcytes are particularly preferred for expression of *in vitro* transcripts of the human calcium channel subunit cDNA clones provided herein.

The functional calcium channels may preferably include at least an α₁ subunit and a β subunit of a human calcium channel. Eukaryotic cells expressing these two subunits and also cells expressing additional subunits, have been prepared by transfection of DNA and by injection of RNA transcripts. Such cells have exhibited voltage-dependent calcium channel activity attributable to calcium channels that contain one or more of the heterologous human calcium channel subunits. For example, eukaryotic cells expressing heterologous calcium channels containing an α₂ subunit in addition to the α₁ subunit and a β subunit have been shown to exhibit increased calcium selective ion flow across the cellular membrane in response to depolarization, indicating that the α₂ subunit may potentiate calcium channel function.

Eukaryotic cells which express heterologous calcium channels containing at least a human α₁ subunit, a human β subunit and a human α₂ subunit are preferred. Eukaryotic cells transformed with a composition containing cDNA or an RNA transcript that encodes an α₁ subunit alone or in combination with a β and/or an α₂ subunit may be used to produce cells that express functional calcium channels. Since recombinant cells expressing human calcium channels containing all of the of the human subunits encoded by the heterologous cDNA or RNA are especially preferred, it is desirable to inject or transfect such host cells with a sufficient concentration of the subunit-encoding nucleic acids to form calcium channels that contain the human subunits encoded by heterologous DNA or RNA. The precise amounts and ratios of DNA or RNA encoding the subunits may be empirically determined and optimized for a particular combination of subunits, cells and assay conditions.

With respect to measurement of the activity of functional heterologous calcium channels, preferably, endogenous ion channel activity and, if desired, heterologous channel activity of channels that do not contain the desired subunits, of a host cell can be inhibited to a significant extent by chemical, pharmacological and electrophysiological means, including the use of differential holding potential, to increase the S/N ratio of the measured heterologous calcium channel activity.

Among the uses for eukaryotic cells which recombinantly express one or more subunits are assays for determining whether a test compound has calcium channel agonist or antagonist activity. Desirably, a host cell for the expression of calcium channel subunits does not produce endogenous calcium channel subunits of the type or in an amount that substantially interferes with the detection of heterologous calcium channel subunits in ligand binding assays or detection of heterologous calcium channel function, such as generation of calcium current, in functional assays.

With respect to ligand binding assays, the host cells preferably should not produce endogenous calcium channels which detectably interact with compounds having, at physiological concentrations (generally nanomolar or picomolar concentrations), affinity for calcium channels that contain one or all of the human calcium channel subunits provided herein.

With respect to ligand binding assays for identifying a compound which has affinity for calcium channels, cells are employed which express, preferably, at least a heterologous α₁ subunit. Transfected eukaryotic cells which express at least an α₁ subunit may be used to determine the ability of a test compound to specifically alter the activity of a calcium channel. Such ligand binding assays may be performed on intact transfected cells or membranes prepared therefrom.

The capacity of a test compound to bind to or otherwise interact with membranes that contain heterologous calcium channels or subunits thereof may be determined by using any appropriate method, such as competitive binding analysis, such as Scatchard plots, in which the binding capacity of such membranes is determined in the presence and absence of one or more concentrations of a compound having known affinity for the calcium channel. Where necessary, the results may be compared to a control experiment designed in accordance with methods known to those of skill in the art. For example, as a negative control, the results may be compared to those of assays of an identically treated membrane preparation from host cells which have not been transfected with one or more subunit-encoding nucleic acids.

Stably or transiently transfected cells or injected cells which express voltage-dependent human calcium channels containing one or more of the subunits of a human calcium channel desirably may be used in assays to identify agents, such as calcium channel agonists and antagonists, that modulate calcium channel activity. Functionally testing the activity of test compounds, including compounds having unknown activity, for calcium channel agonist or antagonist activity to determine if the test compound potentiates, inhibits or otherwise alters the flow of calcium through a human calcium channel can be accomplished by (a) maintaining a eukaryotic cell which is transfected or injected to express a heterologous functional calcium channel capable of regulating the flow of calcium channel selective ions into the cell in a medium containing calcium channel selective ions (i) in the presence of and (ii) in the absence of a test compound; (b) maintaining the cell under conditions such that the heterologous calcium channels are substantially closed and endogenous calcium channels of the cell are substantially inhibited (c) depolarizing the membrane of the cell maintained in step (b) to an extent and for an amount of time sufficient to cause (preferably, substantially only) the heterologous calcium channels to become permeable to the calcium channel selective ions; and (d) comparing the amount and duration of current flow into the cell in the presence of the test compound to that of the current flow into the cell, or a substantially similar cell, in the absence of the test compound.

Functional recombinant or heterologous calcium channels may be identified by any method known to those of skill in the art. For example, electrophysiological procedures for measuring the current across an ion-selective membrane of a cell, which are well known, may be used. The amount and duration of the flow of calcium selective ions through heterologous calcium channels of a recombinant cell containing DNA encoding one or more of the subunits provided herein has been measured using electrophysiological recordings using a two electrode and the whole-cell patch clamp techniques. In order to improve the sensitivity of the assays, known methods can be used to eliminate or reduce non-calcium currents and calcium currents resulting from endogenous calcium channels, when measuring calcium currents through recombinant channels. For example, the DHP Bay K 8644 specifically enhances L-type calcium channel function by increasing the duration of the open state of the channels [see, *e.g.*, Hess, J.B., *et al.* (1984) *Nature 311*:538-544]. Prolonged opening of the channels results in calcium currents of increased magnitude and duration. Tail currents can be observed upon repolarization of the cell membrane after activation of ion channels by a depolarizing voltage command. The opened channels require a finite time to close or "deactivate" upon repolarization, and the current that flows through the channels during this period is referred to as a tail current. Because Bay K 8644 prolongs opening events in calcium channels, it tends to prolong these tail currents and make them more pronounced.

### EXAMPLES

The following examples are included for illustrative purposes only and are not intended to limit the scope of the invention.

### EXAMPLE I: PREPARATION OF LIBRARIES USED FOR ISOLATION OF DNA ENCODING HUMAN NEURONAL VOLTAGE-DEPENDENT CALCIUM CHANNEL SUBUNITS

### A. RNA Isolation

### 1. IMR32 cells

IMR32 cells were obtained from the American Type Culture Collection (ATCC Accession No. CCL127, Rockville, MD) and grown in DMEM, 10% fetal bovine serum, 1% penicillin/streptomycin (GIBC0, Grand Island, NY) plus 1.0 mM dibutyryl cAMP (dbcAMP) for ten days. Total RNA was isolated from the cells according to the procedure described by H.C. Birnboim [(1988) *Nucleic Acids Research 16*:1487-1497]. Poly(A⁺) RNA was selected according to standard procedures [see, *e.g*., Sambrook *et al.* (1989) *Molecular Cloning, A Laboratory Manual,* Cold Spring Harbor Laboratory Press; pg. 7.26-7.29].

### 2. Human thalamus tissue

Human thalamus tissue (2.34 g), obtained from the National Neurological Research Bank, Los Angeles, CA, that had been stored frozen at -70°C was pulverized using a mortar and pestle in the presence of liquid nitrogen and the cells were lysed in 12 ml of lysis buffer (5 M guanidinium isothiocyanate, 50 mM TRIS, pH 7.4, 10 mM EDTA, 5% β-mercaptoethanol). Lysis buffer was added to the lysate to yield a final volume of 17 ml. N-laurylsarcosine and CsCl were added to the mixture to yield final concentrations of 4% and 0.01 g/ml, respectively, in a final volume of 18 ml.

The sample was centrifuged at 9,000 rpm in a Sorvall SS34 rotor for 10 min at room temperature to remove the insoluble material as a pellet. The supernatant was divided into two equal portions and each was layered onto a 2-ml cushion of a solution of 5.7 M CsCl, 0.1 M EDTA contained in separate centrifuge tubes to yield approximately 9 ml per tube. The samples were centrifuged in an SW41 rotor at 37,000 rpm for 24 h at 20°C.

After centrifugation, each RNA pellet was resuspended in 3 ml ETS (10 mM TRIS, pH 7.4, 10 mM EDTA, 0.2% SDS) and combined into a single tube. The RNA was precipitated with 0.25 M NaCl and two volumes of 95% ethanol.

The precipitate was collected by centrifugation and resuspended in 4 ml PK buffer (0.05 M TRIS, pH 8.4, 0.14 M NaCl, 0.01 M EDTA, 1% SDS). Proteinase K was added to the sample to a final concentration of 200 µg/ml. The sample was incubated at 22°C for 1 h, followed by extraction with an equal volume of phenol:chloroform:isoamylalcohol (50:48:2) two times, followed by one extraction with an equal volume of chloroform: isoamylalcohol (24:1). The RNA was precipitated with ethanol and NaCl. The precipitate was resuspended in 400 µl of ETS buffer. The yield of total RNA was approximately 1.0 mg. Poly A⁺ RNA (30 µg) was isolated from the total RNA according to standard methods as stated in Example I.A.1.

### B. Library Construction

Double-stranded cDNA was synthesized according to standard methods [see, *e.g.*, Sambrook *et al*. (1989) IN: *Molecular Cloning, A Laboratory Manual,* Cold Spring Harbor Laboratory Press, Chapter 8]. Each library was prepared in substantially the same manner except for differences in: 1) the oligonucleotide used to prime the first strand cDNA synthesis, 2) the adapters that were attached to the double-stranded cDNA, 3) the method used to remove the free or unused adapters, and 4) the size of the fractionated cDNA ligated into the λ phage vector.

### 1. IMR32 cDNA library #1

Single-stranded cDNA was synthesized using IMR32 poly(A⁺) RNA (Example I.A.1.) as a template and was primed using oligo (dT)₁₂₋₁₈ (Collaborative Research Inc., Bedford, MA). The single-stranded cDNA was converted to double-stranded cDNA and the yield was approximately 2µg. EcoI adapters: also containing *Sna*BI and *Xho*I restriction sites were then added to the double-stranded cDNA according to the following procedure.

### a. Phosphorylation of 18-mer

The 18-mer was phosphorylated using standard methods [see, *e*.*g*., Sambrook *et al.* (1989) IN: *Molecular Cloning*, *A Laboratory Manual,* Cold Spring Harbor Laboratory Press, Chapter 8] by combining in a 10 µl total volume the 18 mer (225 pmoles) with [³²P]γ-ATP (7000 Ci/mmole; 1.0 µl) and kinase (2 U) and incubating at 37° C for 15 minutes. After incubation, 1 µL 10 mM ATP and an additional 2 U of kinase were added and incubated at 37°C for 15 minutes. Kinase was then inactivated by boiling for 10 minutes.

### b. Hybridization of 22-mer

The 22-mer was hybridized to the phosphorylated 18-mer by addition of 225 pmoles of the 22-mer (plus water to bring volume to 15 µl) , and incubation at 65°C for 5 minutes. The reaction was then allowed to slow cool to room temperature.

The adapters were thus present at a concentration of 15 pmoles/µl, and were ready for cDNA-adapter ligation.

### c. Ligation of adapters to cDNA

After the *Eco*RI, *Sna*BI, *Xho*I adapters were ligated to the double-stranded cDNA using a standard protocol [see, *e.g.*, Sambrook *et al.* (1989) IN; *Molecular Cloning*, *A Laboratory Manual*, Cold Spring Harbor Laboratory Press, Chapter 8], the ligase was inactivated by heating the mixture to 72°C for 15 minutes. The following reagents were added to the cDNA ligation reaction and heated at 37°C for 30 minutes: cDNA ligation reaction (20 µl), water (24 µl), 10x kinase buffer (3 µl), 10 mM ATP (1 µl) and kinase (2µl of 2 U/µl). The reaction was stopped by the addition of 2 µl 0.5M EDTA, followed by one phenol/chloroform extraction and one chloroform extraction.

### d. Size Selection and Packaging of cDNA

The double-stranded cDNA with the EcoRI, SnaBI, XhoI adapters ligated was purified away from the free or unligated adapters using a 5 ml Sepharose CL-4B column (Sigma, St. Louis, MO). 100 µl fractions were collected and those containing the cDNA, determined by monitoring the radioactivity, were pooled, ethanol precipitated, resuspended in TE buffer and loaded onto a 1% agarose gel. After the electrophoresis, the gel was stained with ethidium bromide and the 1 to 3 kb fraction was cut from the gel. The cDNA embedded in the agarose was eluted using the "Geneluter Electroelution System" (Invitrogen, San Diego, CA). The eluted cDNA was collected by ethanol precipitation and resuspended in TE buffer at 0.10 pmol/µl. The cDNA was ligated to 1 µg of *Eco*RI digested, dephosphorylated λgt11 in a 5 µl reaction volume at a 2- to 4- fold molar excess ratio of cDNA over the λgt11 vector. The ligated λgt11 containing the cDNA insert was packaged into λ phage virions *in vitro* using the Gigapack (Stratagene, La Jolla, CA) kit. The packaged phage were plated on an *E. coli* Y1088 bacterial lawn in preparation for screening.

### 2. IMR32 cDNA library #2

This library was prepared as described (Example I.B.1.) with the exception that 3 to 9 kb cDNA fragments were ligated into the λgt11 phage vector rather than the 1 to 3 kb fragments.

### 3. IMR32 cDNA library #3

IMR32 cell poly(A⁺) RNA (Example I.A.1.) was used as a template to synthesize single-stranded cDNA. The primers for the first strand cDNA synthesis were random primers (hexadeoxy-nucleotides [pd(N)₆] Cat #5020-1, Clontech, Palo Alto, CA). The double-stranded cDNA was synthesized (Example I.B.1.), *Eco*RI, *Sna*BI, *XhoI* adapters were added to the cDNA (Example I.B.1.), the unligated adapters were removed (Example I.B.1.), and the double-stranded cDNA with the ligated adapters was fractionated on an agarose gel (Example I.B.1.). The cDNA fraction greater than 1.8 kb was eluted from the agarose (Example I.B.1.), ligated into λgt11, packaged, and plated into a bacterial lawn of Y1088 (Example I.B.1.).

### 4. IMR32 cDNA library #4

IMR32 cell poly(A⁺) RNA (Example I.A.1.) was used as a template to synthesize single-stranded cDNA. The primers for the first strand cDNA synthesis were oligonucleotides: 89-365a specific for the α_{1D} (VDCC III) type α₁-subunit (see Example II.A.) coding sequence (the complementary sequence of nt 2927 to 2956, SEQ ID No. 1), 89-495 specific for the α_{1C} (VDCC II) type α₁-subunit (see Example II.B.) coding sequence (the complementary sequence of nt 852 to 873, SEQ ID No. 3), and 90-12 specific for the α_{1C}-subunit coding sequence (the complementary sequence of nt 2496 to 2520, SEQ ID No. 3). The cDNA library was then constructed as described (Example I.B.3), except that the cDNA size-fraction greater than 1.5 kb was eluted from the agarose rather than the greater than 1.8 kb fraction.

### 5. IMR32 cDNA library #5

The cDNA library was constructed as described (Example I.B.3.) with the exception that the size-fraction greater than 1.2 kb was eluted from the agarose rather than the greater than 1.8 kb fraction.

### 6. Human thalamus cDNA library #6

Human thalamus poly (A⁺) RNA (Example I.A.2.) was used as a template to synthesize single-stranded cDNA. Oligo (dT) was used to prime the first strand synthesis (Example I.B.1.). The double-stranded cDNA was synthesized (Example I.B.1.) and *Eco*RI, *Kpn*I, *Nco*I adapters of the following sequence: were ligated to the double-stranded cDNA as described (Example I.B.1.) with the 20-mer replacing the 18-mer and the 24-mer replacing the 22-mer. The unligated adapters were removed by passing the cDNA-adapter mixture through a 1 ml Bio Gel A-50 (Bio-Rad Laboratories, Richmond, CA.) column. Fractions (30 µl) were collected and 1 µl of each fraction in the first peak of radioactivity was electrophoresed on a 1% agarose gel. After electrophoresis, the gel was dried on a vacuum gel drier and exposed to x-ray film. The fractions containing cDNA fragments greater than 600 bp were pooled, ethanol precipitated, and ligated into λgt11 (Example I.B.1.). The construction of the cDNA library was completed as described (Example I.B.1.).

### C. Hybridization and Washing Conditions

Hybridization of radiolabelled nucleic acids to immobilized DNA for the purpose of screening cDNA libraries, DNA Southern transfers, or northern transfers was routinely performed in standard hybridization conditions [5 x SSPE, 5x Denhardt's, 50% deionized formamide, 200 µg/ml sonicated herring sperm DNA (Cat #223646, Boehringer Mannheim Biochemicals, Indianapolis, IN)]. The recipes for SSPE and Denhardt's and the preparation of deionized formamide are described, for example, in Sambrook *et al.* (1989*) Molecular Cloning, A Laboratory Manual,* Cold Spring Harbor Laboratory Press, Chapter 8). In some hybridizations, lower stringency conditions were used in that 10% deionized formamide replaced 50% deionized formamide described for the standard hybridization conditions.

The washing conditions for removing the non-specific probe from the filters was either high, medium, or low stringency as described below:

| | | |
|---|---|---|
| 1) | high stringency | 0.1 x SSPE, 0.1% SDS, 65°C |
| 2) | medium stringency | 0.2 x SSPE, 0.1% SDS, 50°C |
| 3) | low stringency | 1.0 x SSPE, 0.1% SDS, 50°C. |

It is understood that equivalent stringencies may be achieved using alternative buffers, salts and temperatures.

### EXAMPLE II: ISOLATION OF DNA ENCODING THE HUMAN NEURONAL CALCIUM CHANNEL α_{I} SUBUNIT

### A. Isolation of DNA encoding the α_{ID} subunit

### 1. Reference list of partial α_{ID} CDEA clones

Numerous α_{1D}-specific cDNA clones were isolated in order to characterize the complete α_{1D} coding sequence plus portions of the 5' and 3' untranslated sequences. SEQ ID No. 1 shows the complete α_{1D} DNA coding sequence, plus 510 nucleotides of α_{1D} 5' untranslated sequence ending in the guanidine nucleotide adjacent to the adenine nucleotide of the proposed initiation of translation as well as 642 nucleotides of 3' untranslated sequence. Also shown in SEQ ID No. 1 is the deduced amino acid sequence. A list of partial cDNA clones used to characterize the α_{1D} sequence and the nucleotide position of each clone relative to the full-length α_{1D} cDNA sequence, which is set forth in SEQ ID No. 1, is shown below. The isolation and characterization of these clones are described below (Example II.A.2.).

| | | |
|---|---|---|
| IMR32 | 1.144 | nt. 1 to 510 of SEQ ID No. 1 5' untranslated sequence, nt. 511 to 2431, SEQ ID No.1 |
| IMR32* | 1.136 | nt. 1627 to 2988, SEQ ID No. 1 nt. 1 to 104 of SEQ ID No. 2 additional exon, |
| IMR32@ | 1.80 | nt. 2083 to 6468, SEQ ID No. 1 |
| IMR32^{#} | 1.36 | nt. 2857 to 4281, SEQ ID No. 1 |
| IMR32 | 1.163 | nt. 5200 to 7635, SEQ ID No. 1 |

| | | |
|---|---|---|
| * 5' of nt 1627, IMR32 1.136 encodes an intron and an additional exon described in Example II.A.2.d. | | |
| @ IMR32 1.80 contains two deletions, nt 2984 to 3131 and nt 5303 to 5349 (SEQ ID No. 1). The 148 nt deletion (nt. 2984 to 3131) was corrected by performing a polymerase chain reaction described in Example II.A.3.b. | | |
| # IMR32 1.36 contains a 132 nt deletion (nt. 3081 to 3212). | | |

### 2. Isolation and characterization of individual clones listed in Example II.A.1.

### a. IMR32 1.36

Two million recombinants of the IMR32 cDNA library #1 (Example I.B.1.) were screened in duplicate at a density of approximately 200,000 plaques per 150 mm plate using a mixture of radiolabelled fragments of the coding region of the rabbit skeletal muscle calcium channel α₁ cDNA [for the sequence of the rabbit skeletal muscle calcium channel α₁ subunit cDNA, see, Tanabe *et al.* (1987). *Nature 328:*313-318]:

| **Frequent** | **Nucleotides** |
|---|---|
| *Kpn*I-*Eco*RI | -78 to 1006 |
| *Eco*RI-*Xho*I | 1006 to 2653 |
| *Apa*I-*Apa*I | 3093 to 4182 |
| *Bgl*II-*Sac*I | 4487 to 5310 |

The hybridization was performed using low stringency hybridization conditions (Example I.C.) and the filters were washed under low stringency (Example I.C.). Only one α_{1D}-specific recombinant (IMR32 1.36) of the 2 x 10⁶ screened was identified. IMR32 1.36 was plaque purified by standard methods (J. Sambrook *et al.* (1989) *Molecular Cloning, A Laboratory Manual,* Cold Spring Harbor Laboratory Press, Chapter 8) subcloned into pGEM3 (Promega, Madison, WI) and characterized by DNA sequencing.

### b. IMR32 1.80

Approximately 1 x 10⁶ recombinants of the IMR32 cDNA library #2 (Example I.B.2.) were screened in duplicate at a density of approximately 100,000 plaques per 150 mm plate using the IMR32 1.36 cDNA fragment (Example II.A.1) as a probe. Standard hybridization conditions were used (Example I.C), and the filters were washed under high stringency (Example I.C.). Three positive plaques were identified one of which was IMR32 1.80. IMR32 1.80 was plaque purified by standard methods, restriction mapped, subcloned, and characterized by DNA sequencing.

### c. IMR32 1.144

Approximately 1 x 10⁶ recombinants of the IMR32 cDNA library #3 (Example I.B.3) were screened with the EcoRI-PvuII fragment (nt 2083 to 2518, SEQ ID No. 1) of IMR32 1.80. The hybridization was performed using standard hybridization conditions (Example I.C.) and the filters were washed under high stringency (Example I.C.). Three positive plaques were identified one of which was IMR32 1.144. IMR32 1.144 was plaque purified, restriction mapped, and the cDNA insert was subcloned into pGEM7Z (Promega, Madison, WI) and characterized by DNA sequencing. This characterization revealed that IMR32 1.144 has a series of ATG codons encoding seven possible initiating methionines (nt 511 to 531, SEQ ID No. 1). PCR analysis, and DNA sequencing of cloned PCR products encoding these seven ATG codons confirmed that this sequence is present in the α_{1D} transcript expressed in dbcAMP-induced IMR32 cells.

### d. IMR32 1.136

Approximately 1 x 10⁶ recombinants of the IMR32 cDNA library #4 (Example I.B.4) were screened with the *Eco*RI-*Pvu*II fragment (nt 2083 to 2518, SEQ ID No. 1) of IMR32 1.80 (Example II.A.1.). The hybridization was performed using standard hybridization conditions (Example I.C.) and the filters were washed under high stringency (Example I.C.). Six positive plaques were identified one of which was IMR32 1.136. IMR32 1.136 was plaque purified, restriction mapped, and the cDNA insert was subcloned into a standard plasmid vector, pSP72 (Promega, Madison, WI.), and characterized by DNA sequencing. This characterization revealed that IMR32 1.136 encodes an incompletely spliced α_{1D} transcript. The clone contains nucleotides 1627 to 2988 of SEQ ID No. 1 preceded by an approximate 640 bp intron. This intron is then preceded by a 104 nt exon (SEQ ID No. 2) which is an alternative exon encoding the IS6 transmembrane domain [see, *e.g.,* Tanabe *et al.* (1987) Nature 328:313-318 for a description of the IS1 to IVS6 transmembrane terminology] of the α_{1D} subunit and can replace nt 1627 to 1730, SEQ ID No. 1, to produce a completely spliced α_{1D} transcript.

### e. IMR32 1.163

Approximately 1 x 10⁶ recombinants of the IMR32 cDNA library #3 (Example I.B.3.) were screened with the *Nco*I-*Xho*I fragment of IMR32 1.80 (Example II.A.1.) containing nt 5811 to 6468 (SEQ ID No. 1). The hybridization was performed using standard hybridization conditions (Example I.C.) and the filters were washed under high stringency (Example I.C.). Three positive plaques were identified one of which was IMR32 1.163. IMR32 1.163 was plaque purified, restriction mapped, and the cDNA insert was subcloned into a standard plasmid vector, pSP72 (Promega, Madison, WI.), and characterized by DNA sequencing. This characterization revealed that IMR32 1.163 contains the α_{1D} termination codon, nt 6994 to 6996 (SEQ ID No. 1).

### 3. Construction of a full-length α_{1D} cDNA [pVDCCIII(A)]

α_{1D} cDNA clones IMR32 1.144, IMR32 1.136, IMR32 1.80, and IMR32 1.163 (Example II.A.2.) overlap and include the entire α_{1D} coding sequence, nt 511 to 6993 (SEQ ID No. 1), with the exception of a 148 bp deletion, nt 2984 to 3131 (SEQ ID No. 1). Portions of these partial cDNA clones were ligated to generate a full-length α_{1D} cDNA in a eukaryotic expression vector. The resulting vector was called pVDCCIII(A). The construction of pVDCCIII(A) was performed in four steps described in detail below: (1) the construction of pVDCCIII/5' using portions of IMR32 1.144, IMR32 1.136, and IMR32 1.80, (2) the construction of pVDCCIII/5'.3 that corrects the 148 nt deletion in the IMR32 1.80 portion of pVDCCIII/5', (3) the construction of pVDCCIII/3'.1 using portions of IMR32 1.80 and IMR32 1.163, and (4) the ligation of a portion of the pVDCCIII/5'.3 insert, the insert of pVDCCIII/3'.1, and pcDNA1 (Invitrogen, San Diego, CA) to form pVDCCIII(A). The vector pcDNA1 is a eukaryotic expression vector containing a cytomegalovirus (CMV) promoter which is a constitutive promoter recognized by mammalian host cell RNA polymerase II.

Each of the DNA fragments used in preparing the full-length construct was purified by electrophoresis through an agarose gel onto DE81 filter paper (Whatman, Clifton, NJ) and elution from the filter paper using 1.0 M NaCl, 10 mM TRIS, pH 8.0, 1 mM EDTA. The ligations typically were performed in a 10 µl reaction volume with an equal molar ratio of insert fragment and a two-fold molar excess of the total insert relative to the vector. The amount of DNA used was normally about 50 ng to 100 ng.

### a. pVDCCIII/5'

To construct pVDCCIII/5', IMR32 1.144 (Example II.A.2.c.) was digested with *Xho*I and *Eco*RI and the fragment containing the vector (pGEM7Z), α_{1D} nt 1 to 510 (SEQ ID No. 1), and α_{1D} nt 511 to 1732 (SEQ ID No. 1) was isolated by gel electrophoresis. The *Eco*RI-*Apa*I fragment of IMR32 1.136 (Example II.A.2.d.) nucleotides 1732 to 2667 (SEQ ID No. 1) was isolated, and the *Apa*I-*Hind*III fragment of IMR32 1.80 (Example II.A.2.b.), nucleotides 2667 to 4492 (SEQ ID No. 1) was isolated. The three DNA clones were ligated to form pVDCCIII/5' containing nt 1 to 510 (5' untranslated sequence; SEQ ID No. 1) and nt 511 to 4492 (SEQ ID No. 1).

### b. pVDCCIII/5'.3

Comparison of the IMR32 1.36 and IMR32 1.80 DNA sequences revealed that these two cDNA clones differ through the α_{1D} coding sequence, nucleotides 2984 to 3212. PCR analysis of IMR32 1.80 and dbcAMP-induced (1.0 mM, 10 days) IMR32 cytoplasmic RNA (isolated according to Ausubel, F.M. *et al.* (Eds) (1988) *Current Protocols in Molecular Biology,* John Wiley and Sons, New York) revealed that IMR32 1.80 had a 148 nt deletion, nt 2984 to 3131 (SEQ ID No. 1), and that IMR32 1.36 had a 132 nt deletion, nt 3081 to 3212. To perform the PCR analysis, amplification was primed with α_{1D}-specific oligonucleotides 112 (nt 2548 to 2572, SEQ ID No. 1) and 311 (the complementary sequence of nt 3928 to 3957, SEQ ID No. 1). These products were then reamplified using α_{1D}-specific oligonucleotides 310 (nt 2583 to 2608 SEQ ID No. 1) and 312 (the complementary sequence of nt 3883 to 3909). This reamplified product, which contains AccI and BgIII restriction sites, was digested with AccI and BgIII and the AccI-BgIII fragment, nt 2764 to 3890 (SEQ ID No. 1) was cloned into AccI-BgIII digested pVDCCIII/5' to replace the AccI-BglII pVDCCIII/5' fragment that had the deletion. This new construct was named pVDCCIII/5'.3. DNA sequence determination of pVDCCIII/5'.3 through the amplified region confirmed the 148 nt deletion in IMR32 1.80.

### c. pVDCCIII/3'.1

To construct pVDCCIII/3'.1, the cDNA insert of IMR32 1.163 (Example II.A.2.e.) was subcloned into pBluescript II (Stratagene, La Jolla, CA) as an XhoI fragment. The XhoI sites on the cDNA fragment were furnished by the adapters used to construct the cDNA library (I.B.3.). The insert was oriented such that the translational orientation of the insert of IMR32 1.163 was opposite to that of the *lacZ* gene present in the plasmid, as confirmed by analysis of restriction enzyme digests of the resulting plasmid. This was done to preclude the possibility of expression of α_{1D} sequences in DH5α cells transformed with this plasmid due to fusion with the *lacZ* gene. This plasmid was then digested with *Hin*dIII and *Bgl*II and the *Hin*dIII - *Bgl*II fragment (the *Hin*dIII site comes from the vector and the Bg1II site is at nt 6220, SEQ ID No. 1) was eliminated, thus deleting nt 5200 to 6220 (SEQ ID No. 1) of the IHR32 1.163 clone and removing this sequence from the remainder of the plasmid which contained the 3' *Bgl*II - *Xho*l fragment, nt 6220 to 7635 (SEQ ID No. 1). pVDCCIII/3'.1 was then made by splicing together the *Hin*dIII-*Pvu*II fragment from IMR32 1.80 (nucleotides 4492-5294, SEQ ID No. 1), the PvuII - BglII fragment of IMR32 1.163 (nucleotides 5294 to 6220, SEQ ID No. 1) and the HindIII-BglII-digested pBluescript plasmid containing the 3' *Bgl*II/*Xho*I IMR32 1.163 fragment (nt 6220 to 7635, SEQ ID No. 1).

### d. pVDCCIII(A): the full-length α_{ID} construct

. To construct pVDCCIII(A), the *Dra*I-*Hin*dIII fragment (5' untranslated sequence nt 327 to 510, SEQ ID No. 1 and coding sequence nt 511 to 4492, SEQ ID No. 1) of pVDCCIII/5'.3 (Example II.A.3.b.) was isolated; the *Hin*dIII-*Xho*I fragment of pVDCCIII/3'.1 (containing nt 4492 to 7635, SEQ ID No. 1, plus the *Xho*I site of the adapter) (Example II.A.3.c.) was isolated; and the plasmid vector, pcDNA1, was digested with *Eco*RV and *Xho*I and isolated on an agarose gel. The three DNA fragments were ligated and MC1061-P3 (Invitrogen, San Diego, CA) was transformed. Isolated clones were analyzed by restriction mapping and DNA sequencing and pVDCCIII(A) was identified which had the fragments correctly ligated together: DraI-HindIII, *Hin*dIII-*Xho*I, *Xbo*I-*Eco*RV with the blunt-end *Dra*I and *Eco*RV site ligating together to form the circular plasmid.

The amino-terminus of the α_{1D} subunit is encoded by the seven consecutive 5' methionine codons (nt 511 to 531, SEQ ID No. 1). This 5' portion plus nt 532 to 537, encoding two lysine residues, were deleted from pVDCCIII(A) and replaced with an efficient ribosomal binding site (5'-ACCACC-3') to form pVDCCIII.RBS(A). Expression experiments in which transcripts of this construct were injected into *Xenopus laevis* cöcytes did not result in an enhancement in the recombinant voltage-dependent calcium channel expression level relative to the level of expression in oöcytes injected with transcripts of pVDCCIII(A).

### B. Isolation of DNA encoding the α_{1C} subunit

### 1. Reference List of Partial α_{1C} cDNA clones

Numerous α_{1C}-specific cDNA clones were isolated in order to characterize the α_{1C} coding sequence, the α_{1C} initiation of translation, and an alternatively spliced region of α_{1C}. SEQ ID No. 3 sets forth the characterized α_{1C} coding sequence (nt 1 to 5904) and deduced amino acid sequence. SEQ ID No. 4 and No. 5 encode two possible amino terminal ends of the α_{1C} protein. SEQ ID No. 6 encodes an alternative exon for the IV S3 transmembrane domain. Shown below is a list of clones used to characterize the α_{1C} sequence and the nucleotide position of each clone relative to the characterized α_{1C} sequence (SEQ ID No. 3). The isolation and characterization of these cDNA clones are described below (Example II.B.2).

| | | |
|---|---|---|
| **IHR32** | 1.66 | nt 1 to 916, SEQ ID No. 3 nt 1 to 132, SEQ ID No. 4 |
| **IHR32** | 1.157 | nt 1 to 873, SEQ ID No. 3 nt 1 to 89, SEQ ID No. 5 |
| **IMR32** | 1.67 | nt 50 to 1717, SEQ ID No. 3 |
| ***IMR32** | 1.86 | nt 1366 to 2583, SEQ ID No. 3 |
| **@1.16G** | | nt 758 to 867, SEQ ID No. 3 |
| **IHR32** | 1.37 | nt 2804 to 5904, SEQ ID No. 3 |
| **CNS** | 1.30 | nt 2199 to 3903, SEQ ID No. 3 nt 1 to 84 of alternative exon, SEQ ID No. 6 |
| **IMR32** | 1.38 | nt 2448 to 4702, SEQ ID No. 3 |
| | | nt 1 to 84 of alternative exon, SEQ ID No. 6 |

| | | |
|---|---|---|
| * IMR32 1.86 has a 73 nt deletion compared to the rabbit cardiac muscle calcium channel α₁ subunit cDNA sequence. | | |
| ^{@}1. 16G is an α_{1C} genomic clone. | | |

### 2. Isolation and characterization of clones described in Example II.B.1.

### a. CNS 1.30

Approximately 1x 10⁶ recombinants of the human thalamus cDNA library No. 6 (Example I.B.6.) were screened with fragments of the rabbit skeletal muscle calcium channel α₁ cDNA described in Example II.A.2.a. The hybridization was performed using standard hybridization conditions (Example I.C.) and the filters were washed under low stringency (Example I.C.). Six positive plaques were identified, one of which was CNS 1.30. CNS 1.30 was plaque purified, restriction mapped, subcloned, and characterized by DNA sequencing. CNS 1.30 encodes α_{1C}-specific sequence nt 2199 to 3903 (SEQ ID No. 3) followed by nt 1 to 84 of one of two identified alternative α_{1C} exons (SEQ ID No. 6). 3' of SEQ ID No. 6, CNS 1.30 contains an intron and, thus, CNS 1.30 encodes a partially spliced α_{1C} transcript.

### b. 1.16G

Approximately 1x 10⁶ recombinants of a λEMBL3-based human genomic DNA library (Cat # HL1006d Clontech Corp., Palo Alto, CA) were screened using a rabbit skeletal muscle cDNA fragment (nt -78 to 1006, Example II.A.2.a.). The hybridization was performed using standard hybridization conditions (Example I.C.) and the filters were washed under low stringency (Example I.C.). Fourteen positive plaques were identified, one of which was 1.16G. Clone 1.16G was plaque purified, restriction mapped, subcloned, and portions were characterized by DNA sequencing. DNA sequencing revealed that 1.16G encodes α_{1C}-specific sequence as described in Example II.B.1.

### c. IMR32 1.66 and IMR32 1.67

Approximately 1x 10⁶ recombinants of IMR32 cDNA library #5 (Example I.B.5.) were screened with a 151 bp *Kpn*I*-Sac*I fragment of 1.16G (Example II.B.2.b.) encoding α_{1C} sequence (nt 758 to 867, SEQ ID No. 3). The hybridization was performed using standard hybridization conditions (Example I.C.). The filters were then washed in 0.5 x SSPE at 65°C. Of the positive plaques, IMR32 1.66 and IMR32 1.67 were identified. The hybridizing plaques were purified, restriction mapped, subcloned, and characterized by DNA sequencing. Two of these cDNA clones, IMR32 1.66 and 1.67, encode α_{1C} subunits as described (Example II.B.1.). In addition, IMR32 1.66 encodes a partially spliced α_{1C} transcript marked by a GT splice donor dinucleotide beginning at the nucleotide 3' of nt 916 (SEQ ID No. 3). The intron sequence within 1.66 is 101 nt long. IMR32 1.66 encodes the α_{1C} initiation of translation, nt 1 to 3 (SEQ ID No. 3) and 132 nt of 5' untranslated sequence (SEQ ID No. 4) precede the start codon in IMR32 1.66.

### d. IMR32 1.37 and IMR32 1.38

Approximately 2 x 10⁶ recombinants of IMR32 cDNA library #1 (Example I.B.1.) were screened with the CNS 1.30 cDNA fragment (Example II.B.2.a.). The hybridization was performed using low stringency hybridization conditions (Example I.C.) and the filters were washed under low stringency (Example I.C.). Four positive plaques were identified, plaque purified, restriction mapped, subcloned, and characterized by DNA sequencing. Two of the clones, IMR32 1.37 and IMR32 1.38 encode α_{1C}-specific sequences as described in Example II.B.1.

DNA sequence comparison of IMR32 1.37 and IMR32 1.38 revealed that the α_{1C} transcript includes two exons that encode the IVS3 transmembrane domain. IMR32 1.37 has a single exon, nt 3904 to 3987 (SEQ ID No. 3) and IMR32 1.38 appears to be anomalously spliced to contain both exons juxtaposed, nt 3904 to 3987 (SEQ ID No. 3) followed by nt 1 to 84 (SEQ ID No. 6). The alternative splice of the α_{1C} transcript to contain either of the two exons encoding the IVS3 region was confirmed by comparing the CNS 1.30 sequence to the IMR32 1.37 sequence. CNS 1.30 contains nt 1 to 84 (SEQ ID No. 6) preceded by the identical sequence contained in IMR32 1.37 for nt 2199 to 3903 (SEQ ID No. 3). As described in Example II.B.2.a., an intron follows nt 1 to 84 (SEQ ID No. 6). Two alternative exons have been spliced adjacent to nt 3903 (SEQ ID No. 3) represented by CNS 1.30 and IMR32 1.37.

### e. IMR32 1.86

IMR32 cDNA library #1 (Example I.B.1.) was screened in duplicate using oligonucleotide probes 90-9 (nt 1462 to 1491, SEQ ID No. 3) and 90-12 (nt 2496 to 2520, SEQ ID No. 3). These oligonucleotide probes were chosen in order to isolate a clone that encodes the α_{1C} subunit between the 3' end of IMR32 1.67 (nt 1717, SEQ ID No. 3) and the 5' end of CNS 1.30 (nt 2199, SEQ ID No. 3). The hybridization conditions were standard hybridization conditions (Example I.C.) with the exception that the 50% deionized formamide was reduced to 20%. The filters were washed under low stringency (Example I.C.). Three positive plaques were identified one of which was IMR32 1.86. IMR32 1.86 was plaque purified, subcloned, and characterized by restriction mapping and DNA sequencing. IMR32 1.86 encodes α_{1C} sequences as described in Example II.B.1. Characterization by DNA sequencing revealed that IMR32 1.86 contains a 73 nt deletion compared to the DNA encoding rabbit cardiac muscle calcium channel α₁ subunit [Mikami *et al.* (1989) *Nature 340*:230), nt 2191 to 2263. These missing nucleotides correspond to nt 2176-2248 of SEQ ID No. 3. Because the 5'-end of CNS 1.30 overlaps the 3'-end of IMR32 1.86, some of these missing nucleotides, i.e., nt 2205-2248 of SEQ ID No. 3, are accounted for by CNS 1.30. The remaining missing nucleotides of the 73 nucleotide deletion in IMR32 1.86 (i.e., nt 2176-2204 SEQ ID No. 3) were determined by PCR analysis of dbcAMP-induced IMR32 cell RNA. The 73 nt deletion is a frame-shift mutation and, thus, needs to be corrected. The exact human sequence through this region, (which has been determined by the DNA sequence of CNS 1.30 and PCR analysis of IMR32 cell RNA) can be inserted into IMR32 1.86 by standard methods, *e.g.*, replacement of a restriction fragment or site-directed mutagenesis.

### f. IMR32 1.157

one million recombinants of IMR32 cDNA library #4 (Example I.B.4.) were screened with an XhoI-EcoRI fragment of IMR32 1.67 encoding α_{1C} nt 50 to 774 (SEQ ID No. 3). The hybridization was performed using standard hybridization conditions (Example I.C.). The filters were washed under high stringency (Example I.C.). One of the positive plaques identified was IMR32 1.157. This plaque was purified, the insert was restriction mapped and subcloned to a standard plasmid vector pGEM7Z (Promega, Madison, WI). The DNA was characterized by sequencing. IMR32 1.157 appears to encodes an alternative 5' portion of the α_{1C} sequence beginning with nt 1 to 89 (SEQ ID No. 5) and followed by nt 1 to 873 (SEQ ID No. 3). Analysis of the 1.66 and 1.157 5' sequence is described below (Example II.B.3.).

### 3. Characterization of the α_{1C} initiation of translation site

Portions of the sequences of IMR32 1.157 (nt 57 to 89, SEQ ID No. 5; nt 1 to 67, SEQ ID No. 3), IMR32 1.66 (nt 100 to 132, SEQ ID No. 4; nt 1 to 67, SEQ ID No. 3), were compared to he rabbit lung CaCB-receptor cDNA sequence, nt -33 to 67 [Biel *et al.* (1990) *FEBS Lett*. *269*:409]. The human sequences are possible alternative 5' ends of the α_{1C} transcript encoding the region of initiation of translation. IMR32 1.66 closely matches the CaCB receptor cDNA sequence and diverges from the CaCB receptor cDNA sequence in the 5' direction beginning at nt 122 (SEQ ID No. 4). The start codon identified in the CaCB receptor cDNA sequence is the same start codon used to describe the α_{1C} coding sequence, nt 1 to 3 (SEQ ID No. 3). The functional significance of the IMR32 1.157 sequence, nt 1 to 89 (SEQ ID No. 5), is not clear. Chimeras containing sequence between 1.157 and the α_{1C} coding sequence can be constructed and functional differences can be tested.

### C. Isolation of partial cDNA clones encoding the α_{1B} subunit and construction of a full-length clone

A human basal ganglia cDNA library was screened with the rabbit skeletal muscle α₁ subunit cDNA fragments (see Example II.A.2.a for description of fragments) under low stringency conditions. One of the hybridizing clones was used to screen an IMR32 cell cDNA library to obtain additional partial α_{1B} cDNA clones, which were in turn used to further screen an IMR32 cell cDNA library for additional partial cDNA clones. One of the partial IMR32 α_{1B} clones was used to screen a human hippocampus library to obtain a partial α_{1B} clone encoding the 3' end of the α_{1B} coding sequence. The sequence of some of the regions of the partial cDNA clones was compared to the sequence of products of PCR analysis of IMR32 cell RNA to determine the accuracy of the cDNA sequences.

PCR analysis of IMR32 cell RNA and genomic DNA using oligonucleotide primers corresponding to sequences located 5' and 3' of the STOP codon of the DNA encoding the α_{1B} subunit revealed an alternatively spliced α_{1B}-encoding mRNA in IMR32 cells. This second mRNA product is the result of differential splicing of the α_{1B} subunit transcript to include another exon that is not present in the mRNA corresponding to the other 3' α_{1B} cDNA sequence that was initially isolated. To distinguish these splice variants of the α_{1B} subunit, the subunit encoded by a DNA sequence corresponding to the form containing the additional exon is referred to as α_{1B-1} (SEQ ID No. 7), whereas the subunit encoded by a DNA sequence corresponding to the form lacking the additional exon is referred to as α_{1B-2} (SEQ ID No. 8). The sequence of α_{1B-1} diverges from that of α_{1B-2} beginning at nt 6633 (SEQ ID No. 7). Following the sequence of the additional exon in α_{1B-1}(nt 6633-6819; SEQ ID No. 7), the α_{1B-1} and α_{1B-2} sequences are identical (i.e., nt 6820-7362 in SEQ ID No. 7 and nt 6633-7175 in SEQ ID No. 8). SEQ ID No. 7 and No. 8 set forth 143 nt of 5' untranslated sequence (nt 1-143) as well as 202 nt of 3' untranslated sequence (nt 7161-7362, SEQ ID No. 7) of the DNA encoding α_{1B-1} and 321 nt of 3' untranslated sequence (nt 6855-7175, SEQ ID No. 8) of the DNA encoding α_{1B-2}.

PCR analysis of the IS6 region of the α_{1B} transcript revealed what appear to be additional splice variants based on multiple fragment sizes seen on an ethidium bromide-stained agarose gel containing the products of the PCR reaction.

A full-length α_{1B-1} cDNA clone designated pcDNA-α_{1B-1} was prepared in an eight-step process as follows.
- STEP 1:: The *Sac*I restriction site of pGEM3 (Promega, Madison, WI) was destroyed by digestion at the *Sac*I site, producing blunt ends by treatment with T4 DNA polymerase, and religation. The new vector was designated pGEMΔSac.
- STEP 2:: Fragment 1 (*Hind*III/*Kpn*I; nt 2337 to 4303 of SEQ ID No. 7) was ligated into *Hind*III/*Kpn*I digested pGEM3ΔSac to produce pα1.177HK.
- STEP 3:: Fragment 1 has a 2 nucleotide deletion (nt 3852 and 3853 of SEQ ID No. 7). The deletion was repaired by inserting a PCR fragment (fragment 2) of IMR32 RNA into pα1.177HK. Thus, fragment 2 (*Nar*I/*Kpn*I; nt 3828 to 4303 of SEQ ID No. 7) was inserted into *Nar*I/*Kpn*I digested pα1.177HK replacing the *Nar*I/*Kpn*I portion of fragment 1 and producing pα1.177HK/PCR.
- STEP 4:: Fragment 3 (*Kpn*I/*Kpn*I; nt 4303 to 5663 of SEQ ID No. 7) was ligated into *Kpn*I digested pα1.177HK/PCR to produce pα1B5'K.
- STEP 5:: Fragment 4 (*Eco*RI/*Hin*dIII; *Eco*RI adaptor plus nt 1 to 2337 of SEQ ID No. 7) and fragment 5 (*Hind*III/*Xho*I fragment of pα1B5'K; nt 2337 to 5446 of SEQ ID No. 7) were ligated together into *Eco*RI/*Xho*I digested pcDNA1 (Invitrogen, San Diego, CA) to produce pα1B5'.
- STEP 6:: Fragment 6 (*Eco*RI/*Eco*RI; *Eco*RI adapters on both ends plus nt 5749 to 7362 of SEQ ID No. 7) was ligated into *Eco*RI digested pBluescript II KS (Stratagene, La Jolla, CA) with the 5' end of the fragment proximal to the *Kpn*I site in the polylinker to produce pα1.230.
- STEP 7:: Fragment 7 (*Kpn*I/*Xho*I; nt 4303 to 5446 of SEQ ID No. 7), and fragment 8 (*Xho*I/*Csp*I; nt 5446 to 6259 of SEQ ID No. 7) were ligated into *Kpn*I/*Csp*I digested pα1.230 (removes nt 5749 to 6259 of SEQ ID No. 7 that was encoded in pα1.230 and maintains nt 6259 to 7362 of SEQ ID No. 7) to produce pα1B3'.
- STEP 8:: Fragment 9 (SphI/XhoI; nt 4993 to 5446 of SEQ ID No. 7) and fragment 10 (*Xho*I/*Xba*I of pα1B3'; nt 5446 to 7319 of SEQ ID No. 7) were ligated into *Sph*I/*Xba*I digested pα1B5' (removes nt 4993 to 5446 of SEQ ID No. 7 that were encoded in pα1B5' and maintains nt 1 to 4850 of SEQ ID No. 7) to produce PCDNAα_{1B-1}.

The resulting construct, PCDNAα_{1B-1}, contains, in pCDNA1, a full-length coding region encoding α_{1B-1} (nt 144-7362, SEQ ID No. 7), plus 5' untranslated sequence (nt 1-143, SEQ ID No. 7) and 3' untranslated sequence (nt 7161-7319, SEQ ID No. 7) under the transcriptional control of the CMV promoter.

### EXAMPLE III: ISOLATION OF cDNA CLONES ENCODING THE HUMAN NEURONAL CALCIUM CHANNEL β₁ subunit

### A. Isolation of partial cDNA clones encoding the β subunit and construction of a full-length clone encoding the β₁ subunit

A human hippocampus cDNA library was screened with the rabbit skeletal muscle calcium channel β₁ subunit cDNA fragment (nt 441 to 1379) [for isolation and sequence of the rabbit skeletal muscle calcium channel β₁ subunit cDNA, see U.S. Patent Application Serial NO. 482,384 or Ruth *et al.* (1989) *Science 245*:1115] using standard hybridization conditions (Example I.C.). A portion of one of the hybridizing clones was used to rescreen the hippocampus library to obtain additional cDNA clones. The cDNA inserts of hybridizing clones were characterized by restriction napping and DNA sequencing and compared to the rabbit skeletal muscle calcium channel β₁ subunit cDNA sequence.

Portions of the partial β₁ subunit cDNA clones were ligated to generate a full-length clone encoding the entire β₁ subunit. SEQ ID No. 9 shows the β₁ subunit coding sequence (nt 1-1434) as well as a portion of the 3' untranslated sequence (nt 1435-1546). The deduced amino acid sequence is also provided in SEQ ID No. 9. In order to perform expression experiments, full-length β₁ subunit cDNA clones were constructed as follows.

Step 1: DNA fragment 1 (~800 bp of 5' untranslated sequence plus nt 1-277 of SEQ ID No. 9) was ligated to DNA fragment 2 (nt 277-1546 of SEQ ID No. 9 plus 448 bp of intron sequence) and cloned into pGEM7Z. The resulting plasmid, pβ1-1.18, contained a full-length β₁ subunit clone that included a 448-bp intron.

Step 2: To replace the 5' untranslated sequence of pβ1-1.18 with a ribosome binding site, a double-stranded adapter was synthesized that contains an *Eco*RI site, sequence encoding a ribosome binding site (5'-ACCACC-3') and nt 1-25 of SEQ ID No. 9. The adapter was ligated to *Sma*I-digested pβ1-1.18, and the products of the ligation reaction were digested with EcoRI.

Step 3: The EcoRI fragment from step 2 containing the EcoRI adapter, efficient ribosome binding site and nt 1-1546 of SEQ ID No. 9 plus intron sequence was cloned into a plasmid vector and designated pβ1-1.18RBS. The *Eco*RI fragment of pβ1-1.18RBS was subcloned into *Eco*RI-digested pcDNA1 with the initiation codon proximal to CMV promoter to form pHBCaCHβ₁ₐRBS(A).

Step 4: To generate a full-length clone encoding the β₁ subunit lacking intron sequence, DNA fragment 3 (nt 69-1146 of SEQ ID No. 9 plus 448 bp of intron sequence followed by nt 1147-1546 of SEQ ID No. 9), was subjected to site-directed mutagenesis to delete the intron sequence, thereby yielding pβ1(-). The *Eco*RI-*Xho*I fragment of pβ1-1.18RBS (containing of the ribosome binding site and nt 1-277 of SEQ ID No. 9) was ligated to the *Zho*I-*Eco*RI fragment of pβ1(-) (containing of nt 277-1546 of SEQ ID No. 9) and cloned into pcDNA1 with the initiation of translation proximal to the CMV promoter. The resulting expression plasmid was designated pHBCaCHβ_{1b}RBS(A).

### B. Splice Variant β₁₋₃

DNA sequence analysis of the DNA clones encoding the β₁ subunit indicated that in the CNS at least two alternatively spliced forms of the same human β₁ subunit primary transcript are expressed. One form is represented by the sequence shown in SEQ ID No. 9 and is referred to as β₁₋₂. The sequences of β₁₋₂ and the alternative form, β₁₋₃, diverge at nt 1334 (SEQ ID No. 9). The complete β₁₋₃ sequence (nt 1-1851), including 3' untranslated sequence (nt 1795-1851), is set forth in SEQ ID No. 10.

### EXAMPLE IV: ISOLATION OF cDNA CLONES ENCODING THE HUMAN NEURONAL CALCIUM CHANNEL α₂-subunit

### A. Isolation of cDNA clones

The complete human neuronal α₂ coding sequence (nt 35-3307) plus a portion of the 5' untranslated sequence (nt 1 to 34) as well as a portion of the 3' untranslated sequence (nt 3308-3600) is set forth in SEQ ID No. 11.

To isolate DNA encoding the human neuronal α₂ subunit, human α₂ genomic clones first were isolated by probing human genomic Southern blots using a rabbit skeletal muscle calcium channel α₂ subunit cDNA fragment [nt 43 to 272, Ellis *et al.* (1988) *Science 240*:1661]. Human genomic DNA was digested with EcoRI, electrophoresed, blotted, and probed with the rabbit skeletal muscle probe using standard hybridization conditions (Example I.C.) and low stringency washing conditions (Example I.C.). Two restriction fragments were identified, 3.5 kb and 3.0 kb. These EcoRI restriction fragments were cloned by preparing a λgt11 library containing human genomic *Eco*RI fragments ranging from 2.2 kb to 4.3 kb. The library was screened as described above using the rabbit α₂ probe, hybridizing clones were isolated and characterized by DNA sequencing. HGCaCHα2.20 contained the 3.5 kb fragment and HGCaCHα2.9 contained the 3.0 kb fragment.

Restriction mapping and DNA sequencing revealed that HGCaCHα2.20 contains an 82 bp exon (nt 130 to 211 of the human α₂ coding sequence, SEQ ID No. 11) on a 650 bp *Pst*I-*Xba*I restriction fragment and that HGCaCHα2.9 contains 105 bp of an exon (nt 212 to 316 of the coding sequence, SEQ ID No. 11) on a 750 bp *Xba*I-*Bgl*II restriction fragment. These restriction fragments were used to screen the human basal ganglia cDNA library (Example II.C.2.a.). HBCaCHα2.1 was isolated (nt 29 to 1163, SEQ ID No. 11) and used to screen a human brain stem cDNA library (ATCC Accession No. 37432) obtained from the American Type Culture Collection, 12301 Parklawn Drive, Rockville, MD. 20852. Two clones were isolated, HBCaCHα2.5 (nt 1 to 1162, SEQ ID No. 11) and HBCaCHα2.8 (nt 714 to 1562, SEQ ID No. 11, followed by 1600 nt of intervening sequence). A 2400 bp fragment of HBCaCHα2.8 (beginning at nt 759 of SEQ ID No. 11 and ending at a *Sma*I site in the intron) was used to rescreen the brain stem library and to isolate HBCaCHα2.11 (nt 879 to 3600, SEQ ID No. 11). Clones HBCaCHα2.5 and HBCaCHα2.11 overlap to encode an entire human brain α₂ protein.

### B. construction of pBBCaCHα₂A

To construct pHBCaCHα₂A containing DNA encoding a full-length human calcium channel α₂ subunit, an (*Eco*RI)-*Pvu*II fragment of HBCaCHα2.5 (nt 1 to 1061, SEQ ID No. 11, *Eco*RI adapter, PvuII partial digest) and a PvuII-PstI fragment of HBCaCHα2.11 (nt 1061 to 2424 SEQ ID No. 11; *Pvu*II partial digest) were ligated into *Eco*RI-*Pst*I-digested pIBI24 (Stratagene, La Jolla, CA). Subsequently, an (*EcoR*I)-*Pst*I fragment (nt 1 to 2424 SEQ ID No. 11) was isolated and ligated to a *Pst*I-(*Eco*RI) fragment (nt 2424 to 3600 SEQ ID No. 11) of HBCaCHα2.11 in *E*coRI-digested pIBI24 to produce DNA, HBCaCHα2, encoding a full-length human brain α₂ subunit. The 3600 bp *Eco*RI insert of HBCaCHα2 (nt 1 to 3600, SEQ ID No. 11) was subcloned into pcDNA1 (pHBCaCHα2A) with the methionine initiating codon proximal to the CMV promoter. The 3600 bp *Eco*RI insert of HBCaCHα2 was also subcloned into pSV2dHFR [Subramani *et al.* (1981). *Mol*. *Cell. Biol. 1*:854-864] which contains the SV40 early promoter, mouse dihydrofolate reductase (dhfr) gene, SV40 polyadenylation and splice sites and sequences required for maintenance of the vector in bacteria.

### EXAMPLE V. DIFFERENTIAL PROCESSING OF THE HUMAN β₁ TRANSCRIPT AND THE HUMAN α₂ TRANSCRIPT

### A. Differential processing of the β₁ transcript

PCR analysis of the human β₁ transcript present in skeletal muscle, aorta, hippocampus and basal ganglia, and HEK 293 cells revealed differential processing of the region corresponding to nt 615-781 of SEQ ID No. 9 in each of the tissues. Four different sequences that result in five different processed β₁ transcripts through this region were identified. The β₁ transcripts from the different tissues contained different combinations of the four sequences, except for one of the β₁ transcripts expressed in HEK 293 cells (β₁₋₅) which lacked all four sequences.

None of the β₁ transcripts contained each of the four sequences; however, for ease of reference, all four sequences are set forth end-to-end as a single long sequence in SEQ ID No. 12 . The four sequences that are differentially processed are sequence 1 (nt 14-34 in SEQ ID No. 12), sequence 2 (nt 35-55 in SEQ ID No. 12), sequence 3 (nt 56-190 in SEQ ID No. 12) and sequence 4 (nt 191-271 in SEQ ID No. 12). The forms of the β₁ transcript that have been identified include: (1) a form that lacks sequence 1 called β₁₋₁ (expressed in skeletal muscle), (2) a form that lacks sequences 2 and 3 called β₁₋₂ (expressed in CNS), (3) a form that lacks sequences 1, 2 and 3 called β₁₋₄ (expressed in aorta and HEK cells) and (4) a form that lacks sequences 1-4 called β₁₋₅ (expressed in HEK cells). Additionally, the β₁₋₄ and β₁₋₅ forms contain the guanine nucleotide (nt 13 in SEQ ID No. 12) which is absent in the β₁₋₁ and β₁₋₂ forms.

### B. Differential processing of transcripts encoding the α₂ subunit.

The complete human neuronal α₂ coding sequence (nt 35-3307) plus a portion of the 5' untranslated sequence (nt 1 to 34) as well as a portion of the 3' untranslated sequence (nt 3308-3600) is set forth as SEQ ID No. 11.

PCR analysis of the human α₂ transcript present in skeletal muscle, aorta, and CNS revealed differential processing of the region corresponding to nt 1595-1942 of SEQ ID No. 11 in each of the tissues.

The analysis indicated that the primary transcript of the genomic DNA that includes the nucleotides corresponding to nt. 1595-1942 also includes an additional sequence (SEQ ID No. 13: 5'CCTATTGGTGTAGGTATACCAACAATTAATTT AAGAAAAAGGAGACCCAATATCCAG 3') inserted between nt. 1624 and 1625 of SEQ ID No. 11. Five alternatively spliced variant transcripts that differ in the presence or absence of one to three different portions of the region of the primary transcript that includes the region of nt. 1595-1942 of SEQ ID No. 11 plus SEQ ID No. 13 inserted between nt. 1624 and 1625 have been identified. The five α₂-encoding transcripts from the different tissues include different combinations of the three sequences, except for one of the α₂ transcripts expressed in aorta which lacks all three sequences. None of the α₂ transcripts contained each of the three sequences. The sequences of the three regions that are differentially processed are sequence 1 (SEQ ID No. 13), sequence 2 ( 5' AACCCCAAATCTCAG 3', which is nt. 1625-1639 of SEQ ID No. 11), and sequence 3 ( 5' CAAAAAAGGGCAAAATGAAGG 3', which is nt 1908-1928 of SEQ ID No. 11). The five α₂ forms identified are (1) a form that lacks sequence 3 called α₂ₐ (expressed in skeletal muscle), (2) a form that lacks sequence 1 called α_{2b} (expressed in CNS), (3) a form that lacks sequences 1 and 2 called α_{2c} expressed in aorta), (4) a form that lacks sequences 1, 2 and 3 called α_{2d} (expressed in aorta) and (5) a form that lacks sequences 1 and 3 called α_{2c} (expressed in aorta).

### EXEMPLE VI: ISOLATION OF DNA ENCODING A CALCIUM CHANNEL γ SUBUNIT FROM A HUMAIN BRAIN cDNA LIBRARY

### A. Isolation of DNA encoding the γ subunit

Approximately 1 x 10⁶ recombinants from a λgt11-based human hippocampus cDNA library (Clontech catalog #HL1088b, Palo Alto, CA) were screened by hybridization to a 484 bp sequence of the rabbit skeletal muscle calcium channel γ subunit cDNA (nucleotides 621-626 of the coding sequence plus 438 nucleotides of 3'-untranslated sequence) contained in vector γJ10 [Jay, S. *et al.* (1990). *Science 248*:490-492]. Hybridization was performed using moderate stringency conditions (20% deionized formamide, 5x Denhardt's, 6 x SSPE, 0.2% SDS, 20 µg/ml herring sperm DNA, 42°C) and the filters were washed under low stringency (see Example I.C.). A plaque that hybridized to this probe was purified and insert DNA was subcloned into pGEM7Z. This cDNA insert was designated γ1.4.

### B. Characterization of γ1.4

γ1.4 was confirmed by DNA hybridization and characterized by DNA sequencing. The 1500 bp *Ss*tI fragment of γ1.4 hybridized to the rabbit skeletal muscle calcium channel γ subunit cDNA γJ10 on a Southern blot. SEQ analysis of this fragment revealed that it contains of approximately 500 nt of human DNA sequence and -1000 nt of λgt11 sequence (included due to apparent destruction of one of the *EcoR*I cloning sites in λgt11). The human DNA sequence contains of 129 nt of coding sequence followed immediately by a translational STOP codon and 3' untranslated sequence (SEQ ID No. 14).

To isolate the remaining 5' sequence of the human γ subunit cDNA, human CNS cDNA libraries and/or preparations of mRNA from human CNS tissues can first be assayed by PCR methods using oligonucleotide primers based on the γ cDNA-specific sequence of γ1.4. Additional human neuronal γ subunit-encoding DNA can isolated from cDNA libraries that, based on the results of the PCR assay, contain γ-specific amplifiable cDNA. Alternatively, cDNA libraries can be constructed from mRNA preparations that, based on the results of PCR assays, contain γ-specific amplifiable transcripts. Such libraries are constructed by standard methods using oligo dT to prime first-strand cDNA synthesis from poly A⁺ RNA (see Example I.B.). Alternatively, first-strand cDNA can be specified by priming first-strand cDNA synthesis with a y cDNA-specific oligonucleotide based on the human DNA sequence in γ1.4. A cDNA library can then be constructed based on this first-strand synthesis and screened with the γ-specific portion of γ1.4.

### EXAMPLE VII: RECOMBINANT EXPRESSION OF HUMAN NEURONAL CALCIUM CHANNEL SUBUNIT-ENCODING cDNA AND RNA TRANSCRIPTS IN MAMMALIAN CELLS

### A. Recombinant Expression of the Human Neuronal Calcium Channel α₂ subunit cDNA in DG44 Cells

### 1. Stable transfection of DG44 cells

DG44 cells [dhfr⁻ Chinese hamster ovary cells; see, *e.g.*, Urlaub, G. *et al.* (1986) *Som. Cell Molec. Genet. 12*:555-566] obtained from Lawrence Chasin at Columbia University were stably transfected by CaPO₄ precipitation methods [Wigler *et al.* (1979) *Proc. Natl. Acad. Sci. USA 76*:1373-1376] with pSV2dhfr vector containing the human neuronal calcium channel α₂-subunit cDNA (see Example IV) for polycistronic expression/selection in transfected cells. Transfectants were grown on 10% DMEM medium without hypoxanthine or thymidine in order to select cells that bad incorporated the expression vector. Twelve transfectant cell lines were established as indicated by their ability to survive on this medium.

### 2. Analysis of α₂ subunit cDNA expression in transfected DG44 cells

Total RNA was extracted according to the method of Birnboim [(1988) *Nuc. Acids Res. 16*:1487-1497] from four of the DG44 cell lines that had been stably transfected with pSV2dhfr containing the human neuronal calcium channel α₂ subunit cDNA. RNA (~15 µg per lane) was separated on a 1% agarose formaldehyde gel, transferred to nitrocellulose and hybridized to the random-primed human neuronal calcium channel α₂ cDNA (hybridization: 50% formamide, 5 x SSPE, 5 x Denhardt's, 42° C.; wash :0.2 x SSPE, 0.1% SDS, 65° C.). Northern blot analysis of total RNA from four of the DG44 cell lines that had been stably transfected with pSV2dhfr containing the human neuronal calcium channel α₂ subunit cDNA revealed that one of the four cell lines contained hybridizing mRNA the size expected for the transcript of the α₂ subunit cDNA (5000 nt based on the size of the cDNA) when grown in the presence of 10 mM sodium butyrate for two days. Butyrate nonspecifically induces transcription and is often used for inducing the SV40 early promoter [Gorman, C. and Howard, B. (1983) *Nucleic Acids Res. 11*:1631]. This cell line, 44α₂-9, also produced mRNA species smaller (several species) and larger (6800 nt) than the size expected for the transcript of the α₂ cDNA (5000 nt) that hybridized to the α₂ cDNA-based probe. The 5000- and 6800-nt transcripts produced by this transfectant should contain the entire α₂ subunit coding sequence and therefore should yield a full-length α₂ subunit protein. A weakly hybridizing 8000-nucleotide transcript was present in untransfected and transfected DG44 cells. Apparently, DG44 cells transcribe a calcium channel α₂ subunit or similar gene at low levels. The level of expression of this endogenous α₂ subunit transcript did not appear to be affected by exposing the cells to butyrate before isolation of RNA for northern analysis.

Total protein was extracted from three of the DG44 cell lines that had been stably transfected with pSV2dhfr containing the human neuronal calcium channel α₂ subunit cDNA. Approximately 10⁷ cells were sonicated in 300 µl of a solution containing 50 mM HEPES, 1 mM EDTA, 1 mM PMSF. An equal volume of 2x loading dye [Laemmli, U.K. (1970). *Nature 227*:680] was added to the samples and the protein was subjected to electrophoresis on an 8% polyacrylamide gel and then electrotransferred to nitrocellulose. The nitrocellulose was incubated with polyclonal guinea pig antisera (1:200 dilution) directed against the rabbit skeletal muscle calcium channel α₂ subunit (obtained from K. Campbell, University of Iowa) followed by incubation with [¹²⁵I]-protein A. The blot was exposed to X-ray film at -70° C. Reduced samples of protein from the transfected cells as well as from untransfected DG44 cells contained immunoreactive protein of the size expected for the α₂ subunit of the human neuronal calcium channel (130-150 kDa). The level of this immunoreactive protein was higher in 44α₂-9 cells that had been grown in the presence of 10 mM sodium butyrate than in 44α₂-9 cells that were grown in the absence of sodium butyrate. These data correlate well with those obtained in northern analyses of total RNA from 44α₂-9 and untransfected DG44 cells. Cell line 44α₂-9 also produced a 110 kD immunoreactive protein that may be either a product of proteolytic degradation of the full-length α₂ subunit or a product of translation of one of the shorter (<5000 nt) mRNAs produced in this cell line that hybridized to the α₂ subunit cDNA probe.

### B. Expression of DNA encoding human neuronal calcium channel α₁, α₂ and β₁ subunits in HEX cells

Human embryonic kidney cells (HEK 293 cells) were transiently and stably transfected with human neuronal DNA encoding calcium channel subunits. Individual transfectants were analyzed electrophysiologically for the presence of voltage-activated barium currents and functional recombinant voltage-dependent calcium channels were.

### 1. Transfection of HEK 293 cells

Separate expression vectors containing DNA encoding human neuronal calcium channel α_{1D'} α₂ and β₁ subunits, plasmids pVDCCIII(A), pHBCaCHα₂A, and pHBCaCHβ₁ₐRBS(A), respectively, were constructed as described in Examples II.A.3, IV.B. and III.B.3., respectively. These three vectors were used to transiently co-transfect HEK 293 cells. For stable transfection of HEK 293 cells, vector pHBCaCHβ_{1b}RBS (A) (Example III.B.3.) was used in place of pHBCaCHβ₁ₐRBS(A) to introduce the DNA encoding the β₁ subunit into the cells along with pVDCCIII(A) and pHBCaCHα₂A.

### a. Transient transfection

Expression vectors pVDCCIII(A), pHBCaCHα₂A and pHBCaCHβ₁ₐRBS(A) were used in two sets of transient transfections of HEK 293 cells (ATCC Accession No. CRL1573). In one transfection procedure, HEK 293 cells were transiently cotransfected with the α₁ subunit cDNA expression plasmid, the α₂ subunit cDNA expression plasmid, the β₁ subunit cDNA expression plasmid and plasmid pCMVβgal (Clontech Laboratories, Palo Alto, CA). Plasmid pCMVβgal contains the *lacZ* gene (encoding *E. coli* β-galactosidase) fused to the cytomegalovirus (CMV) promoter and was included in this transfection as a marker gene for monitoring the efficiency of transfection. In the other transfection procedure, HEK 293 cells were transiently co-transfected with the a, subunit cDNA expression plasmid pVDCCIII(A) and pCMVβgal. In both transfections, 2-4 x 10⁶ HEK 293 cells in a 10-cm tissue culture plate were transiently co-transfected with 5 µg of each of the plasmids included in the experiment according to standard CaPO₄ precipitation transfection procedures (Wigler *et al.* (1979) Proc. *Natl.* Acad. *Sci. USA* 76:1373-1376). The transfectants were analyzed for β-galactosidase expression by direct staining of the product of a reaction involving β-galactosidase and the X-gal substrate [Jones, J.R. (1986) *EMBO* 5:3133-3142] and by measurement of β-galactosidase activity [Miller, J.H. (1972) Experiments in Molecular Genetics, pp. 352-355, Cold Spring Harbor Press]. To evaluate subunit cDNA expression in these transfectants, the cells were analyzed for subunit transcript production (northern analysis), subunit protein production (immunoblot analysis of cell lysates) and functional calcium channel expression (electrophysiological analysis).

### b. Stable transfection

HEK 293 cells were transfected using the calcium phosphate transfection procedure [*Current Protocols in Molecular Biology,* Vol. 1, Wiley Inter-Science, Supplement 14, Unit 9.1.1-9.1.9 (1990)]. Ten-cm plates, each containing one-to-two million HEK 293 cells, were transfected with 1 ml of DNA/calcium phosphate precipitate containing 5 *µ*g pVDCCIII(A), 5 *µ*g pHBCaCHα₂A, 5*µ*g pHBCaCHβ_{1b}RBS (A) , 5 *µ*g pCMVBgal and 1 *µ*g pSV2neo (as a selectable marker). After 10-20 days of growth in media containing 500 *µ*g G418, colonies had formed and were isolated using cloning cylinders.

### 2. Analysis of HEK 293 cells transiently transfected vith DNA encoding human neuronal calcium channel subunits

### a. Analysis of β-galactosidase expression

Transient transfectants were assayed for β-galactosidase expression by β-galactosidase activity assays (Miller, J.H., (1972) Experiments in Molecular Genetics, pp. 352-355, Cold Spring Harbor Press) of cell lysates (prepared as described in Example VII.A.2) and staining of fixed cells (Jones, J.R. (1986) *EMBO 5*:3133-3142). The results of these assays indicated that approximately 30% of the HEK 293 cells had been transfected.

### b. Northern analysis

PolyA+ RNA was isolated using the Invitrogen Fast Trak Kit (InVitrogen, San Diego, CA) from HEK 293 cells transiently transfected with DNA encoding each of the α₁, α₂ and β₁ subunits and the *lacZ* gene or the α₁ subunit and the *lacZ* gene. The RNA was subjected to electrophoresis on an agarose gel and transferred to nitrocellulose. The nitrocellulose was then hybridized with one or more of the following radiolabeled probes: the *lacZ* gene, human neuronal calcium channel α_{1D} subunit-encoding cDNA, human neuronal calcium channel α₂ subunit-encoding cDNA or human neuronal calcium channel β₁ subunit-encoding cDNA. Two transcripts that hybridized with the α₁ subunit-encoding cDNA were detected in HEK 293 cells transfected with the DNA encoding the α₁, α₂, and β₁ subunits and the *lacZ* gene as well as in HEK 293 cells transfected with the α₁ subunit cDNA and the *lacZ* gene. One mRNA species was the size expected for the transcript of the α₁ subunit cDNA (8000 nucleotides). The second RNA species was smaller (4000 nucleotides) than the size expected for this transcript. RNA of the size expected for the transcript of the *lacZ* gene was detected in cells transfected with the α₁, α₂ and β₁ subunit-encoding cDNA and the *lacZ* gene and in cells transfected with the α₁ subunit cDNA and the *lacZ* gene by hybridization to the *lacZ* gene sequence.

RNA from cells transfected with the α₁, α₂ and β₁ subunit-encoding cDNA and the *lacZ* gene was also hybridized with the α₂ and β₁ subunit cDNA probes. Two mRNA species hybridized to the α₂ subunit cDNA probe. One species was the size expected for the transcript of the α₂ subunit cDNA (4000 nucleotides). The other species was larger (6000 nucleotides) than the expected size of this transcript. Multiple RNA species in the cells co-transfected with α₁, α₂ and β₁ subunit-encoding cDNA and the *lacZ* gene hybridized to the β₁ subunit cDNA probe. Multiple β-subunit transcripts of varying sizes were produced since the β subunit cDNA expression vector contains two potential polyA⁺ addition sites.

### c. Electrophysiological analysis

Individual transiently transfected HEK 293 cells were assayed for the presence of voltage-dependent barium currents using the whole-cell variant of the patch clamp technique [Hamill *et al.* (1981). *Pflugers Arch. 391*:85-100]. HEK 293 cells transiently transfected with pCMVβgal only were assayed for barium currents as a negative control in these experiments. The cells were placed in a bathing solution that contained barium ions to serve as the current carrier. Choline chloride, instead of NaCl or KCl, was used as the major salt component of the bath solution to eliminate currents through sodium and potassium channels. The bathing solution contained 1 mM MgCl₂ and was buffered at pH 7.3 with 10 mM HEPES (pH adjusted with sodium or tetraethylammonium hydroxide). Patch pipettes were filled with a solution containing 135 mM CsCl, 1 mM MgCl₂, 10 mM glucose, 10 mM EGTA, 4 mM ATP and 10 mM HEPES (pH adjusted to 7.3 with tetraethylammonium hydroxide). Cesium and tetraethylammonium ions block most types of potassium channels. Pipettes were coated with Sylgard (Dow-Corning, Midland, MI) and had resistances of 1-4 megohm. Currents were measured through a 500 megohm headstage resistor with the Axopatch IC (Axon Instruments, Poster City, CA) amplifier, interfaced with a Labmaster (Scientific Solutions, Solon, OH) data acquisition board in an IBM-compatible PC. PClamp (Axon Instruments) was used to generate voltage commands and acquire data. Data were analyzed with pClamp or Quattro Professional (Borland International, Scotts Valley, CA) programs.

To apply drugs, "puffer" pipettes positioned within several micrometers of the cell under study were used to apply solutions by pressure application. The drugs used for pharmacological characterization were dissolved in a solution identical to the bathing solution. Samples of a 10 mM stock solution of Bay K 8644 (RBI, Natick, MA), which was prepared in DMSO, were diluted to a final concentration of 1 µM in 15 mM Ba²⁺-containing bath solution before they were applied.

Twenty-one negative control HEK 293 cells (transiently transfected with the *lacZ* gene expression vector pCMVβgal only) were analyzed by the whole-cell variant of the patch clamp method for recording currents. Only one cell displayed a discernable inward barium current; this current was not affected by the presence of 1 *µ*M Bay K 8644. In addition, application of Bay K 8644 to four cells that did not display Ba²⁺ currents did not result in the appearance of any currents.

Two days after transient transfection of HEK 293 cells with α₁, α₂ and β₁ subunit-encoding cDNA and the *lacZ* gene, individual transfectants were assayed for voltage-dependent barium currents. The currents in nine transfectants were recorded. Because the efficiency of transfection of one cell can vary from the efficiency of transfection of another cell, the degree of expression of heterologous proteins in individual transfectants varies and some cells do not incorporate or express the foreign DNA. Inward barium currents were detected in two of these nine transfectants. In these assays, the holding potential of the membrane was -90 mV. The membrane was depolarized in a series of voltage steps to different test potentials and the current in the presence and absence of 1 µM Bay K 8644 was recorded. The inward barium current was significantly enhanced in magnitude by the addition of Bay K 8644. The largest inward barium current (-160 pA) was recorded when the membrane was depolarized to 0 mV in the presence of 1 µM Bay K 8644. A comparison of the I-V curves, generated by plotting the largest current recorded after each depolarization versus the depolarization voltage, corresponding to recordings conducted in the absence and presence of Bay K 8644 illustrated the enhancement of the voltage-activated current in the presence of Bay K 8644.

Pronounced tail currents were detected in the tracings of currents generated in the presence of Bay K 8644 in HEK 293 cells transfected with α₁, *α*₂ and β₁ subunit-encoding cDNA and the *lacZ* gene, indicating that the recombinant calcium channels responsible for the voltage-activated barium currents recorded in this transfected appear to be DHP-sensitive.

The second of the two transfected cells that displayed inward barium currents expressed a ~50 pA current when the membrane was depolarized from -90 mV. This current was nearly completely blocked by 200 µM cadmium, an established calcium channel blocker.

Ten cells that were transiently transfected with the DNA encoding the α₁ subunit and the *lacZ* gene were analyzed by whole-cell patch clamp methods two days after transfection. One of these cells displayed a 30 pA inward barium current. This current amplified 2-fold in the presence of 1 µM Bay K 8644. Furthermore, small tail currents were detected in the presence of Bay K 8644. These data indicate that expression of the human neuronal calcium channel α_{1D} subunit-encoding cDNA in HEK 293 yields a functional DHP-sensitive calcium channel.

### 3. Analysis of HEX 293 cells stably transfected with DNA encoding human neuronal calcium channel subunits

Individual stably transfected HEK 293 cells were assayed electrophysiologically for the presence of voltage-dependent barium currents as described for electrophysiological analysis of transiently transfected HEK 293 cells (see Example VII.B.2.c). In an effort to maximize calcium channel activity via cyclic-AMP-dependent kinase-mediated phosphorylation [Pelzer, et al. (1990) *Rev. Physiol. Biochem. Pharmacol. 114:*107-207],cAMP (Na salt, 250 µM) was added to the pipet solution and forskolin (10 µM) was added to the bath solution in some of the recordings. Qualitatively similar results were obtained whether these compounds were present or not.

Barium currents were recorded from stably transfected cells in the absence and presence of Bay K 8644 (1 µN). When the cell was depolarized to -10 mV from a holding potential of -90 mV in the absence of Bay K 8644, a current of approximately 35pA with a rapidly deactivating tail current was recorded. During application of Bay K 8644, an identical depolarizing protocol elicited a current of approximately 75 pA, accompanied by an augmented and prolonged tail current. The peak magnitude of currents recorded from this same cell as a function of a series of depolarizing voltages were assessed. The responses in the presence of Bay K 8644 not only increased, but the entire current-voltage relation shifted about -10 mV. Thus, three typical hallmarks of Bay K 8644 action, namely increased current magnitude, prolonged tail currents, and negatively shifted activation voltage, were observed, clearly indicating the expression of a DHP-sensitive calcium channel in these stably transfected cells. No such effects of Bay K 8644 were observed in untransfected HEK 293 cells, either with or without cAMP or forskolin.

### C. Use of pCHV-based vectors and pcDNA1-based vectors for expression of DNA encoding human neuronal calcium channel subunits

### 1. Preparation of constructs

To determine if the levels of recombinant expression of human calcium channel subunit-encoding DNA in host cells could be enhanced by using pCMV-based instead of pcDNA1-based expression vectors, additional expression vectors were constructed. The full-length α_{1D} cDNA from pVDCCIII(A) (see Example II.A.3.d), the full-length α₂ cDNA, contained on a 3600 bp *Eco*RI fragment from HBCaCHα₂ (see Example IV.B) and a full-length β₁ subunit cDNA from pHBCaCHβ_{1b}RBS(A) (see Example III.B.3) were separately subcloned into plasmid pCMVβgal. Plasmid pCMVβgal was digested with *Not*I to remove the *lacZ* gene. The remaining vector portion of the plasmid, referred to as pCMV, was blunt-ended at the NotI sites. The full-length α₂-encoding DNA and β₁-encoding DNA, contained on separate *Eco*RI fragments, were isolated, blunt-ended and separately ligated to the blunt-ended vector fragment of pCMV locating the cDNAs between the CMV promoter and SV40 polyadenylation sites in pCMV. To ligate the α_{1D}-encoding cDNA with pCMV, the restriction sites in the polylinkers immediately 5' of the CMV promoter and immediately 3' of the SV40 polyadenylation site were removed from pCMV. A polylinker was added at the *Not*I site. The polylinker had the following sequence of restriction enzyme recognition sites:

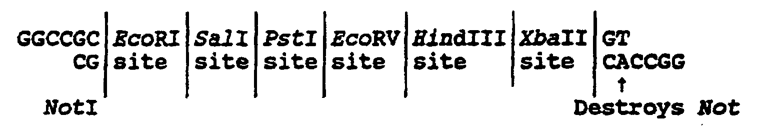

The α_{1D}-encoding DNA, isolated as a *Bam*HI/*Xho*I fragment from pVDCCIII(A), was then ligated to *Xba*II/*Sal*I-digested pCMV to place it between the CMV promoter and SV40 polyadenylation site.

Plasmid pCMV contains the CMV promoter as does pcDNA1, but differs from pcDNA1 in the location of splice donor/splice acceptor sites relative to the inserted subunit-encoding DNA. After inserting the subunit-encoding DNA into pCMV, the splice donor/splice acceptor sites are located 3' of the CMV promoter and 5' of the subunit-encoding DNA start codon. After inserting the subunit-encoding DNA into pcDNA1, the splice donor/splice acceptor sites are located 3' of the subunit cDNA stop codon.

### 2. Transfection of HEX 293 cells

HEK 293 cells were transiently co-transfected with the α_{1D}, α₂ and β₁ subunit-encoding DNA in pCMV or with the α_{1D}, α₂ and β subunit-encoding DNA in pcDNA1 (vectors pVDCCIII (A, pHBCaCHα₂A and pHBCaCHβ_{1b}RBS (A), respectively), as described in Example VII.B.1.a. Plasmid pCMVβgal was included in each transfection to as a measure of transfection efficiency. The results of β-galactosidase assays of the transfectants (see Example VII.B.2.), indicated that HEK 293 cells were transfected equally efficiently with pCMV- and pcDNA1-based plasmids.

### 3. Northern analysis

Total and polyA⁺ RNA were isolated from the transiently transfected cells as described in Examples VII.A.2 and VII.B.2.b. Northern blots of the RNA were hybridized with the following radiolabeled probes: α_{1D} cDNA, human neuronal calcium channel α₂ subunit cDNA and DNA encoding the human neuronal calcium channel β₁ subunit. Messenger RNA of sizes expected for α_{1D}, α₂ and β₁ subunit transcripts were detected in all transfectants. A greater amount of the α_{1D} transcript was present in cells that were co-transfected with pCMV-based plasmids then in cells that were co-transfected with pcDNA1-based plasmids. Equivalent amounts of α₂ and β₁ subunit transcripts were detected in all transfectants.

### D. Expression in Xenopus laevis oöcytes of RNA encoding human neuronal calcium channel subunits

Various combinations of the transcripts of DNA encoding the human neuronal α_{1D}, α₂ and β₁ subunits prepared *in vitro* were injected into *Xenopus laevis* oöcytes. Those injected with combinations that included a_{1D} exhibited voltage-activated barium currents.

### 1. Preparation of transcripts

Transcripts encoding the human neuronal calcium channel α_{1D}, α₂ and β₁ subunits were synthesized according to the instructions of the mCAP mRNA CAPPING KIT (Strategene, La Jolla, CA catalog #200350). Plasmids pVDCC III.RBS(A), containing of pcDNA1 and the α_{1D} cDNA that begins with a ribosome binding site and the eighth ATG codon of the coding sequence (see Example III.A.3.d), plasmid pHBCaCBα₁A containing of pcDNA1 and an α₂ subunit cDNA (see Example IV), and plasmid pHBCaCHβ_{1b}RBS(A) containing pcDNA1 and the DNA lacking intron sequence and containing a ribosome binding site (see Example III), were linearized by restriction digestion. The α_{1D} cDNA- and α₂ subunit-encoding plasmids were digested with *Xho*I*,* and the β₁, subunit-encoding plasmid was digested with *Eco*RV. The DNA insert was transcribed with T7 RNA polymerase.

### 2. Injection of cöcytes

*Xenopus laevis* oöcytes were isolated and defolliculated by collagenase treatment and maintained in 100 mM NaCl, 2 mM KCl, 1.8 mM CaCl₂, 1 mM MgCl₂, 5 mM HEPES, pH 7.6, 20 µg/ml ampicillin and 25 µg/ml streptomycin at 19-25°C for 2 to 5 days after injection and prior to recording. For each transcript that was injected into the oöcyte, 6 ng of the specific mRNA was injected per cell in a total volume of 50 nl.

### 3. Intracellular voltage recordings

Injected oöcytes were examined for voltage-dependent barium currents using two-electrode voltage clamp methods [Dascal, N. (1987) *CRC Crit. Rev. Biochem. 22*:317]. The pClamp (Axon Instruments) software package was used in conjunction with a Labmaster 125 kHz data acquisition interface to generate voltage commands and to acquire and analyze data. Quattro Professional was also used in this analysis. Current signals were digitized at 1-5 kHz, and filtered appropriately. The bath solution contained of the following: 40 mM Bacl₂, 36 mM tetraethylammonium chloride (TEA-Cl), 2 mM KCl, 5 mM 4-aminopyridine, 0.15 mM niflumic acid, 5 mM HEPES, pH 7.6.

### a. Electrophysiological analysis of oöcytes injected with transcripts encoding the human neuronal calcium channel α₁, α₂ and β₁-subunits

Uninjected oöcytes were examined by two-electrode voltage clamp methods and a very small (25 nA) endogenous inward Ba²⁺ current was detected in only one of seven analyzed cells.

Oöcytes coinjected with α_{1D}, α₂ and β₁ subunit transcripts expressed sustained inward barium currents upon depolarization of the membrane from a holding potential of -90 mV or -50 mV (154 ± 129 nA, n=21). These currents typically showed little inactivation when test pulses ranging from 140 to 700 msec. were administered. Depolarization to a series of voltages revealed currents that first appeared at approximately -30 mV and peaked at approximately 0 mV.

Application of the DHP Bay K 8644 increased the magnitude of the currents, prolonged the tail currents present upon repolarization of the cell and induced a hyperpolarizing shift in current activation. Bay K 8644 was prepared fresh from a stock solution in DMSO and introduced as a 10x concentrate directly into the 60 µl bath while the perfusion pump was turned off. The DMSO concentration of the final diluted drug solutions in contact with the cell never exceeded 0.1%. Control experiments showed that 0.1% DMSO had no effect on membrane currents.

Application of the DHP antagonist nifedipine (stock solution prepared in DMSO and applied to the cell as described for application of Bay K 8644) blocked a substantial fraction (91 ± 6%, n=7) of the inward barium current in oöcytes coinjected with transcripts of the α_{1D}, α₂ and β₁ subunits. A residual inactivating component of the inward barium current typically remained after nifedipine application. The inward barium current was blocked completely by 50 µM Cd²⁺, but only approximately 15% by 100 µM Ni²⁺.

The effect of ωCgTX on the inward barium currents in oöcytes co-injected with transcripts of the α_{1D}, α₂, and β₁ subunits was investigated. ωCgTX (Bachem, Inc., Torrance CA) was prepared in the 15 mM BaCl₂ bath solution plus 0.1% cytochrome C (Sigma) to serve as a carrier protein. Control experiments showed that cytochrome C had no effect on currents. A series of voltage pulses from a -90 mV holding potential to 0 mV were recorded at 20 msec. intervals. To reduce the inhibition of ωCgTX binding by divalent cations, recordings were made in 15 mM BaCl₂, 73.5 mM tetraethylammonium chloride, and the remaining ingredients identical to the 40 mM Ba²⁺ recording solution. Bay K 8644 was applied to the cell prior to addition to ωCgTX in order to determine the effect of ωCgTX on the DHP-sensitive current component that was distinguished by the prolonged tail currents. The inward barium current was blocked weakly (54 ± 29%, n=7) and reversibly by relatively high concentrations (10-15 µM) of ωCgTX. The test currents and the accompanying tail currents were blocked progressively within two to three minutes after application of ωCgTX, but both recovered partially as the ωCgTX was flushed from the bath.

### b. Analysis of cocytes injected with only a transcripts encoding the human neuronal calcium channel α_{1D} or transcripts encoding an α_{1D} and other subunits

The contribution of the α₂ and β₁ subunits to the inward barium current in oöcytes injected with transcripts encoding the α_{1D}, α₂ and β₁ subunits was assessed by expression of the α_{1D} subunit alone or in combination with either the β₁ subunit or the α₂ subunit. In oöcytes injected with only the transcript of a α_{1D} cDNA, no Ba²⁺ currents were detected (n=3). In oocytes injected with transcripts of α_{1D} and β₁ cDNAs, small (108 ± 39 nA) Ba²⁺ currents were detected upon depolarization of the membrane from a holding potential of -90 mV that resembled the currents observed in cells injected with transcripts of α_{1D}, α₂ and β₁ cDNAs, although the magnitude of the current was less. In two of the four oöcytes injected with transcripts of the α_{1D}-encoding and β₁-encoding DNA, the Ba²⁺ currents exhibited a sensitivity to Bay K 8644 that was similar to the Bay K 8644 sensitivity of Ba²⁺ currents expressed in oöcytes injected with transcripts encoding the α_{1D} α₁-, α₂- and β₁ subunits.

Three of five oöcytes injected with transcripts encoding the α_{1D} and α₂ subunits exhibited very small Ba²⁺ currents (15-30 nA) upon depolarization of the membrane from a holding potential of -90 mV. These barium currents showed little or no response to Bay K 8644.

### c. Analysis of cocytes injected with transcripts encoding the human neuronal calcium channel α₂, and/or β₁ subunit

To evaluate the contribution of the α_{1D} α₁-subunit to the inward barium currents detected in oöcytes co-injected with transcripts encoding the α_{1D}, α₂ and β₁ subunits, oöcytes injected with transcripts encoding the human neuronal calcium channel α₂ and/or β₁ subunits were assayed for barium currents. Oöcytes injected with transcripts encoding the α₂ subunit displayed no detectable inward barium currents (n=5) . Oöcytes injected with transcripts encoding a β₁ subunit displayed measurable (54 ± 23 nA, n=5) inward barium currents upon depolarization and oöcytes injected with transcripts encoding the α₂ and β₁ subunits displayed inward barium currents that were approximately 50% larger (80 ± 61 nA, n=18) than those detected in oöcytes injected with transcripts of the β₁-encoding DNA only.

The inward barium currents in oöcytes injected with transcripts encoding the β₁ subunit or α₂ and β₁ subunits typically were first observed when the membrane was depolarized to -30 mV from a holding potential of -90 mV and peaked when the membrane was depolarized to 10 to 20 mV. Macroscopically, the currents in oöcytes injected with transcripts encoding the α₂ and β₁ subunits or with transcripts encoding the β₁ subunit were indistinguishable. In contrast to the currents in oöcytes co-injected with transcripts of α_{1D}, α₂ and β₁ subunit cDNAs, these currents showed a significant inactivation during the test pulse and a strong sensitivity to the holding potential. The inward barium currents in oöcytes co-injected with transcripts encoding the α₂ and β₁ subunits usually inactivated to 10-60% of the peak magnitude during a 140-msec pulse and were significantly more sensitive to holding potential than those in oöcytes co-injected with transcripts encoding the α_{1D}, α₂ and β₁ subunits. Changing the holding potential of the membranes of oöcytes co-injected with transcripts encoding the α₂ and β₁ subunits from -90 to -50 mV resulted in an approximately 81% (n=11) reduction in the magnitude of the inward barium current of these cells. In contrast, the inward barium current measured in oöcytes co-injected with transcripts encoding the α_{1D}, α₂ and β₁ subunits were reduced approximately 24% (n=11) when the holding potential was changed from -90 to -50 mV.

The inward barium currents detected in oöcytes injected with transcripts encoding the α₂ and β₁ subunits were pharmacologically distinct from those observed in oöcytes co-injected with transcripts encoding the α_{1D}, α₂ and β₁ subunits. Oöcytes injected with transcripts encoding the α₂ and β₁ subunits displayed inward barium currents that were insensitive to Bay K 8644 (n=11). Nifedipine sensitivity was difficult to measure because of the holding potential sensitivity of nifedipine and the current observed in oöcytes injected with transcripts encoding the α₂ and β₁, subunits. Nevertheless, two oöcytes that were co-injected with transcripts encoding the α₂ and β₁ subunits displayed measurable (25 to 45 nA) inward barium currents when depolarized from a holding potential of -50 mV. These currents were insensitive to nifedipine (5 to 10 *µ*M). The inward barium currents in oöcytes injected with transcripts encoding the α₂ and β₁ subunits showed the same sensitivity to heavy metals as the currents detected in oöcytes injected with transcripts encoding the α_{1D}, α₂ and β₁ subunits.

The inward barium current detected in oöytes injected with transcripts encoding the human neuronal α₂ and β₁ subunits has pharmacological and biophysical properties that resemble calcium currents in uninjected xenopus oöcytes. Because the amino acids of this human neuronal calcium channel β₁ subunit lack hydrophobic segments capable of forming transmembrane domains, it is unlikely that recombinant β₁ subunits alone can form an ion channel. It is more probable that a homologous endogenous α₁ subunit exists in oöcytes and that the activity mediated by such an α₁ subunit is enhanced by expression of a human neuronal β₁ subunit. The following is a glossary describing in more detail each sequence in the sequence listing:

| SEQ ID NO. | DESCRIPTION |
|---|---|
| 1 | DNA encoding an α_{1D} subunit and amino acids encoded by the DNA. |
| 2 | Amino acids contained within an α_{1D} subunit and DNA encoding the amino acids. |
| 3 | DNA encoding an α_{1c} subunit and amino acids encoded by the DNA. |
| 4 | DNA encoding a possible amino terminal end of an α_{1c} subunit. |
| 5 | DNA encoding a possible amino terminal end of an α_{1c} subunit. |
| 6 | DNA encoding an alternative exon for the IV S3 transmembrane domain of an α_{1c} subunit and amino acids encoded by the DNA. |
| 7 | DNA encoding an α_{1B-1} subunit and the amino acids encoded by the DNA. |
| 8 | DNA encoding an α_{1B-2} subunit and the amino acids encoded by the DNA. |
| 9 | DNA encoding a β₁₋₂ subunit and the amino acids encoded by the DNA. |
| 10 | DNA encoding a β₁₋₃ subunit and the amino acids encoded by the DNA. |
| 11 | DNA encoding an α_{2b} subunit and the amino acids encoded by the DNA. |
| 12 | Four different sequences set forth end-to-end that result in five different processed β₁ subunit transcripts through the region corresponding to nt 615-781 of SEQ ID No. 9. |
| 13 | DNA contained within a primary transcript that is alternatively spliced to generate DNA encoding α₂ subunits. |
| 14 | DNA contained within DNA encoding a γ subunit and the amino acids encoded by the DNA. |
| 15 | DNA sequence of an *E*col adapter used in generating an IMR32 cell cDNA library. |
| 16 | DNA sequence of an *Ecol* adapter used in generating an IMR32 cell cDNA library. |
| 17 | DNA sequence of an adapter used in generating a thalamus cDNA library. |
| 18 | DNA sequence of an adapter used in generating a thalamus cDNA library. |
| 19 | DNA sequence contained in a plasmid deposited under Accession No. 69048 at the American Type Culture Collection. |
| 20 | DNA sequence contained in a plasmid deposited under Accession No. 69048 at the American Type Culture Collection. |
| 21 | DNA sequence contained in DNA encoding an α_{1A} subunit that hybridizes to DNA encoding an α_{1A} subunit but not to DNA encoding an α_{1B} subunit. |
| 22 | DNA and amino acid sequence of a splice variant of the α_{1D} subunit. This variant is described on pages 31 (lines 3-6) and 55 (lines 4-10). This variant differs from the α_{1D} variant of SEQ ID No. 1 in that it contains an alternative exon (SEQ ID No. 2) that replaces nucleotides 1627-1730 of SEQ ID No. 1. Thus, SEQ ID No. 22 is SEQ ID No. 1 in which nucleotides 1627-1730 have been replaced with nucleotides 1-104 of SEQ ID No. 2. |

### SEQUENCE LISTING

### (1) GENERAL INFORMATION

(i) APPLICANT: SIBIA Neurosciences
(ii) TITLE OF THE INVENTION: HUMAN CALCIUM CHANNEL COMPOSITIONS AND METHODS
(iii) NUMBER OF SEQUENCES: 54
(iv) CORRESPONDENCE ADDRESS:
   (A) ADDRESSEE: BOULT WADE TENNANT
   (B) STREET: 27 Furnival Street
   (C) CITY: London
   (D) STATE:
   (E) COUNTRY: GB
   (F) ZIP: EC4A 1PQ
(v) COMPUTER READABLE FORM:
   (A) MEDIUM TYPE: Diskette
   (B) COMPUTER: IBM Compatible
   (C) OPERATING SYSTEM: DOS
   (D) SOFTWARE: FastSEQ Version 1.5
(vi) CURRENT APPLICATION DATA:
   (A) APPLICATION NUMBER: EP92918611.2-2105
   (B) FILING DATE: 13-JAN-1994
   (C) CLASSIFICATION:
(vii) PRIOR-APPLICATION DATA:
   (A) APPLICATION NUMBER: PCT/US92/06903
   (B) FILING DATE: 14-AUG-1992

   (A) APPLICATION NUMBER: 07/868/354
   (B) FILING DATE: 10-APR-1992

   (A) APPLICATION NUMBER: 07/745/206
   (B) FILING DATE: 15-AUG-1991
(viii) ATTORNEY/AGENT INFORMATION:
   (A) NAME: BALDOCK; Sharon Claire
   (B) REGISTRATION NUMBER:
   (C) REFERENCE/DOCKET NUMBER: 43522/400
(ix) TELECOMMUNICATION INFORMATION:
   (A) TELEPHONE: 44 171 430 7500
   (B) TELEFAX: 44 171 831 1768
   (C) TELEX:

### (2) INFORMATION FOR SEQ ID NO:1:

(i) SEQUENCE CHARACTERISTICS:
   (A) LENGTH: 7635 base pairs
   (B) TYPE: nucleic acid
   (C) STRANDEDNESS: double
   (D) TOPOLOGY: linear
(ii) MOLECULE TYPE: DNA (genomic)
(ix) FEATURE:
   (A) NAME/KEY: CDS
   (B) LOCATION: 511..6996
(ix) FEATURE:
   (A) NAME/KEY: 5'UTR
   (B) LOCATION: 1..510
(ix) FEATURE:
   (A) NAME/KEY: 3'UTR
   (B) LOCATION: 6994..7635
(xi) SEQUENCE DESCRIPTION: SEQ ID NO:1:
   GGGCGAGCGC CTCCGTCCCC GGATGTGAGC TCCGGCTGCC CGCGGTCCCG AGCCAGCGGC      60
   GCGCGGGCGG CGGCGGCGGG CACCGGGCAC CGCGGCGGGC GGGCAGACGG GCGGGCATGG     120
   GGGGAGCGCC GAGCGGCCCC GGCGGCCGGG CCGGCATCAC CGCGGCGTCT CTCCGCTAGA     180
   GGAGGGGACA AGCCAGTTCT CCTTTGCAGC AAAAAATTAC ATGTATATAT TATTAAGATA     240
   ATATATACAT TGGATTTTAT TTTTTTAAAA AGTTTATTTT GCTCCATTTT TGAAAAAGAG     300
   AGAGCTTGGG TGGCGAGCGG TTTTTTTTTA AAATCAATTA TCCTTATTTT CTGTTATTTG     360
   TCCCCGTCCC TCCCCACCCC CCTGCTGAAG CGAGAATAAG GGCAGGGACC GCGGCTCCTA     420
   CCTCTTGGTG ATCCCCTTCC CCATTCCGCC CCCGCCCCAA CGCCCAGCAC AGTGCCCTGC     480
   ACACAGTAGT CGCTCAATAA ATGTTCGTGG ATG ATG ATG ATG ATG ATG ATG AAA     534
                                    Met Met Met Met Met Met Met Lys
                                      1               5
   AAA ATG CAG CAT CAA CGG CAG CAG CAA GCG GAC CAC GCG AAC GAG GCA     582
   Lys Met Gln His Gln Arg Gln Gln Gln Ala Asp His Ala Asn Glu Ala
        10                  15                  20
   AAC TAT GCA AGA GGC ACC AGA CTT CCT CTT TCT GGT GAA GGA CCA ACT     630
   Asn Tyr Ala Arg Gly Thr Arg Leu Pro Leu Ser Gly Glu Gly Pro Thr
    25                  30                  35                  40
   TCT CAG CCG AAT AGC TCC AAG CAA ACT GTC CTG TCT TGG CAA GCT GCA     678
   Ser Gln Pro Asn Ser Ser Lys Gln Thr Val Leu Ser Trp Gln Ala Ala
                    45                  50                  55
   ATC GAT GCT GCT AGA CAG GCC AAG GCT GCC CAA ACT ATG AGC ACC TCT     726
   Ile Asp Ala Ala Arg Gln Ala Lys Ala Ala Gln Thr Met Ser Thr Ser
                60                  65                  70
   GCA CCC CCA CCT GTA GGA TCT CTC TCC CAA AGA AAA CGT CAG CAA TAC     774
   Ala Pro Pro Pro Val Gly Ser Leu Ser Gln Arg Lys Arg Gln Gln Tyr
            75                  80                  85
   GCC AAG AGC AAA AAA CAG GGT AAC TCG TCC AAC AGC CGA CCT GCC CGC     822
   Ala Lys Ser Lys Lys Gln Gly Asn Ser Ser Asn Ser Arg Pro Ala Arg
        90                  95                 100
   GCC CTT TTC TGT TTA TCA CTC AAT AAC CCC ATC CGA AGA GCC TGC ATT     870
   Ala Leu Phe Cys Leu Ser Leu Asn Asn Pro Ile Arg Arg Ala Cys Ile
   105                 110                 115                 120
   AGT ATA GTG GAA TGG AAA CCA TTT GAC ATA TTT ATA TTA TTG GCT ATT     918
   Ser Ile Val Glu Trp Lys Pro Phe Asp Ile Phe Ile Leu Leu Ala Ile
                   125                 130                 135
   TTT GCC AAT TGT GTG GCC TTA GCT ATT TAC ATC CCA TTC CCT GAA GAT     966
   Phe Ala Asn Cys Val Ala Leu Ala Ile Tyr Ile Pro Phe Pro Glu Asp
               140                 145                 150
   GAT TCT AAT TCA ACA AAT CAT AAC TTG GAA AAA GTA GAA TAT GCC TTC      1014
   Asp Ser Asn Ser Thr Asn His Asn Leu Glu Lys Val Glu Tyr Ala Phe
           155                 160                 165
   CTG ATT ATT TTT ACA GTC GAG ACA TTT TTG AAG ATT ATA GCG TAT GGA      1062
   Leu Ile Ile Phe Thr Val Glu Thr Phe Leu Lys Ile Ile Ala Tyr Gly
       170                 175                 180
   TTA TTG CTA CAT CCT AAT GCT TAT GTT AGG AAT GGA TGG AAT TTA CTG      1110
   Leu Leu Leu His Pro Asn Ala Tyr Val Arg Asn Gly Trp Asn Leu Leu
   185                 190                 195                 200
   GAT TTT GTT ATA GTA ATA GTA GGA TTG TTT AGT GTA ATT TTG GAA CAA      1158
   Asp Phe Val Ile Val Ile Val Gly Leu Phe Ser Val Ile Leu Glu Gln
                   205                 210                 215
   TTA ACC AAA GAA ACA GAA GGC GGG AAC CAC TCA AGC GGC AAA TCT GGA      1206
   Leu Thr Lys Glu Thr Glu Gly Gly Asn His Ser Ser Gly Lys Ser Gly
               220                 225                 230
   GGC TTT GAT GTC AAA GCC CTC CGT GCC TTT CGA GTG TTG CGA CCA CTT      1254
   Gly Phe Asp Val Lys Ala Leu Arg Ala Phe Arg Val Leu Arg Pro Leu
           235                 240                 245
   CGA CTA GTG TCA GGA GTG CCC AGT TTA CAA GTT GTC CTG AAC TCC ATT      1302
   Arg Leu Val Ser Gly Val Pro Ser Leu Gln Val Val Leu Asn Ser Ile
       250                 255                 260
   ATA AAA GCC ATG GTT CCC CTC CTT CAC ATA GCC CTT TTG GTA TTA TTT      1350
   Ile Lys Ala Met Val Pro Leu Leu His Ile Ala Leu Leu Val Leu Phe
   265                 270                 275                 280
   GTA ATC ATA ATC TAT GCT ATT ATA GGA TTG GAA CTT TTT ATT GGA AAA      1398
   Val Ile Ile Ile Tyr Ala Ile Ile Gly Leu Glu Leu Phe Ile Gly Lys
                   285                 290                 295
   ATG CAC AAA ACA TGT TTT TTT GCT GAC TCA GAT ATC GTA GCT GAA GAG      1446
   Met His Lys Thr Cys Phe Phe Ala Asp Ser Asp Ile Val Ala Glu Glu
               300                 305                 310
   GAC CCA GCT CCA TGT GCG TTC TCA GGG AAT GGA CGC CAG TGT ACT GCC      1494
   Asp Pro Ala Pro Cys Ala Phe Ser Gly Asn Gly Arg Gln Cys Thr Ala
           315                 320                 325
   AAT GGC ACG GAA TGT AGG AGT GGC TGG GTT GGC CCG AAC GGA GGC ATC      1542
   Asn Gly Thr Glu Cys Arg Ser Gly Trp Val Gly Pro Asn Gly Gly Ile
       330                 335                 340
   ACC AAC TTT GAT AAC TTT GCC TTT GCC ATG CTT ACT GTG TTT CAG TGC      1590
   Thr Asn Phe Asp Asn Phe Ala Phe Ala Met Leu Thr Val Phe Gln Cys
   345                 350                 355                 360
   ATC ACC ATG GAG GGC TGG ACA GAC GTG CTC TAC TGG ATG AAT GAT GCT      1638
   Ile Thr Met Glu Gly Trp Thr Asp Val Leu Tyr Trp Met Asn Asp Ala
                   365                 370                 375
   ATG GGA TTT GAA TTG CCC TGG GTG TAT TTT GTC AGT CTC GTC ATC TTT      1686
   Met Gly Phe Glu Leu Pro Trp Val Tyr Phe Val Ser Leu Val Ile Phe
               380                 385                 390
   GGG TCA TTT TTC GTA CTA AAT CTT GTA CTT GGT GTA TTG AGC GGA GAA      1734
   Gly Ser Phe Phe Val Leu Asn Leu Val Leu Gly Val Leu Ser Gly Glu
           395                 400                 405
   TTC TCA AAG GAA AGA GAG AAG GCA AAA GCA CGG GGA GAT TTC CAG AAG      1782
   Phe Ser Lys Glu Arg Glu Lys Ala Lys Ala Arg Gly Asp Phe Gln Lys
       410                 415                 420
   CTC CGG GAG AAG CAG CAG CTG GAG GAG GAT CTA AAG GGC TAC TTG GAT      1830
   Leu Arg Glu Lys Gln Gln Leu Glu Glu Asp Leu Lys Gly Tyr Leu Asp
   425                 430                 435                 440
   TGG ATC ACC CAA GCT GAG GAC ATC GAT CCG GAG AAT GAG GAA GAA GGA      1878
   Trp Ile Thr Gln Ala Glu Asp Ile Asp Pro Glu Asn Glu Glu Glu Gly
               445                 450                 455
   GGA GAG GAA GGC AAA CGA AAT ACT AGC ATG CCC ACC AGC GAG ACT GAG      1926
   Gly Glu Glu Gly Lys Arg Asn Thr Ser Met Pro Thr Ser Glu Thr Glu
           460                 465                 470
   TCT GTG AAC ACA GAG AAC GTC AGC GGT GAA GGC GAG AAC CGA GGC TGC      1974
   Ser Val Asn Thr Glu Asn Val Ser Gly Glu Gly Glu Asn Arg Gly Cys
       475                 480                 485
   TGT GGA AGT CTC TGT CAA GCC ATC TCA AAA TCC AAA CTC AGC CGA CGC      2022
   Cys Gly Ser Leu Cys Gln Ala Ile Ser Lys Ser Lys Leu Ser Arg Arg
   490                 495                 500
   TGG CGT CGC TGG AAC CGA TTC AAT CGC AGA AGA TGT AGG GCC GCC GTG      2070
   Trp Arg Arg Trp Asn Arg Phe Asn Arg Arg Arg Cys Arg Ala Ala Val
   505                 510                 515                 520
   AAG TCT GTC ACG TTT TAC TGG CTG GTT ATC GTC CTG GTG TTT CTG AAC      2118
   Lys Ser Val Thr Phe Tyr Trp Leu Val Ile Val Leu Val Phe Leu Asn
               525                 530                 535
   ACC TTA ACC ATT TCC TCT GAG CAC TAC AAT CAG CCA GAT TGG TTG ACA      2166
   Thr Leu Thr Ile Ser Ser Glu His Tyr Asn Gln Pro Asp Trp Leu Thr
           540                 545                 550
   CAG ATT CAA GAT ATT GCC AAC AAA GTC CTC TTG GCT CTG TTC ACC TGC      2214
   Gln Ile Gln Asp Ile Ala Asn Lys Val Leu Leu Ala Leu Phe Thr Cys
       555                 560                 565
   GAG ATG CTG GTA AAA ATG TAC AGC TTG GGC CTC CAA GCA TAT TTC GTC      2262
   Glu Met Leu Val Lys Met Tyr Ser Leu Gly Leu Gln Ala Tyr Phe Val
   570                 575                 580
   TCT CTT TTC AAC CGG TTT GAT TGC TTC GTG GTG TGT GGT GGA ATC ACT      2310
   Ser Leu Phe Asn Arg Phe Asp Cys Phe Val Val Cys Gly Gly Ile Thr
   585                 590                 595                 600
   GAG ACG ATC TTG GTG GAA CTG GAA ATC ATG TCT CCC CTG GGG ATC TCT      2358
   Glu Thr Ile Leu Val Glu Leu Glu Ile Met Ser Pro Leu Gly Ile Ser
               605                 610                 615
   GTG TTT CGG TGT GTG CGC CTC TTA AGA ATC TTC AAA GTG ACC AGG CAC      2406
   Val Phe Arg Cys Val Arg Leu Leu Arg Ile Phe Lys Val Thr Arg His
           620                 625                 630
   TGG ACT TCC CTG AGC AAC TTA GTG GCA TCC TTA TTA AAC TCC ATG AAG      2454
   Trp Thr Ser Leu Ser Asn Leu Val Ala Ser Leu Leu Asn Ser Met Lys
       635                 640                 645
   TCC ATC GCT TCG CTG TTG CTT CTG CTT TTT CTC TTC ATT ATC ATC TTT      2502
   Ser Ile Ala Ser Leu Leu Leu Leu Leu Phe Leu Phe Ile Ile Ile Phe
   650                 655                 660
   TCC TTG CTT GGG ATG CAG CTG TTT GGC GGC AAG TTT AAT TTT GAT GAA      2550
   Ser Leu Leu Gly Met Gln Leu Phe Gly Gly Lys Phe Asn Phe Asp Glu
   665                 670                 675                 680
   ACG CAA ACC AAG CGG AGC ACC TTT GAC AAT TTC CCT CAA GCA CTT CTC      2598
   Thr Gln Thr Lys Arg Ser Thr Phe Asp Asn Phe Pro Gln Ala Leu Leu
               685                 690                 695
   ACA GTG TTC CAG ATC CTG ACA GGC GAA GAC TGG AAT GCT GTG ATG TAC      2646
   Thr Val Phe Gln Ile Leu Thr Gly Glu Asp Trp Asn Ala Val Met Tyr
           700                 705                 710
   GAT GGC ATC ATG GCT TAC GGG GGC CCA TCC TCT TCA GGA ATG ATC GTC 2694
   Asp Gly Ile Met Ala Tyr Gly Gly Pro Ser Ser Ser Gly Met Ile Val
   715 720 725
   TGC ATC TAC TTC ATC ATC CTC TTC ATT TGT GGT AAC TAT ATT CTA CTG 2742
   Cys Ile Tyr Phe Ile Ile Leu Phe Ile Cys Gly Asn Tyr Ile Leu Leu
   730 735 740
   AAT GTC TTC TTG GCC ATC GCT GTA GAC AAT TTG GCT GAT GCT GAA AGT 2790
   Asn Val Phe Leu Ala Ile Ala Val Asp Asn Leu Ala Asp Ala Glu Ser
   745 750 755 760
   CTG AAC ACT GCT CAG AAA GAA GAA GCG GAA GAA AAG GAG AGG AAA AAG 2838
   Leu Asn Thr Ala Gln Lys Glu Glu Ala Glu Glu Lys Glu Arg Lys Lys
   765 770 775
   ATT GCC AGA AAA GAG AGC CTA GAA AAT AAA AAG AAC AAC AAA CCA GAA 2886
   Ile Ala Arg Lys Glu Ser Leu Glu Asn Lys Lys Asn Asn Lys Pro Glu
   780 785 790
   GTC AAC CAG ATA GCC AAC AGT GAC AAC AAG GTT ACA ATT GAT GAC TAT 2934
   Val Asn Gln Ile Ala Asn Ser Asp Asn Lys Val Thr Ile Asp Asp Tyr
   795 800 805
   AGA GAA GAG GAT GAA GAC AAG GAC CCC TAT CCG CCT TGC GAT GTG CCA 2982
   Arg Glu Glu Asp Glu Asp Lys Asp Pro Tyr Pro Pro Cys Asp Val Pro
   810 815 820
   GTA GGG GAA GAG GAA GAG GAA GAG GAG GAG GAT GAA CCT GAG GTT CCT 3030
   Val Gly Glu Glu Glu Glu Glu Glu Glu Glu Asp Glu Pro Glu Val Pro
   825 830 835 840
   GCC GGA CCC CGT CCT CGA AGG ATC TCG GAG TTG AAC ATG AAG GAA AAA 3078
   Ala Gly Pro Arg Pro Arg Arg Ile Ser Glu Leu Asn Met Lys Glu Lys
   845 850 855
   ATT GCC CCC ATC CCT GAA GGG AGC GCT TTC TTC ATT CTT AGC AAG ACC 3126
   Ile Ala Pro Ile Pro Glu Gly Ser Ala Phe Phe Ile Leu Ser Lys Thr
   860 865 870
   AAC CCG ATC CGC GTA GGC TGC CAC AAG CTC ATC AAC CAC CAC ATC TTC 3174
   Asn Pro Ile Arg Val Gly Cys His Lys Leu Ile Asn His His Ile Phe
   875 880 885
   ACC AAC CTC ATC CTT GTC TTC ATC ATG CTG AGC AGT GCT GCC CTG GCC 3222
   Thr Asn Leu Ile Leu Val Phe Ile Met Leu Ser Ser Ala Ala Leu Ala
   890 895 900
   GCA GAG GAC CCC ATC CGC AGC CAC TCC TTC CGG AAC ACG ATA CTG GGT 3270
   Ala Glu Asp Pro Ile Arg Ser His Ser Phe Arg Asn Thr Ile Leu Gly
   905 910 915 920
   TAC TTT GAC TAT GCC TTC ACA GCC ATC TTT ACT GTT GAG ATC CTG TTG 3318
   Tyr Phe Asp Tyr Ala Phe Thr Ala Ile Phe Thr Val Glu Ile Leu Leu
   925 930 935
   AAG ATG ACA ACT TTT GGA GCT TTC CTC CAC AAA GGG GCC TTC TGC AGG 3366
   Lys Met Thr Thr Phe Gly Ala Phe Leu His Lys Gly Ala Phe Cys Arg
   940 945 950
   AAC TAC TTC AAT TTG CTG GAT ATG CTG GTG GTT GGG GTG TCT CTG GTG 3414
   Asn Tyr Phe Asn Leu Leu Asp Met Leu Val Val Gly Val Ser Leu Val
   955 960 965
   TCA TTT GGG ATT CAA TCC AGT GCC ATC TCC GTT GTG AAG ATT CTG AGG 3462
   Ser Phe Gly Ile Gln Ser Ser Ala Ile Ser Val Val Lys Ile Leu Arg
   970 975 980
   GTC TTA AGG GTC CTG CGT CCC CTC AGG GCC ATC AAC AGA GCA AAA GGA 3510
   Val Leu Arg Val Leu Arg Pro Leu Arg Ala Ile Asn Arg Ala Lys Gly
   985                 990                 995                 1000
   CTT AAG CAC GTG GTC CAG TGC GTC TTC GTG GCC ATC CGG ACC ATC GGC       3558
   Leu Lys His Val Val Gln Cys Val Phe Val Ala Ile Arg Thr Ile Gly
                   1005                1010                1015
   AAC ATC ATG ATC GTC ACC ACC CTC CTG CAG TTC ATG TTT GCC TGT ATC       3606
   Asn Ile Met Ile Val Thr Thr Leu Leu Gln Phe Met Phe Ala Cys Ile
               1020                1025                1030
   GGG GTC CAG TTG TTC AAG GGG AAG TTC TAT CGC TGT ACG GAT GAA GCC       3654
   Gly Val Gln Leu Phe Lys Gly Lys Phe Tyr Arg Cys Thr Asp Glu Ala
           1035                1040                1045
   AAA AGT AAC CCT GAA GAA TGC AGG GGA CTT TTC ATC CTC TAC AAG GAT       3702
   Lys Ser Asn Pro Glu Glu Cys Arg Gly Leu Phe Ile Leu Tyr Lys Asp
       1050                1055                1060
   GGG GAT GTT GAC AGT CCT GTG GTC CGT GAA CGG ATC TGG CAA AAC AGT       3750
   Gly Asp Val Asp Ser Pro Val Val Arg Glu Arg Ile Trp Gln Asn Ser
   1065                1070                1075                1080
   GAT TTC AAC TTC GAC AAC GTC CTC TCT GCT ATG ATG GCG CTC TTC ACA       3798
   Asp Phe Asn Phe Asp Asn Val Leu Ser Ala Met Met Ala Leu Phe Thr
                   1085                1090                1095
   GTC TCC ACG TTT GAG GGC TGG CCT GCG TTG CTG TAT AAA GCC ATC GAC       3846
   Val Ser Thr Phe Glu Gly Trp Pro Ala Leu Leu Tyr Lys Ala Ile Asp
               1100                1105                1110
   TCG AAT GGA GAG AAC ATC GGC CCA ATC TAC AAC CAC CGC GTG GAG ATC       3894
   Ser Asn Gly Glu Asn Ile Gly Pro Ile Tyr Asn His Arg Val Glu Ile
           1115                1120                1125
   TCC ATC TTC TTC ATC ATC TAC ATC ATC ATT GTA GCT TTC TTC ATG ATG       3942
   Ser Ile Phe Phe Ile Ile Tyr Ile Ile Ile Val Ala Phe Phe Met Met
       1130                1135                1140
   AAC ATC TTT GTG GGC TTT GTC ATC GTT ACA TTT CAG GAA CAA GGA GAA       3990
   Asn Ile Phe Val Gly Phe Val Ile Val Thr Phe Gln Glu Gln Gly Glu
   1145                1150                1155                1160
   AAA GAG TAT AAG AAC TGT GAG CTG GAC AAA AAT CAG CGT CAG TGT GTT       4038
   Lys Glu Tyr Lys Asn Cys Glu Leu Asp Lys Asn Gln Arg Gln Cys Val
                   1165                1170                1175
   GAA TAC GCC TTG AAA GCA CGT CCC TTG CGG AGA TAC ATC CCC AAA AAC       4086
   Glu Tyr Ala Leu Lys Ala Arg Pro Leu Arg Arg Tyr Ile Pro Lys Asn
               1180                1185                1190
   CCC TAC CAG TAC AAG TTC TGG TAC GTG GTG AAC TCT TCG CCT TTC GAA       4134
   Pro Tyr Gln Tyr Lys Phe Trp Tyr Val Val Asn Ser Ser Pro Phe Glu
           1195                1200                1205
   TAC ATG ATG TTT GTC CTC ATC ATG CTC AAC ACA CTC TGC TTG GCC ATG       4182
   Tyr Met Met Phe Val Leu Ile Met Leu Asn Thr Leu Cys Leu Ala Met
       1210                1215                1220
   CAG CAC TAC GAG CAG TCC AAG ATG TTC AAT GAT GCC ATG GAC ATT CTG       4230
   Gln His Tyr Glu Gln Ser Lys Met Phe Asn Asp Ala Met Asp Ile Leu
   1225                1230                1235                1240
   AAC ATG GTC TTC ACC GGG GTG TTC ACC GTC GAG ATG GTT TTG AAA GTC       4278
   Asn Met Val Phe Thr Gly Val Phe Thr Val Glu Met Val Leu Lys Val
                   1245                1250                1255
   ATC GCA TTT AAG CCT AAG GGG TAT TTT AGT GAC GCC TGG AAC ACG TTT       4326
   Ile Ala Phe Lys Pro Lys Gly Tyr Phe Ser Asp Ala Trp Asn Thr Phe
               1260                1265                1270
   GAC TCC CTC ATC GTA ATC GGC AGC ATT ATA GAC GTG GCC CTC AGC GAA      4374
   Asp Ser Leu Ile Val Ile Gly Ser Ile Ile Asp Val Ala Leu Ser Glu
               1275                1280                1285
   GCA GAC CCA ACT GAA AGT GAA AAT GTC CCT GTC CCA ACT GCT ACA CCT      4422
   Ala Asp Pro Thr Glu Ser Glu Asn Val Pro Val Pro Thr Ala Thr Pro
           1290                1295                1300
   GGG AAC TCT GAA GAG AGC AAT AGA ATC TCC ATC ACC TTT TTC CGT CTT      4470
   Gly Asn Ser Glu Glu Ser Asn Arg Ile Ser Ile Thr Phe Phe Arg Leu
   1305                1310                1315                1320
   TTC CGA GTG ATG CGA TTG GTG AAG CTT CTC AGC AGG GGG GAA GGC ATC      4518
   Phe Arg Val Met Arg Leu Val Lys Leu Leu Ser Arg Gly Glu Gly Ile
                   1325                1330                1335
   CGG ACA TTG CTG TGG ACT TTT ATT AAG TTC TTT CAG GCG CTC CCG TAT      4566
   Arg Thr Leu Leu Trp Thr Phe Ile Lys Phe Phe Gln Ala Leu Pro Tyr
               1340                1345                1350
   GTG GCC CTC CTC ATA GCC ATG CTG TTC TTC ATC TAT GCG GTC ATT GGC      4614
   Val Ala Leu Leu Ile Ala Met Leu Phe Phe Ile Tyr Ala Val Ile Gly
           1355                1360                1365
   ATG CAG ATG TTT GGG AAA GTT GCC ATG AGA GAT AAC AAC CAG ATC AAT      4662
   Met Gln Met Phe Gly Lys Val Ala Met Arg Asp Asn Asn Gln Ile Asn
       1370                1375                1380
   AGG AAC AAT AAC TTC CAG ACG TTT CCC CAG GCG GTG CTG CTG CTC TTC      4710
   Arg Asn Asn Asn Phe Gln Thr Phe Pro Gln Ala Val Leu Leu Leu Phe
   1385                1390                1395                1400
   AGG TGT GCA ACA GGT GAG GCC TGG CAG GAG ATC ATG CTG GCC TGT CTC      4758
   Arg Cys Ala Thr Gly Glu Ala Trp Gln Glu Ile Met Leu Ala Cys Leu
                   1405                1410                1415
   CCA GGG AAG CTC TGT GAC CCT GAG TCA GAT TAC AAC CCC GGG GAG GAG      4806
   Pro Gly Lys Leu Cys Asp Pro Glu Ser Asp Tyr Asn Pro Gly Glu Glu
               1420                1425                1430
   CAT ACA TGT GGG AGC AAC TTT GCC ATT GTC TAT TTC ATC AGT TTT TAC      4854
   His Thr Cys Gly Ser Asn Phe Ala Ile Val Tyr Phe Ile Ser Phe Tyr
           1435                1440                1445
   ATG CTC TGT GCA TTT CTG ATC ATC AAT CTG TTT GTG GCT GTC ATC ATG      4902
   Met Leu Cys Ala Phe Leu Ile Ile Asn Leu Phe Val Ala Val Ile Met
       1450                1455                1460
   GAT AAT TTC GAC TAT CTG ACC CGG GAC TGG TCT ATT TTG GGG CCT CAC      4950
   Asp Asn Phe Asp Tyr Leu Thr Arg Asp Trp Ser Ile Leu Gly Pro His
   1465                1470                1475                1480
   CAT TTA GAT GAA TTC AAA AGA ATA TGG TCA GAA TAT GAC CCT GAG GCA      4998
   His Leu Asp Glu Phe Lys Arg Ile Trp Ser Glu Tyr Asp Pro Glu Ala
                   1485                1490                1495
   AAG GGA AGG ATA AAA CAC CTT GAT GTG GTC ACT CTG CTT CGA CGC ATC      5046
   Lys Gly Arg Ile Lys His Leu Asp Val Val Thr Leu Leu Arg Arg Ile
               1500                1505                1510
   CAG CCT CCC CTG GGG TTT GGG AAG TTA TGT CCA CAC AGG GTA GCG TGC      5094
   Gln Pro Pro Leu Gly Phe Gly Lys Leu Cys Pro His Arg Val Ala Cys
           1515                1520                1525
   AAG AGA TTA GTT GCC ATG AAC ATG CCT CTC AAC AGT GAC GGG ACA GTC      5142
   Lys Arg Leu Val Ala Met Asn Met Pro Leu Asn Ser Asp Gly Thr Val
       1530                1535                1540
   ATG TTT AAT GCA ACC CTG TTT GCT TTG GTT CGA ACG GCT CTT AAG ATC      5190
   Met Phe Asn Ala Thr Leu Phe Ala Leu Val Arg Thr Ala Leu Lys Ile
   1545                1550                1555                1560
   AAG ACC GAA GGG AAC CTG GAG CAA GCT AAT GAA GAA CTT CGG GCT GTG      5238
   Lys Thr Glu Gly Asn Leu Glu Gln Ala Asn Glu Glu Leu Arg Ala Val
                   1565                1570                1575
   ATA AAG AAA ATT TGG AAG AAA ACC AGC ATG AAA TTA CTT GAC CAA GTT      5286
   Ile Lys Lys Ile Trp Lys Lys Thr Ser Met Lys Leu Leu Asp Gln Val
               1580                1585                1590
   GTC CCT CCA GCT GGT GAT GAT GAG GTA ACC GTG GGG AAG TTC TAT GCC      5334
   Val Pro Pro Ala Gly Asp Asp Glu Val Thr Val Gly Lys Phe Tyr Ala
           1595                1600                1605
   ACT TTC CTG ATA CAG GAC TAC TTT AGG AAA TTC AAG AAA CGG AAA GAA      5382
   Thr Phe Leu Ile Gln Asp Tyr Phe Arg Lys Phe Lys Lys Arg Lys Glu
       1610                1615                1620
   CAA GGA CTG GTG GGA AAG TAC CCT GCG AAG AAC ACC ACA ATT GCC CTA      5430
   Gln Gly Leu Val Gly Lys Tyr Pro Ala Lys Asn Thr Thr Ile Ala Leu
   1625                1630                1635                1640
   CAG GCG GGA TTA AGG ACA CTG CAT GAC ATT GGG CCA GAA ATC CGG CGT      5478
   Gln Ala Gly Leu Arg Thr Leu His Asp Ile Gly Pro Glu Ile Arg Arg
                   1645                1650                1655
   GCT ATA TCG TGT GAT TTG CAA GAT GAC GAG CCT GAG GAA ACA AAA CGA      5526
   Ala Ile Ser Cys Asp Leu Gln Asp Asp Glu Pro Glu Glu Thr Lys Arg
               1660                1665                1670
   GAA GAA GAA GAT GAT GTG TTC AAA AGA AAT GGT GCC CTG CTT GGA AAC      5574
   Glu Glu Glu Asp Asp Val Phe Lys Arg Asn Gly Ala Leu Leu Gly Asn
           1675                1680                1685
   CAT GTC AAT CAT GTT AAT AGT GAT AGG AGA GAT TCC CTT CAG CAG ACC      5622
   His Val Asn His Val Asn Ser Asp Arg Arg Asp Ser Leu Gln Gln Thr
       1690                1695                1700
   AAT ACC ACC CAC CGT CCC CTG CAT GTC CAA AGG CCT TCA ATT CCA CCT      5670
   Asn Thr Thr His Arg Pro Leu His Val Gln Arg Pro Ser Ile Pro Pro
   1705                1710                1715                1720
   GCA AGT GAT ACT GAG AAA CCG CTG TTT CCT CCA GCA GGA AAT TCG GTG      5718
   Ala Ser Asp Thr Glu Lys Pro Leu Phe Pro Pro Ala Gly Asn Ser Val
                   1725                1730                1735
   TGT CAT AAC CAT CAT AAC CAT AAT TCC ATA GGA AAG CAA GTT CCC ACC      5766
   Cys His Asn His His Asn His Asn Ser Ile Gly Lys Gln Val Pro Thr
               1740                1745                1750
   TCA ACA AAT GCC AAT CTC AAT AAT GCC AAT ATG TCC AAA GCT GCC CAT      5814
   Ser Thr Asn Ala Asn Leu Asn Asn Ala Asn Met Ser Lys Ala Ala His
           1755                1760                1765
   GGA AAG CGG CCC AGC ATT GGG AAC CTT GAG CAT GTG TCT GAA AAT GGG      5862
   Gly Lys Arg Pro Ser Ile Gly Asn Leu Glu His Val Ser Glu Asn Gly
       1770                1775                1780
   CAT CAT TCT TCC CAC AAG CAT GAC CGG GAG CCT CAG AGA AGG TCC AGT      5910
   His His Ser Ser His Lys His Asp Arg Glu Pro Gln Arg Arg Ser Ser
   1785                1790                1795                1800
   GTG AAA AGA ACC CGC TAT TAT GAA ACT TAC ATT AGG TCC GAC TCA GGA      5958
   Val Lys Arg Thr Arg Tyr Tyr Glu Thr Tyr Ile Arg Ser Asp Ser Gly
                   1805                1810                1815
   GAT GAA CAG CTC CCA ACT ATT TGC CGG GAA GAC CCA GAG ATA CAT GGC      6006
   Asp Glu Gln Leu Pro Thr Ile Cys Arg Glu Asp Pro Glu Ile His Gly
               1820                1825                1830
   TAT TTC AGG GAC CCC CAC TGC TTG GGG GAG CAG GAG TAT TTC AGT AGT      6054
   Tyr Phe Arg Asp Pro His Cys Leu Gly Glu Gln Glu Tyr Phe Ser Ser
               1835                1840                1845
   GAG GAA TGC TAC GAG GAT GAC AGC TCG CCC ACC TGG AGC AGG CAA AAC      6102
   Glu Glu Cys Tyr Glu Asp Asp Ser Ser Pro Thr Trp Ser Arg Gln Asn
           1850                1855                1860
   TAT GGC TAC TAC AGC AGA TAC CCA GGC AGA AAC ATC GAC TCT GAG AGG      6150
   Tyr Gly Tyr Tyr Ser Arg Tyr Pro Gly Arg Asn Ile Asp Ser Glu Arg
   1865                1870                1875                1880
   CCC CGA GGC TAC CAT CAT CCC CAA GGA TTC TTG GAG GAC GAT GAC TCG      6198
   Pro Arg Gly Tyr His His Pro Gln Gly Phe Leu Glu Asp Asp Asp Ser
                   1885                1890                1895
   CCC GTT TGC TAT GAT TCA CGG AGA TCT CCA AGG AGA CGC CTA CTA CCT      6246
   Pro Val Cys Tyr Asp Ser Arg Arg Ser Pro Arg Arg Arg Leu Leu Pro
               1900                1905                1910
   CCC ACC CCA GCA TCC CAC CGG AGA TCC TCC TTC AAC TTT GAG TGC CTG      6294
   Pro Thr Pro Ala Ser His Arg Arg Ser Ser Phe Asn Phe Glu Cys Leu
           1915                1920                1925
   CGC CGG CAG AGC AGC CAG GAA GAG GTC CCG TCG TCT CCC ATC TTC CCC      6342
   Arg Arg Gln Ser Ser Gln Glu Glu Val Pro Ser Ser Pro Ile Phe Pro
       1930                1935                1940
   CAT CGC ACG GCC CTG CCT CTG CAT CTA ATG CAG CAA CAG ATC ATG GCA      6390
   His Arg Thr Ala Leu Pro Leu His Leu Met Gln Gln Gln Ile Met Ala
   1945                1950                1955                1960
   GTT GCC GGC CTA GAT TCA AGT AAA GCC CAG AAG TAC TCA CCG AGT CAC      6438
   Val Ala Gly Leu Asp Ser Ser Lys Ala Gln Lys Tyr Ser Pro Ser His
                   1965                1970                1975
   TCG ACC CGG TCG TGG GCC ACC CCT CCA GCA ACC CCT CCC TAC CGG GAC      6486
   Ser Thr Arg Ser Trp Ala Thr Pro Pro Ala Thr Pro Pro Tyr Arg Asp
               1980                1985                1990
   TGG ACA CCG TGC TAC ACC CCC CTG ATC CAA GTG GAG CAG TCA GAG GCC      6534
   Trp Thr Pro Cys Tyr Thr Pro Leu Ile Gln Val Glu Gln Ser Glu Ala
           1995                2000                2005
   CTG GAC CAG GTG AAC GGC AGC CTG CCG TCC CTG CAC CGC AGC TCC TGG      6582
   Leu Asp Gln Val Asn Gly Ser Leu Pro Ser Leu His Arg Ser Ser Trp
       2010                2015                2020
   TAC ACA GAC GAG CCC GAC ATC TCC TAC CGG ACT TTC ACA CCA GCC AGC      6630
   Tyr Thr Asp Glu Pro Asp Ile Ser Tyr Arg Thr Phe Thr Pro Ala Ser
   2025                2030                2035                2040
   CTG ACT GTC CCC AGC AGC TTC CGG AAC AAA AAC AGC GAC AAG CAG AGG      6678
   Leu Thr Val Pro Ser Ser Phe Arg Asn Lys Asn Ser Asp Lys Gln Arg
                   2045                2050                2055
   AGT GCG GAC AGC TTG GTG GAG GCA GTC CTG ATA TCC GAA GGC TTG GGA      6726
   Ser Ala Asp Ser Leu Val Glu Ala Val Leu Ile Ser Glu Gly Leu Gly
               2060                2065                2070
   CGC TAT GCA AGG GAC CCA AAA TTT GTG TCA GCA ACA AAA CAC GAA ATC      6774
   Arg Tyr Ala Arg Asp Pro Lys Phe Val Ser Ala Thr Lys His Glu Ile
           2075                2080                2085
   GCT GAT GCC TGT GAC CTC ACC ATC GAC GAG ATG GAG AGT GCA GCC AGC      6822
   Ala Asp Ala Cys Asp Leu Thr Ile Asp Glu Met Glu Ser Ala Ala Ser
       2090                2095                2100
   ACC CTG CTT AAT GGG AAC GTG CGT CCC CGA GCC AAC GGG GAT GTG GGC
   Thr Leu Leu Asn Gly Asn Val Arg Pro Arg Ala Asn Gly Asp Val Gly
   2105                2110                2115                2120
   CCC CTC TCA CAC CGG CAG GAC TAT GAG CTA CAG GAC TTT GGT CCT GGC      6918
   Pro Leu Ser His Arg Gln Asp Tyr Glu Leu Gln Asp Phe Gly Pro Gly
                   2125                2130                2135
   TAC AGC GAC GAA GAG CCA GAC CCT GGG AGG GAT GAG GAG GAC CTG GCG      6966
   Tyr Ser Asp Glu Glu Pro Asp Pro Gly Arg Asp Glu Glu Asp Leu Ala
               2140                2145                2150
   GAT GAA ATG ATA TGC ATC ACC ACC TTG TAGCCCCCAG CGAGGGGCAG            7013
   Asp Glu Met Ile Cys Ile Thr Thr Leu
           2155                2160
   ACTGGCTCTG GCCTCAGGTG GGGCGCAGGA GAGCCAGGGG AAAAGTGCCT CATAGTTAGG    7073
   AAAGTTTAGG CACTAGTTGG GAGTAATATT CAATTAATTA GACTTTTGTA TAAGAGATGT    7133
   CATGCCTCAA GAAAGCCATA AACCTGGTAG GAACAGGTCC CAAGCGGTTG AGCCTGGCAG    7193
   AGTACCATGC GCTCGGCCCC AGCTGCAGGA AACAGCAGGC CCCGCCCTCT CACAGAGGAT    7253
   GGGTGAGGAG GCCAGACCTG CCCTGCCCCA TTGTCCAGAT GGGCACTGCT GTGGAGTCTG    7313
   CTTCTCCCAT GTACCAGGGC ACCAGGCCCA CCCAACTGAA GGCATGGCGG CGGGGTGCAG    7373
   GGGAAAGTTA AAGGTGATGA CGATCATCAC ACCTGTGTCG TTACCTCAGC CATCGGTCTA    7433
   GCATATCAGT CACTGGGCCC AACATATCCA TTTTTAAACC CTTTCCCCCA AATACACTGC    7493
   GTCCTGGTTC CTGTTTAGCT GTTCTGAAAT ACGGTGTGTA AGTAAGTCAG AACCCAGCTA    7553
   CCAGTGATTA TTGCGAGGGC AATGGGACCT CATAAATAAG GTTTTCTGTG ATGTGACGCC    7613
   AGTTTACATA AGAGAATATC AC                                             7635

### (2) INFORMATION FOR SEQ ID NO:2:

(i) SEQUENCE CHARACTERISTICS:
   (A) LENGTH: 104 base pairs
   (B) TYPE: nucleic acid
   (C) STRANDEDNESS: double
   (D) TOPOLOGY: linear
(ii) MOLECULE TYPE: DNA (genomic)
(ix) FEATURE:
   (A) NAME/KEY: CDS
   (B) LOCATION: 1. 102
(ix) FEATURE:
   (A) NAME/KEY: misc feature
   (B) LOCATION: 1..104
   (D) OTHER INFORMATION: /note= "A 104-nucleotide alternative exon of alpha-1D."
(xi) SEQUENCE DESCRIPTION: SEQ ID NO:2:
   GTA AAT GAT GCG ATA GGA TGG GAA TGG CCA TGG GTG TAT TTT GTT AGT      48
   Val Asn Asp Ala Ile Gly Trp Glu Trp Pro Trp Val Tyr Phe Val Ser
     1               5                  10                  15
   CTG ATC ATC CTT GGC TCA TTT TTC GTC CTT AAC CTG GTT CTT GGT GTC      96
   Leu Ile Ile Leu Gly Ser Phe Phe Val Leu Asn Leu Val Leu Gly Val
                20                  25                  30
   CTT AGT GG                                                          104
   Leu Ser

### (2) INFORMATION FOR SEQ ID NO:3:

(i) SEQUENCE CHARACTERISTICS:
   (A) LENGTH: 5904 base pairs
   (B) TYPE: nucleic acid
   (C) STRANDEDNESS: single
   (D) TOPOLOGY: linear
(ii) MOLECULE TYPE: DNA (genomic)
(ix) FEATURE:
   (A) NAME/KEY: CDS
   (B) LOCATION: 1..5904
(xi) SEQUENCE DESCRIPTION: SEQ ID NO:3:
   ATG GTC AAT GAG AAT ACG AGG ATG TAC ATT CCA GAG GAA AAC CAC CAA      48
   Met Val Asn Glu Asn Thr Arg Met Tyr Ile Pro Glu Glu Asn His Gln
    1               5                  10                  15
   GGT TCC AAC TAT GGG AGC CCA CGC CCC GCC CAT GCC AAC ATG AAT GCC      96
   Gly Ser Asn Tyr Gly Ser Pro Arg Pro Ala His Ala Asn Met Asn Ala
               20                  25                  30
   AAT GCG GCA GCG GGG CTG GCC CCT GAG CAC ATC CCC ACC CCG GGG GCT     144
   Asn Ala Ala Ala Gly Leu Ala Pro Glu His Ile Pro Thr Pro Gly Ala
           35                  40                  45
   GCC CTG TCG TGG CAG GCG GCC ATC GAC GCA GCC CGG CAG GCT AAG CTG     192
   Ala Leu Ser Trp Gln Ala Ala Ile Asp Ala Ala Arg Gln Ala Lys Leu
       50                  55                  60
   ATG GGC AGC GCT GGC AAT GCG ACC ATC TCC ACA GTC AGC TCC ACG CAG     240
   Met Gly Ser Ala Gly Asn Ala Thr Ile Ser Thr Val Ser Ser Thr Gln
    65                 70                  75                  80
   CGG AAG CGC CAG CAA TAT GGG AAA CCC AAG AAG CAG GGC AGC ACC ACG     288
   Arg Lys Arg Gln Gln Tyr Gly Lys Pro Lys Lys Gln Gly Ser Thr Thr
                   85                  90                  95
   GCC ACA CGC CCG CCC CGA GCC CTG CTC TGC CTG ACC CTG AAG AAC CCC     336
   Ala Thr Arg Pro Pro Arg Ala Leu Leu Cys Leu Thr Leu Lys Asn Pro
               100                 105                 110
   ATC CGG AGG GCC TGC ATC AGC ATT GTC GAA TGG AAA CCA TTT GAA ATA     384
   Ile Arg Arg Ala Cys Ile Ser Ile Val Glu Trp Lys Pro Phe Glu Ile
           115                 120                 125
   ATT ATT TTA CTG ACT ATT TTT GCC AAT TGT GTG GCC TTA GCG ATC TAT     432
   Ile Ile Leu Leu Thr Ile Phe Ala Asn Cys Val Ala Leu Ala Ile Tyr
       130                 135                 140
   ATT CCC TTT CCA GAA GAT GAT TCC AAC GCC ACC AAT TCC AAC CTG GAA     480
   Ile Pro Phe Pro Glu Asp Asp Ser Asn Ala Thr Asn Ser Asn Leu Glu
   145                 150                 155                 160
   CGA GTG GAA TAT CTC TTT CTC ATA ATT TTT ACG GTG GAA GCG TTT TTA     528
   Arg Val Glu Tyr Leu Phe Leu Ile Ile Phe Thr Val Glu Ala Phe Leu
                   165                 170                 175
   AAA GTA ATC GCC TAT GGA CTC CTC TTT CAC CCC AAT GCC TAC CTC CGC     576
   Lys Val Ile Ala Tyr Gly Leu Leu Phe His Pro Asn Ala Tyr Leu Arg
               180                 185                 190
   AAC GGC TGG AAC CTA CTA GAT TTT ATA ATT GTG GTT GTG GGG CTT TTT     624
   Asn Gly Trp Asn Leu Leu Asp Phe Ile Ile Val Val Val Gly Leu Phe
           195                 200                 205
   AGT GCA ATT TTA GAA CAA GCA ACC AAA GCA GAT GGG GCA AAC GCT CTC          672
   Ser Ala Ile Leu Glu Gln Ala Thr Lys Ala Asp Gly Ala Asn Ala Leu
       210                 215                 220
   GGA GGG AAA GGG GCC GGA TTT GAT GTG AAG GCG CTG AGG GCC TTC CGC          720
   Gly Gly Lys Gly Ala Gly Phe Asp Val Lys Ala Leu Arg Ala Phe Arg
   225                 230                 235                 240
   GTG CTG CGC CCC CTG CGG CTG GTG TCC GGA GTC CCA AGT CTC CAG GTG          768
   Val Leu Arg Pro Leu Arg Leu Val Ser Gly Val Pro Ser Leu Gln Val
                   245                 250                 255
   GTC CTG AAT TCC ATC ATC AAG GCC ATG GTC CCC CTG CTG CAC ATC GCC          816
   Val Leu Asn Ser Ile Ile Lys Ala Met Val Pro Leu Leu His Ile Ala
               260                 265                 270
   CTG CTT GTG CTG TTT GTC ATC ATC ATC TAC GCC ATC ATC GGC TTG GAG          864
   Leu Leu Val Leu Phe Val Ile Ile Ile Tyr Ala Ile Ile Gly Leu Glu
           275                 280                 285
   CTC TTC ATG GGG AAG ATG CAC AAG ACC TGC TAC AAC CAG GAG GGC ATA          912
   Leu Phe Met Gly Lys Met His Lys Thr Cys Tyr Asn Gln Glu Gly Ile
       290                 295                 300
   GCA GAT GTT CCA GCA GAA GAT GAC CCT TCC CCT TGT GCG CTG GAA ACG          960
   Ala Asp Val Pro Ala Glu Asp Asp Pro Ser Pro Cys Ala Leu Glu Thr
   305                 310                 315                 320
   GGC CAC GGG CGG CAG TGC CAG AAC GGC ACG GTG TGC AAG CCC GGC TGG         1008
   Gly His Gly Arg Gln Cys Gln Asn Gly Thr Val Cys Lys Pro Gly Trp
                   325                 330                 335
   GAT GGT CCC AAG CAC GGC ATC ACC AAC TTT GAC AAC TTT GCC TTC GCC         1056
   Asp Gly Pro Lys His Gly Ile Thr Asn Phe Asp Asn Phe Ala Phe Ala
               340                 345                 350
   ATG CTC ACG GTG TTC CAG TGC ATC ACC ATG GAG GGC TGG ACG GAC GTG         1104
   Met Leu Thr Val Phe Gln Cys Ile Thr Met Glu Gly Trp Thr Asp Val
           355                 360                 365
   CTG TAC TGG GTC AAT GAT GCC GTA GGA AGG GAC TGG CCC TGG ATC TAT         1152
   Leu Tyr Trp Val Asn Asp Ala Val Gly Arg Asp Trp Pro Trp Ile Tyr
       370                 375                 380
   TTT GTT ACA CTA ATC ATC ATA GGG TCA TTT TTT GTA CTT AAC TTG GTT         1200
   Phe Val Thr Leu Ile Ile Ile Gly Ser Phe Phe Val Leu Asn Leu Val
   385                 390                 395                 400
   CTC GGT GTG CTT AGC GGA GAG TTT TCC AAA GAG AGG GAG AAG GCC AAG         1248
   Leu Gly Val Leu Ser Gly Glu Phe Ser Lys Glu Arg Glu Lys Ala Lys
                   405                 410                 415
   GCC CGG GGA GAT TTC CAG AAG CTG CGG GAG AAG CAG CAG CTA GAA GAG         1296
   Ala Arg Gly Asp Phe Gln Lys Leu Arg Glu Lys Gln Gln Leu Glu Glu
               420                 425                 430
   GAT CTC AAA GGC TAC CTG GAT TGG ATC ACT CAG GCC GAA GAC ATC GNT         1344
   Asp Leu Lys Gly Tyr Leu Asp Trp Ile Thr Gln Ala Glu Asp Ile Xaa
           435                 440                 445
   CCT GAG AAT GAG GAC GAA GGC ATG GAT GAG GAG AAG CCC CGA AAC AGA         1392
   Pro Glu Asn Glu Asp Glu Gly Met Asp Glu Glu Lys Pro Arg Asn Arg
       450                 455                 460
   GGC ACT CCG GCG GGC ATG CTT GAT CAG AAG AAA GGG AAG TTT GCT TGG         1440
   Gly Thr Pro Ala Gly Met Leu Asp Gln Lys Lys Gly Lys Phe Ala Trp
   465                 470                 475                 480
   TTT AGT CAC TCC ACA GAA ACC CAT GTG AGC ATG CCC ACC AGT GAG ACC      1488
   Phe Ser His Ser Thr Glu Thr His Val Ser Met Pro Thr Ser Glu Thr
                   485                 490                 495
   GAG TCC GTC AAC ACC GAA AAC GTG GCT GGA GGT GAC ATC GAG GGA GAA      1536
   Glu Ser Val Asn Thr Glu Asn Val Ala Gly Gly Asp Ile Glu Gly Glu
               500                 505                 510
   AAC TGC GGG GCC AGG CTG GCC CAC CGG ATC TCC AAG TCA AAG TTC AGC      1584
   Asn Cys Gly Ala Arg Leu Ala His Arg Ile Ser Lys Ser Lys Phe Ser
           515                 520                 525
   CGC TAC TGG CGC CGG TGG AAT CGG TTC TGC AGA AGG AAG TGC CGC GCC      1632
   Arg Tyr Trp Arg Arg Trp Asn Arg Phe Cys Arg Arg Lys Cys Arg Ala
       530                 535                 540
   GCA GTC AAG TCT AAT GTC TTC TAC TGG CTG GTG ATT TTC CTG GTG TTC      1680
   Ala Val Lys Ser Asn Val Phe Tyr Trp Leu Val Ile Phe Leu Val Phe
   545                 550                 555                 560
   CTC AAC ACG CTC ACC ATT GCC TCT GAG CAC TAC AAC CAG CCC AAC TGG      1728
   Leu Asn Thr Leu Thr Ile Ala Ser Glu His Tyr Asn Gln Pro Asn Trp
                   565                 570                 575
   CTC ACA GAA GTC CAA GAC ACG GCA AAC AAG GCC CTG CTG GCC CTG TTC      1776
   Leu Thr Glu Val Gln Asp Thr Ala Asn Lys Ala Leu Leu Ala Leu Phe
               580                 585                 590
   ACG GCA GAG ATG CTC CTG AAG ATG TAC AGC CTG GGC CTG CAG GCC TAC      1824
   Thr Ala Glu Met Leu Leu Lys Met Tyr Ser Leu Gly Leu Gln Ala Tyr
           595                 600                 605
   TTC GTG TCC CTC TTC AAC CGC TTT GAC TGC TTC GTC GTG TGT GGC GGC      1872
   Phe Val Ser Leu Phe Asn Arg Phe Asp Cys Phe Val Val Cys Gly Gly
       610                 615                 620
   ATC CTG GAG ACC ATC CTG GTG GAG ACC AAG ATC ATG TCC CCA CTG GGC      1920
   Ile Leu Glu Thr Ile Leu Val Glu Thr Lys Ile Met Ser Pro Leu Gly
   625                 630                 635                 640
   ATC TCC GTG CTC AGA TGC GTC CGG CTG CTG AGG ATT TTC AAG ATC ACG      1968
   Ile Ser Val Leu Arg Cys Val Arg Leu Leu Arg Ile Phe Lys Ile Thr
                   645                 650                 655
   AGG TAC TGG AAC TCC TTG AGC AAC CTG GTG GCA TCC TTG CTG AAC TCT      2016
   Arg Tyr Trp Asn Ser Leu Ser Asn Leu Val Ala Ser Leu Leu Asn Ser
               660                 665                 670
   GTG CGC TCC ATC GCC TCC CTG CTC CTT CTC CTC TTC CTC TTC ATC ATC      2064
   Val Arg Ser Ile Ala Ser Leu Leu Leu Leu Leu Phe Leu Phe Ile Ile
           675                 680                 685
   ATC TTC TCC CTC CTG GGG ATG CAG CTC TTT GGA GGA AAG TTC AAC TTT      2112
   Ile Phe Ser Leu Leu Gly Met Gln Leu Phe Gly Gly Lys Phe Asn Phe
       690                 695                 700
   GAT GAG ATG CAG ACC CGG AGG AGC ACA TTC GAT AAC TTC CCC CAG TCC      2160
   Asp Glu Met Gln Thr Arg Arg Ser Thr Phe Asp Asn Phe Pro Gln Ser
   705                 710                 715                 720
   CTC CTC ACT GTG TTT CAG ATC CTG ACC GGG GAG GAC TGG AAT TCG GTG      2208
   Leu Leu Thr Val Phe Gln Ile Leu Thr Gly Glu Asp Trp Asn Ser Val
                   725                 730                 735
   ATG TAT GAT GGG ATC ATG GCT TAT GGG GGC CCC TCT TTT CCA GGG ATG      2256
   Met Tyr Asp Gly Ile Met Ala Tyr Gly Gly Pro Ser Phe Pro Gly Met
               740                 745                 750
   TTA GTC TGT ATT TAC TTC ATC ATC CTC TTC ATC TCT GGA AAC TAT ATC      2304
   Leu Val Cys Ile Tyr Phe Ile Ile Leu Phe Ile Ser Gly Asn Tyr Ile
           755                 760                 765
   CTA CTG AAT GTG TTC TTG GCC ATT GCT GTG GAC AAC CTG GCT GAT GCT      2352
   Leu Leu Asn Val Phe Leu Ala Ile Ala Val Asp Asn Leu Ala Asp Ala
       770                 775                 780
   GAG AGC CTC ACA TCT GCC CTA AAG GAG GAG GAA GAG GAG AAG GAG AGA      2400
   Glu Ser Leu Thr Ser Ala Leu Lys Glu Glu Glu Glu Glu Lys Glu Arg
   785                 790                 795                 800
   AAG AAG CTG GCC AGG ACT GCC AGC CCA GAG AAG AAA CAA GAG TTG GTG      2448
   Lys Lys Leu Ala Arg Thr Ala Ser Pro Glu Lys Lys Gln Glu Leu Val
                   805                 810                 815
   GAG AAG CCG GCA GTG GGG GAA TCC AAG GAG GAG AAG ATT GAG CTG AAA      2496
   Glu Lys Pro Ala Val Gly Glu Ser Lys Glu Glu Lys Ile Glu Leu Lys
               820                 825                 830
   TCC ATC ACG GCT GAC GGA GAG TCT CCA CCC GCC ACC AAG ATC AAC ATG      2544
   Ser Ile Thr Ala Asp Gly Glu Ser Pro Pro Ala Thr Lys Ile Asn Met
           835                 840                 845
   GAT GAC CTC CAG CCC AAT GAA AAT GAG GAT AAG AGC CCC TAC CCC AAC      2592
   Asp Asp Leu Gln Pro Asn Glu Asn Glu Asp Lys Ser Pro Tyr Pro Asn
       850                 855                 860
   CCA GAA ACT ACA GGA GAA GAG GAT GAG GAG GAG CCA GAG ATG CCT GTC      2640
   Pro Glu Thr Thr Gly Glu Glu Asp Glu Glu Glu Pro Glu Met Pro Val
   865                 870                 875                 880
   GGC CCT CGC CCA CGA CCA CTC TCT GAG CTT CAC CTT AAG GAA AAG GCA      2688
   Gly Pro Arg Pro Arg Pro Leu Ser Glu Leu His Leu Lys Glu Lys Ala
                   885                 890                 895
   GTG CCC ATG CCA GAA GCC AGC GCG TTT TTC ATC TTC AGC TCT AAC AAC      2736
   Val Pro Met Pro Glu Ala Ser Ala Phe Phe Ile Phe Ser Ser Asn Asn
               900                 905                 910
   AGG TTT CGC CTC CAG TGC CAC CGC ATT GTC AAT GAC ACG ATC TTC ACC      2784
   Arg Phe Arg Leu Gln Cys His Arg Ile Val Asn Asp Thr Ile Phe Thr
           915                 920                 925
   AAC CTG ATC CTC TTC TTC ATT CTG CTC AGC AGC ATT TCC CTG GCT GCT      2832
   Asn Leu Ile Leu Phe Phe Ile Leu Leu Ser Ser Ile Ser Leu Ala Ala
       930                 935                 940
   GAG GAC CCG GTC CAG CAC ACC TCC TTC AGG AAC CAT ATT CTG TTT TAT      2880
   Glu Asp Pro Val Gln His Thr Ser Phe Arg Asn His Ile Leu Phe Tyr
   945                 950                 955                 960
   TTT GAT ATT GTT TTT ACC ACC ATT TTC ACC ATT GAA ATT GCT CTG AAG      2928
   Phe Asp Ile Val Phe Thr Thr Ile Phe Thr Ile Glu Ile Ala Leu Lys
                   965                 970                 975
   ATG ACT GCT TAT GGG GCT TTC TTG CAC AAG GGT TCT TTC TGC CGG AAC      2976
   Met Thr Ala Tyr Gly Ala Phe Leu His Lys Gly Ser Phe Cys Arg Asn
               980                 985                 990
   TAC TTC AAC ATC CTG GAC CTG CTG GTG GTC AGC GTG TCC CTC ATC TCC      3024
   Tyr Phe Asn Ile Leu Asp Leu Leu Val Val Ser Val Ser Leu Ile Ser
           995                 1000                1005
   TTT GGC ATC CAG TCC AGT GCA ATC AAT GTC GTG AAG ATC TTG CGA GTC      3072
   Phe Gly Ile Gln Ser Ser Ala Ile Asn Val Val Lys Ile Leu Arg Val
       1010                1015                1020
   CTG CGA GTA CTC AGG CCC CTG AGG GCC ATC AAC AGG GCC AAG GGG CTA      3120
   Leu Arg Val Leu Arg Pro Leu Arg Ala Ile Asn Arg Ala Lys Gly Leu
   1025                1030                1035                1040
   AAG CAT GTG GTT CAG TGT GTG TTT GTC GCC ATC CGG ACC ATC GGG AAC      3168
   Lys His Val Val Gln Cys Val Phe Val Ala Ile Arg Thr Ile Gly Asn
                   1045                1050                1055
   ATC GTG ATT GTC ACC ACC CTG CTG CAG TTC ATG TTT GCC TGC ATC GGG      3216
   Ile Val Ile Val Thr Thr Leu Leu Gln Phe Met Phe Ala Cys Ile Gly
               1060                1065                1070
   GTC CAG CTC TTC AAG GGA AAG CTG TAC ACC TGT TCA GAC AGT TCC AAG      3264
   Val Gln Leu Phe Lys Gly Lys Leu Tyr Thr Cys Ser Asp Ser Ser Lys
           1075                1080                1085
   CAG ACA GAG GCG GAA TGC AAG GGC AAC TAC ATC ACG TAC AAA GAC GGG      3312
   Gln Thr Glu Ala Glu Cys Lys Gly Asn Tyr Ile Thr Tyr Lys Asp Gly
       1090                1095                1100
   GAG GTT GAC CAC CCC ATC ATC CAA CCC CGC AGC TGG GAG AAC AGC AAG      3360
   Glu Val Asp His Pro Ile Ile Gln Pro Arg Ser Trp Glu Asn Ser Lys
   1105                1110                1115                1120
   TTT GAC TTT GAC AAT GTT CTG GCA GCC ATG ATG GCC CTC TTC ACC GTC      3408
   Phe Asp Phe Asp Asn Val Leu Ala Ala Met Met Ala Leu Phe Thr Val
                   1125                1130                1135
   TCC ACC TTC GAA GGG TGG CCA GAG CTG CTG TAC CGC TCC ATC GAC TCC      3456
   Ser Thr Phe Glu Gly Trp Pro Glu Leu Leu Tyr Arg Ser Ile Asp Ser
               1140                1145                1150
   CAC ACG GAA GAC AAG GGC CCC ATC TAC AAC TAC CGT GTG GAG ATC TCC      3504
   His Thr Glu Asp Lys Gly Pro Ile Tyr Asn Tyr Arg Val Glu Ile Ser
           1155                1160                1165
   ATC TTC TTC ATC ATC TAC ATC ATC ATC ATC GCC TTC TTC ATG ATG AAC      3552
   Ile Phe Phe Ile Ile Tyr Ile Ile Ile Ile Ala Phe Phe Met Met Asn
       1170                1175                1180
   ATC TTC GTG GGC TTC GTC ATC GTC ACC TTT CAG GAG CAG GGG GAG CAG      3600
   Ile Phe Val Gly Phe Val Ile Val Thr Phe Gln Glu Gln Gly Glu Gln
   1185                1190                1195                1200
   GAG TAC AAG AAC TGT GAG CTG GAC AAG AAC CAG CGA CAG TGC GTG GAA      3648
   Glu Tyr Lys Asn Cys Glu Leu Asp Lys Asn Gln Arg Gln Cys Val Glu
                   1205                1210                1215
   TAC GCC CTC AAG GCC CGG CCC CTG CGG AGG TAC ATC CCC AAG AAC CAG      3696
   Tyr Ala Leu Lys Ala Arg Pro Leu Arg Arg Tyr Ile Pro Lys Asn Gln
               1220                1225                1230
   CAC CAG TAC AAA GTG TGG TAC GTG GTC AAC TCC ACC TAC TTC GAG TAC      3744
   His Gln Tyr Lys Val Trp Tyr Val Val Asn Ser Thr Tyr Phe Glu Tyr
           1235                1240                1245
   CTG ATG TTC GTC CTC ATC CTG CTC AAC ACC ATC TGC CTG GCC ATG CAG      3792
   Leu Met Phe Val Leu Ile Leu Leu Asn Thr Ile Cys Leu Ala Met Gln
       1250                1255                1260
   CAC TAC GGC CAG AGC TGC CTG TTC AAA ATC GCC ATG AAC ATC CTC AAC      3840
   His Tyr Gly Gln Ser Cys Leu Phe Lys Ile Ala Met Asn Ile Leu Asn
   1265                1270                1275                1280
   ATG CTC TTC ACT GGC CTC TTC ACC GTG GAG ATG ATC CTG AAG CTC ATT      3888
   Met Leu Phe Thr Gly Leu Phe Thr Val Glu Met Ile Leu Lys Leu Ile
                   1285                1290                1295
   GCC TTC AAA CCC AAG GGT TAC TTT AGT GAT CCC TGG AAT GTT TTT GAC      3936
   Ala Phe Lys Pro Lys Gly Tyr Phe Ser Asp Pro Trp Asn Val Phe Asp
               1300                1305                1310
   TTC CTC ATC GTA ATT GGC AGC ATA ATT GAC GTC ATT CTC AGT GAG ACT      3984
   Phe Leu Ile Val Ile Gly Ser Ile Ile Asp Val Ile Leu Ser Glu Thr
           1315                1320                1325
   AAT CCA GCT GAA CAT ACC CAA TGC TCT CCC TCT ATG AAC GCA GAG GAA      4032
   Asn Pro Ala Glu His Thr Gln Cys Ser Pro Ser Met Asn Ala Glu Glu
       1330                1335                1340
   AAC TCC CGC ATC TCC ATC ACC TTC TTC CGC CTG TTC CGG GTC ATG CGT      4080
   Asn Ser Arg Ile Ser Ile Thr Phe Phe Arg Leu Phe Arg Val Met Arg
   1345                1350                1355                1360
   CTG GTG AAG CTG CTG AGC CGT GGG GAG GGC ATC CGG ACG CTG CTG TGG      4128
   Leu Val Lys Leu Leu Ser Arg Gly Glu Gly Ile Arg Thr Leu Leu Trp
                   1365                1370                1375
   ACC TTC ATC AAG TCC TTC CAG GCC CTG CCC TAT GTG GCC CTC CTG ATC      4176
   Thr Phe Ile Lys Ser Phe Gln Ala Leu Pro Tyr Val Ala Leu Leu Ile
               1380                1385                1390
   GTG ATG CTG TTC TTC ATC TAC GCG GTG ATC GGG ATG CAG GTG TTT GGG      4224
   Val Met Leu Phe Phe Ile Tyr Ala Val Ile Gly Met Gln Val Phe Gly
           1395                1400                1405
   AAA ATT GCC CTG AAT GAT ACC ACA GAG ATC AAC CGG AAC AAC AAC TTT      4272
   Lys Ile Ala Leu Asn Asp Thr Thr Glu Ile Asn Arg Asn Asn Asn Phe
       1410                1415                1420
   CAG ACC TTC CCC CAG GCC GTG CTG CTC CTC TTC AGG TGT GCC ACC GGG      4320
   Gln Thr Phe Pro Gln Ala Val Leu Leu Leu Phe Arg Cys Ala Thr Gly
   1425                1430                1435                1440
   GAG GCC TGG CAG GAC ATC ATG CTG GCC TGC ATG CCA GGC AAG AAG TGT      4368
   Glu Ala Trp Gln Asp Ile Met Leu Ala Cys Met Pro Gly Lys Lys Cys
                   1445                1450                1455
   GCC CCA GAG TCC GAG CCC AGC AAC AGC ACG GAG GGT GAA ACA CCC TGT      4416
   Ala Pro Glu Ser Glu Pro Ser Asn Ser Thr Glu Gly Glu Thr Pro Cys
               1460                1465                1470
   GGT AGC AGC TTT GCT GTC TTC TAC TTC ATC AGC TTC TAC ATG CGC TGT      4464
   Gly Ser Ser Phe Ala Val Phe Tyr Phe Ile Ser Phe Tyr Met Arg Cys
           1475                1480                1485
   GCC TTC CTG ATC ATC AAC CTC TTT GTA GCT GTC ATC ATG GAC AAC TTT      4512
   Ala Phe Leu Ile Ile Asn Leu Phe Val Ala Val Ile Met Asp Asn Phe
       1490                1495                1500
   GAC TAC CTG ACA AGG GAC TGG TCC ATC CTT GGT CCC CAC CAC CTG GAT      4560
   Asp Tyr Leu Thr Arg Asp Trp Ser Ile Leu Gly Pro His His Leu Asp
   1505                1510                1515                1520
   GAG TTT AAA AGA ATC TGG GCA GAG TAT GAC CCT GAA GCC AAG GGT CGT      4608
   Glu Phe Lys Arg Ile Trp Ala Glu Tyr Asp Pro Glu Ala Lys Gly Arg
                   1525                1530                1535
   ATC AAA CAC CTG GAT GTG GTG ACC CTC CTC CGG CGG ATT CAG CCG CCA      4656
   Ile Lys His Leu Asp Val Val Thr Leu Leu Arg Arg Ile Gln Pro Pro
               1540                1545                1550
   CTA GGT TTT GGG AAG CTG TGC CCT CAC CGC GTG GCT TGC AAA CGC CTG      4704
   Leu Gly Phe Gly Lys Leu Cys Pro His Arg Val Ala Cys Lys Arg Leu
           1555                1560                1565
   GTC TCC ATG AAC ATG CCT CTG AAC AGC GAC GGG ACA GTC ATG TTC AAT      4752
   Val Ser Met Asn Met Pro Leu Asn Ser Asp Gly Thr Val Met Phe Asn
       1570                1575                1580
   GCC ACC CTG TTT GCC CTG GTC AGG ACG GCC CTG AGG ATC AAA ACA GAA      4800
   Ala Thr Leu Phe Ala Leu Val Arg Thr Ala Leu Arg Ile Lys Thr Glu
   1585                1590                1595                1600
   GGG AAC CTA GAA CAA GCC AAT GAG GAG CTG CGG GCG ATC ATC AAG AAG     4848
   Gly Asn Leu Glu Gln Ala Asn Glu Glu Leu Arg Ala Ile Ile Lys Lys
                   1605                1610                1615
   ATC TGG AAG CGG ACC AGC ATG AAG CTG CTG GAC CAG GTG GTG CCC CCT     4896
   Ile Trp Lys Arg Thr Ser Met Lys Leu Leu Asp Gln Val Val Pro Pro
               1620                1625                1630
   GCA GGT GAT GAT GAG GTC ACC GTT GGC AAG TTC TAC GCC ACG TTC CTG     4944
   Ala Gly Asp Asp Glu Val Thr Val Gly Lys Phe Tyr Ala Thr Phe Leu
           1635                1640                1645
   ATC CAG GAG TAC TTC CGG AAG TTC AAG AAG CGC AAA GAG CAG GGC CTT     4992
   Ile Gln Glu Tyr Phe Arg Lys Phe Lys Lys Arg Lys Glu Gln Gly Leu
       1650                1655                1660
   GTG GGC AAG CCC TCC CAG AGG AAC GCG CTG TCT CTG CAG GCT GGC TTG     5040
   Val Gly Lys Pro Ser Gln Arg Asn Ala Leu Ser Leu Gln Ala Gly Leu
   1665                1670                1675                1680
   CGC ACA CTG CAT GAC ATC GGG CCT GAG ATC CGA CGG GCC ATC TCT GGA     5088
   Arg Thr Leu His Asp Ile Gly Pro Glu Ile Arg Arg Ala Ile Ser Gly
                   1685                1690                1695
   GAT CTC ACC GCT GAG GAG GAG CTG GAC AAG GCC ATG AAG GAG GCT GTG     5136
   Asp Leu Thr Ala Glu Glu Glu Leu Asp Lys Ala Met Lys Glu Ala Val
               1700                1705                1710
   TCC GCT GCT TCT GAA GAT GAC ATC TTC AGG AGG GCC GGT GGC CTG TTC     5184
   Ser Ala Ala Ser Glu Asp Asp Ile Phe Arg Arg Ala Gly Gly Leu Phe
           1715                1720                1725
   GGC AAC CAC GTC AGC TAC TAC CAA AGC GAC GGC CGG AGC GCC TTC CCC     5232
   Gly Asn His Val Ser Tyr Tyr Gln Ser Asp Gly Arg Ser Ala Phe Pro
       1730                1735                1740
   CAG ACC TTC ACC ACT CAG CGC CCG CTG CAC ATC AAC AAG GCG GGC AGC     5280
   Gln Thr Phe Thr Thr Gln Arg Pro Leu His Ile Asn Lys Ala Gly Ser
   1745                1750                1755                1760
   AGC CAG GGC GAC ACT GAG TCG CCA TCC CAC GAG AAG CTG GTG GAC TCC     5328
   Ser Gln Gly Asp Thr Glu Ser Pro Ser His Glu Lys Leu Val Asp Ser
                   1765                1770                1775
   ACC TTC ACC CCG AGC AGC TAC TCG TCC ACC GGC TCC AAC GCC AAC ATC     5376
   Thr Phe Thr Pro Ser Ser Tyr Ser Ser Thr Gly Ser Asn Ala Asn Ile
               1780                1785                1790
   AAC AAC GCC AAC AAC ACC GCC CTG GGT CGC CTC CCT CGC CCC GCC GGC     5424
   Asn Asn Ala Asn Asn Thr Ala Leu Gly Arg Leu Pro Arg Pro Ala Gly
           1795                1800                1805
   TAC CCC AGC ACA GTC AGC ACT GTG GAG GGC CAC GGG CCC CCC TTG TCC     5472
   Tyr Pro Ser Thr Val Ser Thr Val Glu Gly His Gly Pro Pro Leu Ser
       1810                1815                1820
   CCT GCC ATC CGG GTG CAG GAG GTG GCG TGG AAG CTC AGC TCC AAC AGG     5520
   Pro Ala Ile Arg Val Gln Glu Val Ala Trp Lys Leu Ser Ser Asn Arg
   1825                1830                1835                1840
   TGC CAC TCC CGG GAG AGC CAG GCA GCC ATG GCG CGT CAG GAG GAG ACG     5568
   Cys His Ser Arg Glu Ser Gln Ala Ala Met Ala Arg Gln Glu Glu Thr
                   1845                1850                1855
   TCT CAG GAT GAG ACC TAT GAA GTG AAG ATG AAC CAT GAC ACG GAG GCC     5616
   Ser Gln Asp Glu Thr Tyr Glu Val Lys Met Asn His Asp Thr Glu Ala
               1860                1865                1870
   TGC AGT GAG CCC AGC CTG CTC TCC ACA GAG ATG CTC TCC TAC CAG GAT     5664
   Cys Ser Glu Pro Ser Leu Leu Ser Thr Glu Met Leu Ser Tyr Gln Asp
           1875                1880                1885
   GAC GAA AAT CGG CAA CTG ACG CTC CCA GAG GAG GAC AAG AGG GAC ATC      5712
   Asp Glu Asn Arg Gln Leu Thr Leu Pro Glu Glu Asp Lys Arg Asp Ile
       1890                1895                1900
   CGG CAA TCT CCG AAG AGG GGT TTC CTC CGC TCT TCC TCA CTA GGT CGA      5760
   Arg Gln Ser Pro Lys Arg Gly Phe Leu Arg Ser Ser Ser Leu Gly Arg
   1905                1910                1915                1920
   AGG GCC TCC TTC CAC CTG GAA TGT CTG AAG CGA CAG AAG GAC CGA GGG      5808
   Arg Ala Ser Phe His Leu Glu Cys Leu Lys Arg Gln Lys Asp Arg Gly
                   1925                1930                1935
   GGA GAC ATC TCT CAG AAG ACA GTC CTG CCC TTG CAT CTG GTT CAT CAT      5856
   Gly Asp Ile Ser Gln Lys Thr Val Leu Pro Leu His Leu Val His His
               1940                1945                1950
   CAG GCA TTG GCA GTG GCA GGC CTG AGC CCC CTC CTC CAG AGA AGC CAT      5904
   Gln Ala Leu Ala Val Ala Gly Leu Ser Pro Leu Leu Gln Arg Ser His
           1955                1960                1965

### (2) INFORMATION FOR SEQ ID NO:4:

(i) SEQUENCE CHARACTERISTICS:
   (A) LENGTH: 132 base pairs
   (B) TYPE: nucleic acid
   (C) STRANDEDNESS: double
   (D) TOPOLOGY: linear
(ii) MOLECULE TYPE: DNA (genomic)
(xi) SEQUENCE DESCRIPTION: SEQ ID NO:4:
   AGACCACGGC TTCCTCGAAT CTTGCGCGAA GCCGCCGGCC TCGGAGGAGG GATTAATCCA      60
   GACCCGCCGG GGGGTGTTTT CACATTTCTT CCTCTTCGTG GCTGCTCCTC CTATTAAAAC     120
   CATTTTTGGT CC                                                         132

### (2) INFORMATION FOR SEQ ID NO:5:

(i) SEQUENCE CHARACTERISTICS:
   (A) LENGTH: 89 base pairs
   (B) TYPE: nucleic acid
   (C) STRANDEDNESS: double
   (D) TOPOLOGY: linear
(ii) MOLECULE TYPE: DNA (genomic)
(xi) SEQUENCE DESCRIPTION: SEX ID NO:5:
   CGCTGAGGGC CTTCCGCGTG CTGCGCCCCC TGCGGCTGGT GTCCGGAGTC CCAAGTCTCC        60
   AGGTGGTCCT GAATTCCATC ATCAAGGCC                                          89

### (2) INFORMATION FOR SEQ ID NO:6:

(i) SEQUENCE CHARACTERISTICS:
   (A) LENGTH: 84 base pairs
   (B) TYPE: nucleic acid
   (C) STRANDEDNESS: double
   (D) TOPOLOGY: linear
(ii) MOLECULE TYPE: DNA (genomic)
(ix) FEATURE:
   (A) NAME/KEY: CDS
   (B) LOCATION: 1..84
   (D) OTHER INFORMATION: /note= "An alternative exon of alpha-1C."
(xi) SEQUENCE DESCRIPTION: SEQ ID NO:6:
   CAC TAT TTC TGT GAT GCA TGG AAT ACA TTT GAC GCC TTG ATT GTT GTG        48
   His Tyr Phe Cys Asp Ala Trp Asn Thr Phe Asp Ala Leu Ile Val Val
    1               5                  10                  15
   GGT AGC ATT GTT GAT ATA GCA ATC ACC GAG GTA AAC                        84
   Gly Ser Ile Val Asp Ile Ala Ile Thr Glu Val Asn
               20                  25

### (2) INFORMATION FOR SEQ ID NO:7:

(i) SEQUENCE CHARACTERISTICS:
   (A) LENGTH: 7362 base pairs
   (B) TYPE: nucleic acid
   (C) STRANDEDNESS: double
   (D) TOPOLOGY: linear
(ii) MOLECULE TYPE: DNA (genomic)
(ix) FEATURE:
   (A) NAME/KEY: CDS
   (B) LOCATION: 144..7163
(ix) FEATURE:
   (A) NAME/KEY: 5'UTR
   (B) LOCATION: 1..143
(ix) FEATURE:
   (A) NAME/KEY: 3'UTR
   (B) LOCATION: 7161..7362
(xi) SEQUENCE DESCRIPTION: SEQ ID NO:7:
   GCGGCGGCGG CTGCGGCGGT GGGGCCGGGC GAGGTCCGTG CGGTCCCGGC GGCTCCGTGG       60
   CTGCTCCGCT CTGAGCGCCT GCGCGCCCCG CGCCCTCCCT GCCGGGGCCG CTGGGCCGGG      120
   GATGCACGCG GGGCCCGGGA GCC ATG GTC CGC TTC GGG GAC GAG CTG GGC          170
                             Met Val Arg Phe Gly Asp Glu Leu Gly
                              1               5
   GGC CGC TAT GGA GGC CCC GGC GGC GGA GAG CGG GCC CGG GGC GGC GGG        218
   Gly Arg Tyr Gly Gly Pro Gly Gly Gly Glu Arg Ala Arg Gly Gly Gly
    10                  15                  20                  25
   GCC GGC GGG GCG GGG GGC CCG GGT CCC GGG GGG CTG CAG CCC GGC CAG        266
   Ala Gly Gly Ala Gly Gly Pro Gly Pro Gly Gly Leu Gln Pro Gly Gln
                    30                  35                  40
   CGG GTC CTC TAC AAG CAA TCG ATC GCG CAG CGC GCG CGG ACC ATG GCG        314
   Arg Val Leu Tyr Lys Gln Ser Ile Ala Gln Arg Ala Arg Thr Met Ala
                45                  50                  55
   CTG TAC AAC CCC ATC CCG GTC AAG CAG AAC TGC TTC ACC GTC AAC CGC        362
   Leu Tyr Asn Pro Ile Pro Val Lys Gln Asn Cys Phe Thr Val Asn Arg
            60                  65                  70
   TCG CTC TTC GTC TTC AGC GAG GAC AAC GTC GTC CGC AAA TAC GCG AAG        410
   Ser Leu Phe Val Phe Ser Glu Asp Asn Val Val Arg Lys Tyr Ala Lys
        75                  80                  85
   CGC ATC ACC GAG TGG CCT CCA TTC GAG AAT ATG ATC CTG GCC ACC ATC        458
   Arg Ile Thr Glu Trp Pro Pro Phe Glu Asn Met Ile Leu Ala Thr Ile
    90                  95                 100                 105
   ATC GCC AAC TGC ATC GTG CTG GCC CTG GAG CAG CAC CTC CCT GAT GGG      506
   Ile Ala Asn Cys Ile Val Leu Ala Leu Glu Gln His Leu Pro Asp Gly
                   110                 115                 120
   GAC AAA ACG CCC ATG TCC GAG CGG CTG GAC GAC ACG GAG CCC TAT TTC      554
   Asp Lys Thr Pro Met Ser Glu Arg Leu Asp Asp Thr Glu Pro Tyr Phe
               125                 130                 135
   ATC GGG ATC TTT TGC TTC GAG GCA GGG ATC AAA ATC ATC GCT CTG GGC      602
   Ile Gly Ile Phe Cys Phe Glu Ala Gly Ile Lys Ile Ile Ala Leu Gly
           140                 145                 150
   TTT GTC TTC CAC AAG GGC TCT TAC CTG CGG AAC GGC TGG AAC GTC ATG      650
   Phe Val Phe His Lys Gly Ser Tyr Leu Arg Asn Gly Trp Asn Val Met
       155                 160                 165
   GAC TTC GTG GTC GTC CTC ACA GGG ATC CTT GCC ACG GCT GGA ACT GAC      698
   Asp Phe Val Val Val Leu Thr Gly Ile Leu Ala Thr Ala Gly Thr Asp
   170                 175                 180                 185
   TTC GAC CTG CGA ACA CTG AGG GCT GTG CGT GTG CTG AGG CCC CTG AAG      746
   Phe Asp Leu Arg Thr Leu Arg Ala Val Arg Val Leu Arg Pro Leu Lys
                   190                 195                 200
   CTG GTG TCT GGG ATT CCA AGT TTG CAG GTG GTG CTC AAG TCC ATC ATG      794
   Leu Val Ser Gly Ile Pro Ser Leu Gln Val Val Leu Lys Ser Ile Met
               205                 210                 215
   AAG GCC ATG GTT CCA CTC CTG CAG ATT GGG CTG CTT CTC TTC TTT GCC      842
   Lys Ala Met Val Pro Leu Leu Gln Ile Gly Leu Leu Leu Phe Phe Ala
               220                 225                 230
   ATC CTC ATG TTT GCC ATC ATT GGC CTG GAG TTC TAC ATG GGC AAG TTC      890
   Ile Leu Met Phe Ala Ile Ile Gly Leu Glu Phe Tyr Met Gly Lys Phe
       235                 240                 245
   CAC AAG GCC TGT TTC CCC AAC AGC ACA GAT GCG GAG CCC GTG GGT GAC      938
   His Lys Ala Cys Phe Pro Asn Ser Thr Asp Ala Glu Pro Val Gly Asp
   250                 255                 260                 265
   TTC CCC TGT GGC AAG GAG GCC CCA GCC CGG CTG TGC GAG GGC GAC ACT      986
   Phe Pro Cys Gly Lys Glu Ala Pro Ala Arg Leu Cys Glu Gly Asp Thr
                   270                 275                 280
   GAG TGC CGG GAG TAC TGG CCA GGA CCC AAC TTT GGC ATC ACC AAC TTT     1034
   Glu Cys Arg Glu Tyr Trp Pro Gly Pro Asn Phe Gly Ile Thr Asn Phe
               285                 290                 295
   GAC AAT ATC CTG TTT GCC ATC TTG ACG GTG TTC CAG TGC ATC ACC ATG     1082
   Asp Asn Ile Leu Phe Ala Ile Leu Thr Val Phe Gln Cys Ile Thr Met
           300                 305                 310
   GAG GGC TGG ACT GAC ATC CTC TAT AAT ACA AAC GAT GCG GCC GGC AAC     1130
   Glu Gly Trp Thr Asp Ile Leu Tyr Asn Thr Asn Asp Ala Ala Gly Asn
       315                 320                 325
   ACC TGG AAC TGG CTC TAC TTC ATC CCT CTC ATC ATC ATC GGC TCC TTC     1178
   Thr Trp Asn Trp Leu Tyr Phe Ile Pro Leu Ile Ile Ile Gly Ser Phe
   330                 335                 340                 345
   TTC ATG CTC AAC CTG GTG CTG GGC GTG CTC TCG GGG GAG TTT GCC AAG     1226
   Phe Met Leu Asn Leu Val Leu Gly Val Leu Ser Gly Glu Phe Ala Lys
                   350                 355                 360
   GAG CGA GAG AGG GTG GAG AAC CGC CGC GCC TTC CTG AAG CTG CGC CGG     1274
   Glu Arg Glu Arg Val Glu Asn Arg Arg Ala Phe Leu Lys Leu Arg Arg
               365                 370                 375
   CAG CAG CAG ATC GAG CGA GAG CTC AAC GGG TAC CTG GAG TGG ATC TTC     1322
   Gln Gln Gln Ile Glu Arg Glu Leu Asn Gly Tyr Leu Glu Trp Ile Phe
            380                 385                 390
   AAG GCG GAG GAA GTC ATG CTG GCC GAG GAG GAC AGG AAT GCA GAG GAG     1370
   Lys Ala Glu Glu Val Met Leu Ala Glu Glu Asp Arg Asn Ala Glu Glu
       395                 400                 405
   AAG TCC CCT TTG GAC GTG CTG AAG AGA GCG GCC ACC AAG AAG AGC AGA     1418
   Lys Ser Pro Leu Asp Val Leu Lys Arg Ala Ala Thr Lys Lys Ser Arg
   410                 415                 420                 425
   AAT GAC CTG ATC CAC GCA GAG GAG GGA GAG GAC CGG TTT GCA GAT CTC     1466
   Asn Asp Leu Ile His Ala Glu Glu Gly Glu Asp Arg Phe Ala Asp Leu
                   430                 435                 440
   TGT GCT GTT GGA TCC CCC TTC GCC CGC GCC AGC CTC AAG AGC GGG AAG     1514
   Cys Ala Val Gly Ser Pro Phe Ala Arg Ala Ser Leu Lys Ser Gly Lys
               445                 450                 455
   ACA GAG AGC TCG TCA TAC TTC CGG AGG AAG GAG AAG ATG TTC CGG TTT     1562
   Thr Glu Ser Ser Ser Tyr Phe Arg Arg Lys Glu Lys Met Phe Arg Phe
           460                 465                 470
   TTT ATC CGG CGC ATG GTG AAG GCT CAG AGC TTC TAC TGG GTG GTG CTG     1610
   Phe Ile Arg Arg Met Val Lys Ala Gln Ser Phe Tyr Trp Val Val Leu
       475                 480                 485
   TGC GTG GTG GCC CTG AAC ACA CTG TGT GTG GCC ATG GTG CAT TAC AAC     1658
   Cys Val Val Ala Leu Asn Thr Leu Cys Val Ala Met Val His Tyr Asn
   490                 495                 500                 505
   CAG CCG CGG CGG CTT ACC ACG ACC CTG TAT TTT GCA GAG TTT GTT TTC     1706
   Gln Pro Arg Arg Leu Thr Thr Thr Leu Tyr Phe Ala Glu Phe Val Phe
                   510                 515                 520
   CTG GGT CTC TTC CTC ACA GAG ATG TCC CTG AAG ATG TAT GGC CTG GGG     1754
   Leu Gly Leu Phe Leu Thr Glu Met Ser Leu Lys Met Tyr Gly Leu Gly
               525                 530                 535
   CCC AGA AGC TAC TTC CGG TCC TCC TTC AAC TGC TTC GAC TTT GGG GTC     1802
   Pro Arg Ser Tyr Phe Arg Ser Ser Phe Asn Cys Phe Asp Phe Gly Val
           540                 545                 550
   ATC GTG GGG AGC GTC TTT GAA GTG GTC TGG GCG GCC ATC AAG CCG GGA     1850
   Ile Val Gly Ser Val Phe Glu Val Val Trp Ala Ala Ile Lys Pro Gly
       555                 560                 565
   AGC TCC TTT GGG ATC AGT GTG CTG CGG GCC CTC CGC CTG CTG AGG ATC     1898
   Ser Ser Phe Gly Ile Ser Val Leu Arg Ala Leu Arg Leu Leu Arg Ile
   570                 575                 580                 585
   TTC AAA GTC ACG AAG TAC TGG AGC TCC CTG CGG AAC CTG GTG GTG TCC     1946
   Phe Lys Val Thr Lys Tyr Trp Ser Ser Leu Arg Asn Leu Val Val Ser
                   590                 595                 600
   CTG CTG AAC TCC ATG AAG TCC ATC ATC AGC CTG CTC TTC TTG CTC TTC     1994
   Leu Leu Asn Ser Met Lys Ser Ile Ile Ser Leu Leu Phe Leu Leu Phe
               605                 610                 615
   CTG TTC ATT GTG GTC TTC GCC CTG CTG GGG ATG CAG CTG TTT GGG GGA     2042
   Leu Phe Ile Val Val Phe Ala Leu Leu Gly Met Gln Leu Phe Gly Gly
           620                 625                 630
   CAG TTC AAC TTC CAG GAT GAG ACT CCC ACA ACC AAC TTC GAC ACC TTC     2090
   Gln Phe Asn Phe Gln Asp Glu Thr Pro Thr Thr Asn Phe Asp Thr Phe
       635                 640                 645
   CCT GCC GCC ATC CTC ACT GTC TTC CAG ATC CTG ACG GGA GAG GAC TGG     2138
   Pro Ala Ala Ile Leu Thr Val Phe Gln Ile Leu Thr Gly Glu Asp Trp
   650                 655                 660                 665
   AAT GCA GTG ATG TAT CAC GGG ATC GAA TCG CAA GGC GGC GTC AGC AAA      2186
   Asn Ala Val Met Tyr His Gly Ile Glu Ser Gln Gly Gly Val Ser Lys
                   670                 675                 680
   GGC ATG TTC TCG TCC TTT TAC TTC ATT GTC CTG ACA CTG TTC GGA AAC      2234
   Gly Met Phe Ser Ser Phe Tyr Phe Ile Val Leu Thr Leu Phe Gly Asn
               685                 690                 695
   TAC ACT CTG CTG AAT GTC TTT CTG GCC ATC GCT GTG GAC AAC CTG GCC      2282
   Tyr Thr Leu Leu Asn Val Phe Leu Ala Ile Ala Val Asp Asn Leu Ala
           700                 705                 710
   AAC GCC CAA GAG CTG ACC AAG GAT GAA GAG GAG ATG GAA GAA GCA GCC      2330
   Asn Ala Gln Glu Leu Thr Lys Asp Glu Glu Glu Met Glu Glu Ala Ala
       715                 720                 725
   AAT CAG AAG CTT GCT CTG CAA AAG GCC AAA GAA GTG GCT GAA GTC AGC      2378
   Asn Gln Lys Leu Ala Leu Gln Lys Ala Lys Glu Val Ala Glu Val Ser
   730                 735                 740                 745
   CCC ATG TCT GCC GCG AAC ATC TCC ATC GCC GCC AGG CAG CAG AAC TCG      2426
   Pro Met Ser Ala Ala Asn Ile Ser Ile Ala Ala Arg Gln Gln Asn Ser
                   750                 755                 760
   GCC AAG GCG CGC TCG GTG TGG GAG CAG CGG GCC AGC CAG CTA CGG CTG      2474
   Ala Lys Ala Arg Ser Val Trp Glu Gln Arg Ala Ser Gln Leu Arg Leu
               765                 770                 775
   CAG AAC CTG CGG GCC AGC TGC GAG GCG CTG TAC AGC GAG ATG GAC CCC      2522
   Gln Asn Leu Arg Ala Ser Cys Glu Ala Leu Tyr Ser Glu Met Asp Pro
           780                 785                 790
   GAG GAG CGG CTG CGC TTC GCC ACT ACG CGC CAC CTG CGG CCC GAC ATG      2570
   Glu Glu Arg Leu Arg Phe Ala Thr Thr Arg His Leu Arg Pro Asp Met
       795                 800                 805
   AAG ACG CAC CTG GAC CGG CCG CTG GTG GTG GAG CTG GGC CGC GAC GGC      2618
   Lys Thr His Leu Asp Arg Pro Leu Val Val Glu Leu Gly Arg Asp Gly
   810                 815                 820                 825
   GCG CGG GGG CCC GTG GGA GGC AAA GCC CGA CCT GAG GCT GCG GAG GCC      2666
   Ala Arg Gly Pro Val Gly Gly Lys Ala Arg Pro Glu Ala Ala Glu Ala
                   830                 835                 840
   CCC GAG GGC GTC GAC CCT CCG CGC AGG CAC CAC CGG CAC CGC GAC AAG      2714
   Pro Glu Gly Val Asp Pro Pro Arg Arg His His Arg His Arg Asp Lys
               845                 850                 855
   GAC AAG ACC CCC GCG GCG GGG GAC CAG GAC CGA GCA GAG GCC CCG AAG      2762
   Asp Lys Thr Pro Ala Ala Gly Asp Gln Asp Arg Ala Glu Ala Pro Lys
           860                 865                 870
   GCG GAG AGC GGG GAG CCC GGT GCC CGG GAG GAG CGG CCG CGG CCG CAC      2810
   Ala Glu Ser Gly Glu Pro Gly Ala Arg Glu Glu Arg Pro Arg Pro His
       875                 880                 885
   CGC AGC CAC AGC AAG GAG GCC GCG GGG CCC CCG GAG GCG CGG AGC GAG      2858
   Arg Ser His Ser Lys Glu Ala Ala Gly Pro Pro Glu Ala Arg Ser Glu
   890                 895                 900                 905
   CGC GGC CGA GGC CCA GGC CCC GAG GGC GGC CGG CGG CAC CAC CGG CGC      2906
   Arg Gly Arg Gly Pro Gly Pro Glu Gly Gly Arg Arg His His Arg Arg
                   910                 915                 920
   GGC TCC CCG GAG GAG GCG GCC GAG CGG GAG CCC CGA CGC CAC CGC GCG      2954
   Gly Ser Pro Glu Glu Ala Ala Glu Arg Glu Pro Arg Arg His Arg Ala
               925                 930                 935
   CAC CGG CAC CAG GAT CCG AGC AAG GAG TGC GCC GGC GCC AAG GGC GAG      3002
   His Arg His Gln Asp Pro Ser Lys Glu Cys Ala Gly Ala Lys Gly Glu
           940                 945                 950
   CGG CGC GCG CGG CAC CGC GGC GGC CCC CGA GCG GGG CCC CGG GAG GCG        3050
   Arg Arg Ala Arg His Arg Gly Gly Pro Arg Ala Gly Pro Arg Glu Ala
       955                 960                 965
   GAG AGC GGG GAG GAG CCG GCG CGG CGG CAC CGG GCC CGG CAC AAG GCG        3098
   Glu Ser Gly Glu Glu Pro Ala Arg Arg His Arg Ala Arg His Lys Ala
   970                 975                 980                 985
   CAG CCT GCT CAC GAG GCT GTG GAG AAG GAG ACC ACG GAG AAG GAG GCC        3146
   Gln Pro Ala His Glu Ala Val Glu Lys Glu Thr Thr Glu Lys Glu Ala
                   990                 995                 1000
   ACG GAG AAG GAG GCT GAG ATA GTG GAA GCC GAC AAG GAA AAG GAG CTC        3194
   Thr Glu Lys Glu Ala Glu Ile Val Glu Ala Asp Lys Glu Lys Glu Leu
               1005                1010                1015
   CGG AAC CAC CAG CCC CGG GAG CCA CAC TGT GAC CTG GAG ACC AGT GGG        3242
   Arg Asn His Gln Pro Arg Glu Pro His Cys Asp Leu Glu Thr Ser Gly
           1020                1025                1030
   ACT GTG ACT GTG GGT CCC ATG CAC ACA CTG CCC AGC ACC TGT CTC CAG        3290
   Thr Val Thr Val Gly Pro Met His Thr Leu Pro Ser Thr Cys Leu Gln
       1035                1040                1045
   AAG GTG GAG GAA CAG CCA GAG GAT GCA GAC AAT CAG CGG AAC GTC ACT        3338
   Lys Val Glu Glu Gln Pro Glu Asp Ala Asp Asn Gln Arg Asn Val Thr
   1050                1055                1060                1065
   CGC ATG GGC AGT CAG CCC CCA GAC CCG AAC ACT ATT GTA CAT ATC CCA        3386
   Arg Met Gly Ser Gln Pro Pro Asp Pro Asn Thr Ile Val His Ile Pro
                   1070                1075                1080
   GTG ATG CTG ACG GGC CCT CTT GGG GAA GCC ACG GTC GTT CCC AGT GGT        3434
   Val Met Leu Thr Gly Pro Leu Gly Glu Ala Thr Val Val Pro Ser Gly
               1085                1090                1095
   AAC GTG GAC CTG GAA AGC CAA GCA GAG GGG AAG AAG GAG GTG GAA GCG        3482
   Asn Val Asp Leu Glu Ser Gln Ala Glu Gly Lys Lys Glu Val Glu Ala
           1100                1105                1110
   GAT GAC GTG ATG AGG AGC GGC CCC CGG CCT ATC GTC CCA TAC AGC TCC        3530
   Asp Asp Val Met Arg Ser Gly Pro Arg Pro Ile Val Pro Tyr Ser Ser
       1115                1120                1125
   ATG TTC TGT TTA AGC CCC ACC AAC CTG CTC CGC CGC TTC TGC CAC TAC        3578
   Met Phe Cys Leu Ser Pro Thr Asn Leu Leu Arg Arg Phe Cys His Tyr
   1130                1135                1140                1145
   ATC GTG ACC ATG AGG TAC TTC GAG GTG GTC ATT CTC GTG GTC ATC GCC        3626
   Ile Val Thr Met Arg Tyr Phe Glu Val Val Ile Leu Val Val Ile Ala
                   1150                1155                1160
   TTG AGC AGC ATC GCC CTG GCT GCT GAG GAC CCA GTG CGC ACA GAC TCG        3674
   Leu Ser Ser Ile Ala Leu Ala Ala Glu Asp Pro Val Arg Thr Asp Ser
               1165                1170                1175
   CCC AGG AAC AAC GCT CTG AAA TAC CTG GAT TAC ATT TTC ACT GGT GTC        3722
   Pro Arg Asn Asn Ala Leu Lys Tyr Leu Asp Tyr Ile Phe Thr Gly Val
           1180                1185                1190
   TTT ACC TTT GAG ATG GTG ATA AAG ATG ATC GAC TTG GGA CTG CTG CTT        3770
   Phe Thr Phe Glu Met Val Ile Lys Met Ile Asp Leu Gly Leu Leu Leu
       1195                1200                1205
   CAC CCT GGA GCC TAT TTC CGG GAC TTG TGG AAC ATT CTG GAC TTC ATT        3818
   His Pro Gly Ala Tyr Phe Arg Asp Leu Trp Asn Ile Leu Asp Phe Ile
   1210                1215                1220                1225
   GTG GTC AGT GGC GCC CTG GTG GCG TTT GCT TTC TCA GGA TCC AAA GGG      3866
   Val Val Ser Gly Ala Leu Val Ala Phe Ala Phe Ser Gly Ser Lys Gly
                   1230                1235                1240
   AAA GAC ATC AAT ACC ATC AAG TCT CTG AGA GTC CTT CGT GTC CTG CGG      3914
   Lys Asp Ile Asn Thr Ile Lys Ser Leu Arg Val Leu Arg Val Leu Arg
               1245                1250                1255
   CCC CTC AAG ACC ATC AAA CGG CTG CCC AAG CTC AAG GCT GTG TTT GAC      3962
   Pro Leu Lys Thr Ile Lys Arg Leu Pro Lys Leu Lys Ala Val Phe Asp
           1260                1265                1270
   TGT GTG GTG AAC TCC CTG AAG AAT GTC CTC AAC ATC TTG ATT GTC TAC      4010
   Cys Val Val Asn Ser Leu Lys Asn Val Leu Asn Ile Leu Ile Val Tyr
       1275                1280                1285
   ATG CTC TTC ATG TTC ATA TTT GCC GTC ATT GCG GTG CAG CTC TTC AAA      4058
   Met Leu Phe Met Phe Ile Phe Ala Val Ile Ala Val Gln Leu Phe Lys
   1290                1295                1300                1305
   GGG AAG TTT TTC TAC TGC ACA GAT GAA TCC AAG GAG CTG GAG AGG GAC      4106
   Gly Lys Phe Phe Tyr Cys Thr Asp Glu Ser Lys Glu Leu Glu Arg Asp
                   1310                1315                1320
   TGC AGG GGT CAG TAT TTG GAT TAT GAG AAG GAG GAA GTG GAA GCT CAG      4154
   Cys Arg Gly Gln Tyr Leu Asp Tyr Glu Lys Glu Glu Val Glu Ala Gln
               1325                1330                1335
   CCC AGG CAG TGG AAG AAA TAC GAC TTT CAC TAC GAC AAT GTG CTC TGG      4202
   Pro Arg Gln Trp Lys Lys Tyr Asp Phe His Tyr Asp Asn Val Leu Trp
           1340                1345                1350
   GCT CTG CTG ACG CTG TTC ACA GTG TCC ACG GGA GAA GGC TGG CCC ATG      4250
   Ala Leu Leu Thr Leu Phe Thr Val Ser Thr Gly Glu Gly Trp Pro Met
       1355                1360                1365
   GTG CTG AAA CAC TCC GTG GAT GCC ACC TAT GAG GAG CAG GGT CCA AGC      4298
   Val Leu Lys His Ser Val Asp Ala Thr Tyr Glu Glu Gln Gly Pro Ser
   1370                1375                1380                1385
   CCT GGG TAC CGC ATG GAG CTG TCC ATC TTC TAC GTG GTC TAC TTT GTG      4346
   Pro Gly Tyr Arg Met Glu Leu Ser Ile Phe Tyr Val Val Tyr Phe Val
                   1390                1395                1400
   GTC TTT CCC TTC TTC TTC GTC AAC ATC TTT GTG GCT TTG ATC ATC ATC      4394
   Val Phe Pro Phe Phe Phe Val Asn Ile Phe Val Ala Leu Ile Ile Ile
               1405                1410                1415
   ACC TTC CAG GAG CAG GGG GAC AAG GTG ATG TCT GAA TGC AGC CTG GAG      4442
   Thr Phe Gln Glu Gln Gly Asp Lys Val Met Ser Glu Cys Ser Leu Glu
           1420                1425                1430
   AAG AAC GAG AGG GCT TGC ATT GAC TTC GCC ATC AGC GCC AAA CCC CTG      4490
   Lys Asn Glu Arg Ala Cys Ile Asp Phe Ala Ile Ser Ala Lys Pro Leu
       1435                1440                1445
   ACA CGG TAC ATG CCC CAA AAC CGG CAG TCG TTC CAG TAT AAG ACG TGG      4538
   Thr Arg Tyr Met Pro Gln Asn Arg Gln Ser Phe Gln Tyr Lys Thr Trp
   1450                1455                1460                1465
   ACA TTT GTG GTC TCC CCG CCC TTT GAA TAC TTC ATC ATG GCC ATG ATA      4586
   Thr Phe Val Val Ser Pro Pro Phe Glu Tyr Phe Ile Met Ala Met Ile
                   1470                1475                1480
   GCC CTC AAC ACT GTG GTG CTG ATG ATG AAG TTC TAT GAT GCA CCC TAT      4634
   Ala Leu Asn Thr Val Val Leu Met Met Lys Phe Tyr Asp Ala Pro Tyr
               1485                1490                1495
   GAG TAC GAG CTG ATG CTG AAA TGC CTG AAC ATC GTG TTC ACA TCC ATG      4682
   Glu Tyr Glu Leu Met Leu Lys Cys Leu Asn Ile Val Phe Thr Ser Met
           1500                    1505                    1510
   TTC TCC ATG GAA TGC GTG CTG AAG ATC ATC GCC TTT GGG GTG CTG AAC        4730
   Phe Ser Met Glu Cys Val Leu Lys Ile Ile Ala Phe Gly Val Leu Asn
       1515                    1520                    1525
   TAT TTC AGA GAT GCC TGG AAT GTC TTT GAC TTT GTC ACT GTG TTG GGA        4778
   Tyr Phe Arg Asp Ala Trp Asn Val Phe Asp Phe Val Thr Val Leu Gly
   1530                    1535                    1540                    1545
   AGT ATT ACT GAT ATT TTA GTA ACA GAG ATT GCG GAA ACG AAC AAT TTC        4826
   Ser Ile Thr Asp Ile Leu Val Thr Glu Ile Ala Glu Thr Asn Asn Phe
                   1550                    1555                    1560
   ATC AAC CTC AGC TTC CTC CGC CTC TTT CGA GCT GCG CGG CTG ATC AAG        4874
   Ile Asn Leu Ser Phe Leu Arg Leu Phe Arg Ala Ala Arg Leu Ile Lys
               1565                    1570                    1575
   CTG CTC CGC CAG GGC TAC ACC ATC CGC ATC CTG CTG TGG ACC TTT GTC        4922
   Leu Leu Arg Gln Gly Tyr Thr Ile Arg Ile Leu Leu Trp Thr Phe Val
           1580                    1585                    1590
   CAG TCC TTC AAG GCC CTG CCC TAC GTG TGT CTG CTC ATT GCC ATG CTG        4970
   Gln Ser Phe Lys Ala Leu Pro Tyr Val Cys Leu Leu Ile Ala Met Leu
       1595                    1600                    1605
   TTC TTC ATC TAC GCC ATC ATC GGC ATG CAG GTG TTT GGG AAT ATT GCC        5018
   Phe Phe Ile Tyr Ala Ile Ile Gly Met Gln Val Phe Gly Asn Ile Ala
   1610                    1615                    1620                    1625
   CTG GAT GAT GAC ACC AGC ATC AAC CGC CAC AAC AAC TTC CGG ACG TTT        5066
   Leu Asp Asp Asp Thr Ser Ile Asn Arg His Asn Asn Phe Arg Thr Phe
                   1630                    1635                    1640
   TTG CAA GCC CTG ATG CTG CTG TTC AGG AGC GCC ACG GGG GAG GCC TGG        5114
   Leu Gln Ala Leu Met Leu Leu Phe Arg Ser Ala Thr Gly Glu Ala Trp
               1645                    1650                    1655
   CAC GAG ATC ATG CTG TCC TGC CTG AGC AAC CAG GCC TGT GAT GAG CAG        5162
   His Glu Ile Met Leu Ser Cys Leu Ser Asn Gln Ala Cys Asp Glu Gln
           1660                    1665                    1670
   GCC AAT GCC ACC GAG TGT GGA AGT GAC TTT GCC TAC TTC TAC TTC GTC        5210
   Ala Asn Ala Thr Glu Cys Gly Ser Asp Phe Ala Tyr Phe Tyr Phe Val
       1675                    1680                    1685
   TCC TTC ATC TTC CTG TGC TCC TTT CTG ATG TTG AAC CTC TTT GTG GCT        5258
   Ser Phe Ile Phe Leu Cys Ser Phe Leu Met Leu Asn Leu Phe Val Ala
   1690                    1695                    1700                    1705
   GTG ATC ATG GAC AAT TTT GAG TAC CTC ACG CGG GAC TCT TCC ATC CTA        5306
   Val Ile Met Asp Asn Phe Glu Tyr Leu Thr Arg Asp Ser Ser Ile Leu
                   1710                    1715                    1720
   GGT CCT CAC CAC TTG GAT GAG TTC ATC CGG GTC TGG GCT GAA TAC GAC        5354
   Gly Pro His His Leu Asp Glu Phe Ile Arg Val Trp Ala Glu Tyr Asp
               1725                    1730                    1735
   CCG GCT GCG TGT GGG CGC ATC AGT TAC AAT GAC ATG TTT GAG ATG CTG        5402
   Pro Ala Ala Cys Gly Arg Ile Ser Tyr Asn Asp Met Phe Glu Met Leu
           1740                    1745                    1750
   AAA CAC ATG TCC CCG CCT CTG GGG CTG GGG AAG AAA TGC CCT GCT CGA        5450
   Lys His Met Ser Pro Pro Leu Gly Leu Gly Lys Lys Cys Pro Ala Arg
       1755                    1760                    1765
   GTT GCT TAC AAG CGC CTG GTT CGC ATG AAC ATG CCC ATC TCC AAC GAG        5498
   Val Ala Tyr Lys Arg Leu Val Arg Met Asn Met Pro Ile Ser Asn Glu
   1770                    1775                    1780                    1785
   GAC ATG ACT GTT CAC TTC ACG TCC ACG CTG ATG GCC CTC ATC CGG ACG      5546
   Asp Met Thr Val His Phe Thr Ser Thr Leu Met Ala Leu Ile Arg Thr
                   1790                1795                1800
   GCA CTG GAG ATC AAG CTG GCC CCA GCT GGG ACA AAG CAG CAT CAG TGT      5594
   Ala Leu Glu Ile Lys Leu Ala Pro Ala Gly Thr Lys Gln His Gln Cys
               1805                1810                1815
   GAC GCG GAG TTG AGG AAG GAG ATT TCC GTT GTG TGG GCC AAT CTG CCC      5642
   Asp Ala Glu Leu Arg Lys Glu Ile Ser Val Val Trp Ala Asn Leu Pro
           1820                1825                1830
   CAG AAG ACT TTG GAC TTG CTG GTA CCA CCC CAT AAG CCT GAT GAG ATG      5690
   Gln Lys Thr Leu Asp Leu Leu Val Pro Pro His Lys Pro Asp Glu Met
       1835                1840                1845
   ACA GTG GGG AAG GTT TAT GCA GCT CTG ATG ATA TTT GAC TTC TAC AAG      5738
   Thr Val Gly Lys Val Tyr Ala Ala Leu Met Ile Phe Asp Phe Tyr Lys
   1850                1855                1860                1865
   CAG AAC AAA ACC ACC AGA GAC CAG ATG CAG CAG GCT CCT GGA GGC CTC      5786
   Gln Asn Lys Thr Thr Arg Asp Gln Met Gln Gln Ala Pro Gly Gly Leu
                   1870                1875                1880
   TCC CAG ATG GGT CCT GTG TCC CTG TTC CAC CCT CTG AAG GCC ACC CTG      5834
   Ser Gln Met Gly Pro Val Ser Leu Phe His Pro Leu Lys Ala Thr Leu
               1885                1890                1895
   GAG CAG ACA CAG CCG GCT GTG CTC CGA GGA GCC CGG GTT TTC CTT CGA      5882
   Glu Gln Thr Gln Pro Ala Val Leu Arg Gly Ala Arg Val Phe Leu Arg
           1900                1905                1910
   CAG AAG AGT TCC ACC TCC CTC AGC AAT GGC GGG GCC ATA CAA AAC CAA      5930
   Gln Lys Ser Ser Thr Ser Leu Ser Asn Gly Gly Ala Ile Gln Asn Gln
       1915                1920                1925
   GAG AGT GGC ATC AAA GAG TCT GTC TCC TGG GGC ACT CAA AGG ACC CAG      5978
   Glu Ser Gly Ile Lys Glu Ser Val Ser Trp Gly Thr Gln Arg Thr Gln
   1930                1935                1940                1945
   GAT GCA CCC CAT GAG GCC AGG CCA CCC CTG GAG CGT GGC CAC TCC ACA      6026
   Asp Ala Pro His Glu Ala Arg Pro Pro Leu Glu Arg Gly His Ser Thr
                   1950                1955                1960
   GAG ATC CCT GTG GGG CGG TCA GGA GCA CTG GCT GTG GAC GTT CAG ATG      6074
   Glu Ile Pro Val Gly Arg Ser Gly Ala Leu Ala Val Asp Val Gln Met
               1965                1970                1975
   CAG AGC ATA ACC CGG AGG GGC CCT GAT GGG GAG CCC CAG CCT GGG CTG      6122
   Gln Ser Ile Thr Arg Arg Gly Pro Asp Gly Glu Pro Gln Pro Gly Leu
           1980                1985                1990
   GAG AGC CAG GGT CGA GCG GCC TCC ATG CCC CGC CTT GCG GCC GAG ACT      6170
   Glu Ser Gln Gly Arg Ala Ala Ser Met Pro Arg Leu Ala Ala Glu Thr
       1995                2000                2005
   CAG CCC GTC ACA GAT GCC AGC CCC ATG AAG CGC TCC ATC TCC ACG CTG      6218
   Gln Pro Val Thr Asp Ala Ser Pro Met Lys Arg Ser Ile Ser Thr Leu
   2010                2015                2020                2025
   GCC CAG CGG CCC CGT GGG ACT CAT CTT TGC AGC ACC ACC CCG GAC CGC      6266
   Ala Gln Arg Pro Arg Gly Thr His Leu Cys Ser Thr Thr Pro Asp Arg
                   2030                2035                2040
   CCA CCC CCT AGC CAG GCG TCG TCG CAC CAC CAC CAC CAC CGC TGC CAC      6314
   Pro Pro Pro Ser Gln Ala Ser Ser His His His His His Arg Cys His
               2045                2050                2055
   CGC CGC AGG GAC AGG AAG CAG AGG TCC CTG GAG AAG GGG CCC AGC CTG      6362
   Arg Arg Arg Asp Arg Lys Gln Arg Ser Leu Glu Lys Gly Pro Ser Leu
           2060                2065                2070
   TCT GCC GAT ATG GAT GGC GCA CCA AGC AGT GCT GTG GGG CCG GGG CTG        6410
   Ser Ala Asp Met Asp Gly Ala Pro Ser Ser Ala Val Gly Pro Gly Leu
       2075                2080                2085
   CCC CCG GGA GAG GGG CCT ACA GGC TGC CGG CGG GAA CGA GAG CGC CGG        6458
   Pro Pro Gly Glu Gly Pro Thr Gly Cys Arg Arg Glu Arg Glu Arg Arg
   2090                2095                2100                2105
   CAG GAG CGG GGC CGG TCC CAG GAG CGG AGG CAG CCC TCA TCC TCC TCC        6506
   Gln Glu Arg Gly Arg Ser Gln Glu Arg Arg Gln Pro Ser Ser Ser Ser
                   2110                2115                2120
   TCG GAG AAG CAG CGC TTC TAC TCC TGC GAC CGC TTT GGG GGC CGT GAG        6554
   Ser Glu Lys Gln Arg Phe Tyr Ser Cys Asp Arg Phe Gly Gly Arg Glu
               2125                2130                2135
   CCC CCG AAG CCC AAG CCC TCC CTC AGC AGC CAC CCA ACG TCG CCA ACA        6602
   Pro Pro Lys Pro Lys Pro Ser Leu Ser Ser His Pro Thr Ser Pro Thr
           2140                2145                2150
   GCT GGC CAG GAG CCG GGA CCC CAC CCA CAG GGC AGT GGT TCC GTG AAT        6650
   Ala Gly Gln Glu Pro Gly Pro His Pro Gln Gly Ser Gly Ser Val Asn
       2155                2160                2165
   GGG AGC CCC TTG CTG TCA ACA TCT GGT GCT AGC ACC CCC GGC CGC GGT        6698
   Gly Ser Pro Leu Leu Ser Thr Ser Gly Ala Ser Thr Pro Gly Arg Gly
   2170                2175                2180                2185
   GGG CGG AGG CAG CTC CCC CAG ACG CCC CTG ACT CCC CGC CCC AGC ATC        6746
   Gly Arg Arg Gln Leu Pro Gln Thr Pro Leu Thr Pro Arg Pro Ser Ile
                   2190                2195                2200
   ACC TAC AAG ACG GCC AAC TCC TCA CCC ATC CAC TTC GCC GGG GCT CAG        6794
   Thr Tyr Lys Thr Ala Asn Ser Ser Pro Ile His Phe Ala Gly Ala Gln
               2205                2210                2215
   ACC AGC CTC CCT GCC TTC TCC CCA GGC CGG CTC AGC CGT GGG CTT TCC        6842
   Thr Ser Leu Pro Ala Phe Ser Pro Gly Arg Leu Ser Arg Gly Leu Ser
           2220                2225                2230
   GAA CAC AAC GCC CTG CTG CAG AGA GAC CCC CTC AGC CAG CCC CTG GCC        6890
   Glu His Asn Ala Leu Leu Gln Arg Asp Pro Leu Ser Gln Pro Leu Ala
       2235                2240                2245
   CCT GGC TCT CGA ATT GGC TCT GAC CCT TAC CTG GGG CAG CGT CTG GAC        6938
   Pro Gly Ser Arg Ile Gly Ser Asp Pro Tyr Leu Gly Gln Arg Leu Asp
   2250                2255                2260                2265
   AGT GAG GCC TCT GTC CAC GCC CTG CCT GAG GAC ACG CTC ACT TTC GAG        6986
   Ser Glu Ala Ser Val His Ala Leu Pro Glu Asp Thr Leu Thr Phe Glu
                   2270                2275                2280
   GAG GCT GTG GCC ACC AAC TCG GGC CGC TCC TCC AGG ACT TCC TAC GTG        7034
   Glu Ala Val Ala Thr Asn Ser Gly Arg Ser Ser Arg Thr Ser Tyr Val
               2285                2290                2295
   TCC TCC CTG ACC TCC CAG TCT CAC CCT CTC CGC CGC GTG CCC AAC GGT        7082
   Ser Ser Leu Thr Ser Gln Ser His Pro Leu Arg Arg Val Pro Asn Gly
           2300                2305                2310
   TAC CAC TGC ACC CTG GGA CTC AGC TCG GGT GGC CGA GCA CGG CAC AGC        7130
   Tyr His Cys Thr Leu Gly Leu Ser Ser Gly Gly Arg Ala Arg His Ser
       2315                2320                2325
   TAC CAC CAC CCT GAC CAA GAC CAC TGG TGC TAGCTGCACC GTGACCGCTC          7180
   Tyr His His Pro Asp Gln Asp His Trp Cys
   2330                2335
   AGACGCCTGC ATGCAGCAGG CGTGTGTTCC AGTGGATGAG TTTTATCATC CACACGGGGC 7240
   AGTCGGCCCT CGGGGGAGGC CTTGCCCACC TTGGTGAGGC TCCTGTGGCC CCTCCCTCCC 7300
   CCTCCTCCCC TCTTTTACTC TAGACGACGA ATAAAGCCCT GTTGCTTGAG TGTACGTACC 7360
   GC 7362

### (2) INFORMATION FOR SEQ ID NO:8:

(i) SEQUENCE CHARACTERISTICS:
   (A) LENGTH: 7175 base pairs
   (B) TYPE: nucleic acid
   (C) STRANDEDNESS: double
   (D) TOPOLOGY: linear
(ii) MOLECULE TYPE: DNA (genomic)
(ix) FEATURE:
   (A) NAME/KEY: CDS
   (B) LOCATION: 144..6857
(ix) FEATURE:
   (A) NAME/KEY: 5'UTR
   (B) LOCATION: 1..143
(ix) FEATURE:
   (A) NAME/KEY: 3'UTR
   (B) LOCATION: 6855..7175
(xi) SEQUENCE DESCRIPTION: SEQ ID NO:8:
   GCGGCGGCGG CTGCGGCGGT GGGGCCGGGC GAGGTCCGTG CGGTCCCGGC GGCTCCGTGG      60
   CTGCTCCGCT CTGAGCGCCT GCGCGCCCCG CGCCCTCCCT GCCGGGGCCG CTGGGCCGGG     120
   GATGCACGCG GGGCCCGGGA GCC ATG GTC CGC TTC GGG GAC GAG CTG GGC        170
                             Met Val Arg Phe Gly Asp Glu Leu Gly
                               1               5
   GGC CGC TAT GGA GGC CCC GGC GGC GGA GAG CGG GCC CGG GGC GGC GGG      218
   Gly Arg Tyr Gly Gly Pro Gly Gly Gly Glu Arg Ala Arg Gly Gly Gly
    10                  15                  20                  25
   GCC GGC GGG GCG GGG GGC CCG GGT CCC GGG GGG CTG CAG CCC GGC CAG      266
   Ala Gly Gly Ala Gly Gly Pro Gly Pro Gly Gly Leu Gln Pro Gly Gln
                        30                  35                  40
   CGG GTC CTC TAC AAG CAA TCG ATC GCG CAG CGC GCG CGG ACC ATG GCG      314
   Arg Val Leu Tyr Lys Gln Ser Ile Ala Gln Arg Ala Arg Thr Met Ala
                    45                  50                  55
   CTG TAC AAC CCC ATC CCG GTC AAG CAG AAC TGC TTC ACC GTC AAC CGC      362
   Leu Tyr Asn Pro Ile Pro Val Lys Gln Asn Cys Phe Thr Val Asn Arg
            60                  65                  70
   TCG CTC TTC GTC TTC AGC GAG GAC AAC GTC GTC CGC AAA TAC GCG AAG      410
   Ser Leu Phe Val Phe Ser Glu Asp Asn Val Val Arg Lys Tyr Ala Lys
        75                  80                  85
   CGC ATC ACC GAG TGG CCT CCA TTC GAG AAT ATG ATC CTG GCC ACC ATC      458
   Arg Ile Thr Glu Trp Pro Pro Phe Glu Asn Met Ile Leu Ala Thr Ile
    90                  95                 100                 105
   ATC GCC AAC TGC ATC GTG CTG GCC CTG GAG CAG CAC CTC CCT GAT GGG      506
   Ile Ala Asn Cys Ile Val Leu Ala Leu Glu Gln His Leu Pro Asp Gly
                       110                 115                 120
   GAC AAA ACG CCC ATG TCC GAG CGG CTG GAC GAC ACG GAG CCC TAT TTC      554
   Asp Lys Thr Pro Met Ser Glu Arg Leu Asp Asp Thr Glu Pro Tyr Phe
                   125                     130                     135
   ATC GGG ATC TTT TGC TTC GAG GCA GGG ATC AAA ATC ATC GCT CTG GGC          602
   Ile Gly Ile Phe Cys Phe Glu Ala Gly Ile Lys Ile Ile Ala Leu Gly
               140                     145                     150
   TTT GTC TTC CAC AAG GGC TCT TAC CTG CGG AAC GGC TGG AAC GTC ATG          650
   Phe Val Phe His Lys Gly Ser Tyr Leu Arg Asn Gly Trp Asn Val Met
           155                     160                     165
   GAC TTC GTG GTC GTC CTC ACA GGG ATC CTT GCC ACG GCT GGA ACT GAC          698
   Asp Phe Val Val Val Leu Thr Gly Ile Leu Ala Thr Ala Gly Thr Asp
   170                 175                     180                     185
   TTC GAC CTG CGA ACA CTG AGG GCT GTG CGT GTG CTG AGG CCC CTG AAG          746
   Phe Asp Leu Arg Thr Leu Arg Ala Val Arg Val Leu Arg Pro Leu Lys
                   190                     195                     200
   CTG GTG TCT GGG ATT CCA AGT TTG CAG GTG GTG CTC AAG TCC ATC ATG          794
   Leu Val Ser Gly Ile Pro Ser Leu Gln Val Val Leu Lys Ser Ile Met
               205                     210                     215
   AAG GCC ATG GTT CCA CTC CTG CAG ATT GGG CTG CTT CTC TTC TTT GCC          842
   Lys Ala Met Val Pro Leu Leu Gln Ile Gly Leu Leu Leu Phe Phe Ala
           220                     225                     230
   ATC CTC ATG TTT GCC ATC ATT GGC CTG GAG TTC TAC ATG GGC AAG TTC          890
   Ile Leu Met Phe Ala Ile Ile Gly Leu Glu Phe Tyr Met Gly Lys Phe
       235                     240                     245
   CAC AAG GCC TGT TTC CCC AAC AGC ACA GAT GCG GAG CCC GTG GGT GAC          938
   His Lys Ala Cys Phe Pro Asn Ser Thr Asp Ala Glu Pro Val Gly Asp
   250                 255                     260                     265
   TTC CCC TGT GGC AAG GAG GCC CCA GCC CGG CTG TGC GAG GGC GAC ACT          986
   Phe Pro Cys Gly Lys Glu Ala Pro Ala Arg Leu Cys Glu Gly Asp Thr
                   270                     275                     280
   GAG TGC CGG GAG TAC TGG CCA GGA CCC AAC TTT GGC ATC ACC AAC TTT         1034
   Glu Cys Arg Glu Tyr Trp Pro Gly Pro Asn Phe Gly Ile Thr Asn Phe
               285                     290                     295
   GAC AAT ATC CTG TTT GCC ATC TTG ACG GTG TTC CAG TGC ATC ACC ATG         1082
   Asp Asn Ile Leu Phe Ala Ile Leu Thr Val Phe Gln Cys Ile Thr Met
           300                     305                     310
   GAG GGC TGG ACT GAC ATC CTC TAT AAT ACA AAC GAT GCG GCC GGC AAC         1130
   Glu Gly Trp Thr Asp Ile Leu Tyr Asn Thr Asn Asp Ala Ala Gly Asn
       315                     320                     325
   ACC TGG AAC TGG CTC TAC TTC ATC CCT CTC ATC ATC ATC GGC TCC TTC         1178
   Thr Trp Asn Trp Leu Tyr Phe Ile Pro Leu Ile Ile Ile Gly Ser Phe
   330                 335                     340                     345
   TTC ATG CTC AAC CTG GTG CTG GGC GTG CTC TCG GGG GAG TTT GCC AAG         1226
   Phe Met Leu Asn Leu Val Leu Gly Val Leu Ser Gly Glu Phe Ala Lys
                   350                     355                     360
   GAG CGA GAG AGG GTG GAG AAC CGC CGC GCC TTC CTG AAG CTG CGC CGG         1274
   Glu Arg Glu Arg Val Glu Asn Arg Arg Ala Phe Leu Lys Leu Arg Arg
               365                     370                     375
   CAG CAG CAG ATC GAG CGA GAG CTC AAC GGG TAC CTG GAG TGG ATC TTC         1322
   Gln Gln Gln Ile Glu Arg Glu Leu Asn Gly Tyr Leu Glu Trp Ile Phe
           380                     385                     390
   AAG GCG GAG GAA GTC ATG CTG GCC GAG GAG GAC AGG AAT GCA GAG GAG         1370
   Lys Ala Glu Glu Val Met Leu Ala Glu Glu Asp Arg Asn Ala Glu Glu
   395 400 405
   AAG TCC CCT TTG GAC GTG CTG AAG AGA GCG GCC ACC AAG AAG AGC AGA 1418
   Lys Ser Pro Leu Asp Val Leu Lys Arg Ala Ala Thr Lys Lys Ser Arg
   410 415 420 425
   AAT GAC CTG ATC CAC GCA GAG GAG GGA GAG GAC CGG TTT GCA GAT CTC 1466
   Asn Asp Leu Ile His Ala Glu Glu Gly Glu Asp Arg Phe Ala Asp Leu
   430 435 440
   TGT GCT GTT GGA TCC CCC TTC GCC CGC GCC AGC CTC AAG AGC GGG AAG 1514
   Cys Ala Val Gly Ser Pro Phe Ala Arg Ala Ser Leu Lys Ser Gly Lys
   445 450 455
   ACA GAG AGC TCG TCA TAC TTC CGG AGG AAG GAG AAG ATG TTC CGG TTT 1562
   Thr Glu Ser Ser Ser Tyr Phe Arg Arg Lys Glu Lys Met Phe Arg Phe
   460 465 470
   TTT ATC CGG CGC ATG GTG AAG GCT CAG AGC TTC TAC TGG GTG GTG CTG 1610
   Phe Ile Arg Arg Met Val Lys Ala Gln Ser Phe Tyr Trp Val Val Leu
   475 480 485
   TGC GTG GTG GCC CTG AAC ACA CTG TGT GTG GCC ATG GTG CAT TAC AAC 1658
   Cys Val Val Ala Leu Asn Thr Leu Cys Val Ala Met Val His Tyr Asn
   490 495 500 505
   CAG CCG CGG CGG CTT ACC ACG ACC CTG TAT TTT GCA GAG TTT GTT TTC 1706
   Gln Pro Arg Arg Leu Thr Thr Thr Leu Tyr Phe Ala Glu Phe Val Phe
   510 515 520
   CTG GGT CTC TTC CTC ACA GAG ATG TCC CTG AAG ATG TAT GGC CTG GGG 1754
   Leu Gly Leu Phe Leu Thr Glu Met Ser Leu Lys Met Tyr Gly Leu Gly
   525 530 535
   CCC AGA AGC TAC TTC CGG TCC TCC TTC AAC TGC TTC GAC TTT GGG GTC 1802
   Pro Arg Ser Tyr Phe Arg Ser Ser Phe Asn Cys Phe Asp Phe Gly Val
   540 545 550
   ATC GTG GGG AGC GTC TTT GAA GTG GTC TGG GCG GCC ATC AAG CCG GGA 1850
   Ile Val Gly Ser Val Phe Glu Val Val Trp Ala Ala Ile Lys Pro Gly
   555 560 565
   AGC TCC TTT GGG ATC AGT GTG CTG CGG GCC CTC CGC CTG CTG AGG ATC 1898
   Ser Ser Phe Gly Ile Ser Val Leu Arg Ala Leu Arg Leu Leu Arg Ile
   570 575 580 585
   TTC AAA GTC ACG AAG TAC TGG AGC TCC CTG CGG AAC CTG GTG GTG TCC 1946
   Phe Lys Val Thr Lys Tyr Trp Ser Ser Leu Arg Asn Leu Val Val Ser
   590 595 600
   CTG CTG AAC TCC ATG AAG TCC ATC ATC AGC CTG CTC TTC TTG CTC TTC 1994
   Leu Leu Asn Ser Met Lys Ser Ile Ile Ser Leu Leu Phe Leu Leu Phe
   605 610 615
   CTG TTC ATT GTG GTC TTC GCC CTG CTG GGG ATG CAG CTG TTT GGG GGA 2042
   Leu Phe Ile Val Val Phe Ala Leu Leu Gly Met Gln Leu Phe Gly Gly
   620 625 630
   CAG TTC AAC TTC CAG GAT GAG ACT CCC ACA ACC AAC TTC GAC ACC TTC 2090
   Gln Phe Asn Phe Gln Asp Glu Thr Pro Thr Thr Asn Phe Asp Thr Phe
   635 640 645
   CCT GCC GCC ATC CTC ACT GTC TTC CAG ATC CTG ACG GGA GAG GAC TGG 2138
   Pro Ala Ala Ile Leu Thr Val Phe Gln Ile Leu Thr Gly Glu Asp Trp
   650 655 660 665
   AAT GCA GTG ATG TAT CAC GGG ATC GAA TCG CAA GGC GGC GTC AGC AAA 2186
   Asn Ala Val Met Tyr His Gly Ile Glu Ser Gln Gly Gly Val Ser Lys
   670 675 680
   GGC ATG TTC TCG TCC TTT TAC TTC ATT GTC CTG ACA CTG TTC GGA AAC      2234
   Gly Met Phe Ser Ser Phe Tyr Phe Ile Val Leu Thr Leu Phe Gly Asn
               685                 690                 695
   TAC ACT CTG CTG AAT GTC TTT CTG GCC ATC GCT GTG GAC AAC CTG GCC      2282
   Tyr Thr Leu Leu Asn Val Phe Leu Ala Ile Ala Val Asp Asn Leu Ala
           700                 705                 710
   AAC GCC CAA GAG CTG ACC AAG GAT GAA GAG GAG ATG GAA GAA GCA GCC      2330
   Asn Ala Gln Glu Leu Thr Lys Asp Glu Glu Glu Met Glu Glu Ala Ala
       715                 720                 725
   AAT CAG AAG CTT GCT CTG CAA AAG GCC AAA GAA GTG GCT GAA GTC AGC      2378
   Asn Gln Lys Leu Ala Leu Gln Lys Ala Lys Glu Val Ala Glu Val Ser
   730                 735                 740                 745
   CCC ATG TCT GCC GCG AAC ATC TCC ATC GCC GCC AGG CAG CAG AAC TCG      2426
   Pro Met Ser Ala Ala Asn Ile Ser Ile Ala Ala Arg Gln Gln Asn Ser
                   750                 755                 760
   GCC AAG GCG CGC TCG GTG TGG GAG CAG CGG GCC AGC CAG CTA CGG CTG      2474
   Ala Lys Ala Arg Ser Val Trp Glu Gln Arg Ala Ser Gln Leu Arg Leu
               765                 770                 775
   CAG AAC CTG CGG GCC AGC TGC GAG GCG CTG TAC AGC GAG ATG GAC CCC      2522
   Gln Asn Leu Arg Ala Ser Cys Glu Ala Leu Tyr Ser Glu Met Asp Pro
           780                 785                 790
   GAG GAG CGG CTG CGC TTC GCC ACT ACG CGC CAC CTG CGG CCC GAC ATG      2570
   Glu Glu Arg Leu Arg Phe Ala Thr Thr Arg His Leu Arg Pro Asp Met
       795                 800                 805
   AAG ACG CAC CTG GAC CGG CCG CTG GTG GTG GAG CTG GGC CGC GAC GGC      2618
   Lys Thr His Leu Asp Arg Pro Leu Val Val Glu Leu Gly Arg Asp Gly
   810                 815                 820                 825
   GCG CGG GGG CCC GTG GGA GGC AAA GCC CGA CCT GAG GCT GCG GAG GCC      2666
   Ala Arg Gly Pro Val Gly Gly Lys Ala Arg Pro Glu Ala Ala Glu Ala
                   830                 835                 840
   CCC GAG GGC GTC GAC CCT CCG CGC AGG CAC CAC CGG CAC CGC GAC AAG      2714
   Pro Glu Gly Val Asp Pro Pro Arg Arg His His Arg His Arg Asp Lys
               845                 850                 855
   GAC AAG ACC CCC GCG GCG GGG GAC CAG GAC CGA GCA GAG GCC CCG AAG      2762
   Asp Lys Thr Pro Ala Ala Gly Asp Gln Asp Arg Ala Glu Ala Pro Lys
           860                 865                 870
   GCG GAG AGC GGG GAG CCC GGT GCC CGG GAG GAG CGG CCG CGG CCG CAC      2810
   Ala Glu Ser Gly Glu Pro Gly Ala Arg Glu Glu Arg Pro Arg Pro His
       875                 880                 885
   CGC AGC CAC AGC AAG GAG GCC GCG GGG CCC CCG GAG GCG CGG AGC GAG      2858
   Arg Ser His Ser Lys Glu Ala Ala Gly Pro Pro Glu Ala Arg Ser Glu
   890                 895                 900                 905
   CGC GGC CGA GGC CCA GGC CCC GAG GGC GGC CGG CGG CAC CAC CGG CGC      2906
   Arg Gly Arg Gly Pro Gly Pro Glu Gly Gly Arg Arg His His Arg Arg
                   910                 915                 920
   GGC TCC CCG GAG GAG GCG GCC GAG CGG GAG CCC CGA CGC CAC CGC GCG      2954
   Gly Ser Pro Glu Glu Ala Ala Glu Arg Glu Pro Arg Arg His Arg Ala
               925                 930                 935
   CAC CGG CAC CAG GAT CCG AGC AAG GAG TGC GCC GGC GCC AAG GGC GAG      3002
   His Arg His Gln Asp Pro Ser Lys Glu Cys Ala Gly Ala Lys Gly Glu
           940                 945                 950
   CGG CGC GCG CGG CAC CGC GGC GGC CCC CGA GCG GGG CCC CGG GAG GCG      3050
   Arg Arg Ala Arg His Arg Gly Gly Pro Arg Ala Gly Pro Arg Glu Ala
       955                 960                 965
   GAG AGC GGG GAG GAG CCG GCG CGG CGG CAC CGG GCC CGG CAC AAG GCG        3098
   Glu Ser Gly Glu Glu Pro Ala Arg Arg His Arg Ala Arg His Lys Ala
   970                 975                 980                 985
   CAG CCT GCT CAC GAG GCT GTG GAG AAG GAG ACC ACG GAG AAG GAG GCC        3146
   Gln Pro Ala His Glu Ala Val Glu Lys Glu Thr Thr Glu Lys Glu Ala
                   990                 995                 1000
   ACG GAG AAG GAG GCT GAG ATA GTG GAA GCC GAC AAG GAA AAG GAG CTC        3194
   Thr Glu Lys Glu Ala Glu Ile Val Glu Ala Asp Lys Glu Lys Glu Leu
               1005                1010                1015
   CGG AAC CAC CAG CCC CGG GAG CCA CAC TGT GAC CTG GAG ACC AGT GGG        3242
   Arg Asn His Gln Pro Arg Glu Pro His Cys Asp Leu Glu Thr Ser Gly
           1020                1025                1030
   ACT GTG ACT GTG GGT CCC ATG CAC ACA CTG CCC AGC ACC TGT CTC CAG        3290
   Thr Val Thr Val Gly Pro Met His Thr Leu Pro Ser Thr Cys Leu Gln
       1035                1040                1045
   AAG GTG GAG GAA CAG CCA GAG GAT GCA GAC AAT CAG CGG AAC GTC ACT        3338
   Lys Val Glu Glu Gln Pro Glu Asp Ala Asp Asn Gln Arg Asn Val Thr
   1050                1055                1060                1065
   CGC ATG GGC AGT CAG CCC CCA GAC CCG AAC ACT ATT GTA CAT ATC CCA        3386
   Arg Met Gly Ser Gln Pro Pro Asp Pro Asn Thr Ile Val His Ile Pro
                   1070                1075                1080
   GTG ATG CTG ACG GGC CCT CTT GGG GAA GCC ACG GTC GTT CCC AGT GGT        3434
   Val Met Leu Thr Gly Pro Leu Gly Glu Ala Thr Val Val Pro Ser Gly
               1085                1090                1095
   AAC GTG GAC CTG GAA AGC CAA GCA GAG GGG AAG AAG GAG GTG GAA GCG        3482
   Asn Val Asp Leu Glu Ser Gln Ala Glu Gly Lys Lys Glu Val Glu Ala
           1100                1105                1110
   GAT GAC GTG ATG AGG AGC GGC CCC CGG CCT ATC GTC CCA TAC AGC TCC        3530
   Asp Asp Val Met Arg Ser Gly Pro Arg Pro Ile Val Pro Tyr Ser Ser
       1115                1120                1125
   ATG TTC TGT TTA AGC CCC ACC AAC CTG CTC CGC CGC TTC TGC CAC TAC        3578
   Met Phe Cys Leu Ser Pro Thr Asn Leu Leu Arg Arg Phe Cys His Tyr
   1130                1135                1140                1145
   ATC GTG ACC ATG AGG TAC TTC GAG GTG GTC ATT CTC GTG GTC ATC GCC        3626
   Ile Val Thr Met Arg Tyr Phe Glu Val Val Ile Leu Val Val Ile Ala
                   1150                1155                1160
   TTG AGC AGC ATC GCC CTG GCT GCT GAG GAC CCA GTG CGC ACA GAC TCG        3674
   Leu Ser Ser Ile Ala Leu Ala Ala Glu Asp Pro Val Arg Thr Asp Ser
               1165                1170                1175
   CCC AGG AAC AAC GCT CTG AAA TAC CTG GAT TAC ATT TTC ACT GGT GTC        3722
   Pro Arg Asn Asn Ala Leu Lys Tyr Leu Asp Tyr Ile Phe Thr Gly Val
           1180                1185                1190
   TTT ACC TTT GAG ATG GTG ATA AAG ATG ATC GAC TTG GGA CTG CTG CTT        3770
   Phe Thr Phe Glu Met Val Ile Lys Met Ile Asp Leu Gly Leu Leu Leu
       1195                1200                1205
   CAC CCT GGA GCC TAT TTC CGG GAC TTG TGG AAC ATT CTG GAC TTC ATT        3818
   His Pro Gly Ala Tyr Phe Arg Asp Leu Trp Asn Ile Leu Asp Phe Ile
   1210                1215                1220                1225
   GTG GTC AGT GGC GCC CTG GTG GCG TTT GCT TTC TCA GGA TCC AAA GGG        3866
   Val Val Ser Gly Ala Leu Val Ala Phe Ala Phe Ser Gly Ser Lys Gly
                   1230                1235                1240
   AAA GAC ATC AAT ACC ATC AAG TCT CTG AGA GTC CTT CGT GTC CTG CGG        3914
   Lys Asp Ile Asn Thr Ile Lys Ser Leu Arg Val Leu Arg Val Leu Arg
               1245                1250                1255
   CCC CTC AAG ACC ATC AAA CGG CTG CCC AAG CTC AAG GCT GTG TTT GAC        3962
   Pro Leu Lys Thr Ile Lys Arg Leu Pro Lys Leu Lys Ala Val Phe Asp
           1260                1265                1270
   TGT GTG GTG AAC TCC CTG AAG AAT GTC CTC AAC ATC TTG ATT GTC TAC        4010
   Cys Val Val Asn Ser Leu Lys Asn Val Leu Asn Ile Leu Ile Val Tyr
       1275                1280                1285
   ATG CTC TTC ATG TTC ATA TTT GCC GTC ATT GCG GTG CAG CTC TTC AAA        4058
   Met Leu Phe Met Phe Ile Phe Ala Val Ile Ala Val Gln Leu Phe Lys
   1290                1295                1300                1305
   GGG AAG TTT TTC TAC TGC ACA GAT GAA TCC AAG GAG CTG GAG AGG GAC        4106
   Gly Lys Phe Phe Tyr Cys Thr Asp Glu Ser Lys Glu Leu Glu Arg Asp
                   1310                1315                1320
   TGC AGG GGT CAG TAT TTG GAT TAT GAG AAG GAG GAA GTG GAA GCT CAG        4154
   Cys Arg Gly Gln Tyr Leu Asp Tyr Glu Lys Glu Glu Val Glu Ala Gln
               1325                1330                1335
   CCC AGG CAG TGG AAG AAA TAC GAC TTT CAC TAC GAC AAT GTG CTC TGG        4202
   Pro Arg Gln Trp Lys Lys Tyr Asp Phe His Tyr Asp Asn Val Leu Trp
           1340                1345                1350
   GCT CTG CTG ACG CTG TTC ACA GTG TCC ACG GGA GAA GGC TGG CCC ATG        4250
   Ala Leu Leu Thr Leu Phe Thr Val Ser Thr Gly Glu Gly Trp Pro Met
       1355                1360                1365
   GTG CTG AAA CAC TCC GTG GAT GCC ACC TAT GAG GAG CAG GGT CCA AGC        4298
   Val Leu Lys His Ser Val Asp Ala Thr Tyr Glu Glu Gln Gly Pro Ser
   1370                1375                1380                1385
   CCT GGG TAC CGC ATG GAG CTG TCC ATC TTC TAC GTG GTC TAC TTT GTG        4346
   Pro Gly Tyr Arg Met Glu Leu Ser Ile Phe Tyr Val Val Tyr Phe Val
                   1390                1395                1400
   GTC TTT CCC TTC TTC TTC GTC AAC ATC TTT GTG GCT TTG ATC ATC ATC        4394
   Val Phe Pro Phe Phe Phe Val Asn Ile Phe Val Ala Leu Ile Ile Ile
               1405                1410                1415
   ACC TTC CAG GAG CAG GGG GAC AAG GTG ATG TCT GAA TGC AGC CTG GAG        4442
   Thr Phe Gln Glu Gln Gly Asp Lys Val Met Ser Glu Cys Ser Leu Glu
           1420                1425                1430
   AAG AAC GAG AGG GCT TGC ATT GAC TTC GCC ATC AGC GCC AAA CCC CTG        4490
   Lys Asn Glu Arg Ala Cys Ile Asp Phe Ala Ile Ser Ala Lys Pro Leu
       1435                1440                1445
   ACA CGG TAC ATG CCC CAA AAC CGG CAG TCG TTC CAG TAT AAG ACG TGG        4538
   Thr Arg Tyr Met Pro Gln Asn Arg Gln Ser Phe Gln Tyr Lys Thr Trp
   1450                1455                1460                1465
   ACA TTT GTG GTC TCC CCG CCC TTT GAA TAC TTC ATC ATG GCC ATG ATA        4586
   Thr Phe Val Val Ser Pro Pro Phe Glu Tyr Phe Ile Met Ala Met Ile
                   1470                1475                1480
   GCC CTC AAC ACT GTG GTG CTG ATG ATG AAG TTC TAT GAT GCA CCC TAT        4634
   Ala Leu Asn Thr Val Val Leu Met Met Lys Phe Tyr Asp Ala Pro Tyr
               1485                1490                1495
   GAG TAC GAG CTG ATG CTG AAA TGC CTG AAC ATC GTG TTC ACA TCC ATG        4682
   Glu Tyr Glu Leu Met Leu Lys Cys Leu Asn Ile Val Phe Thr Ser Met
           1500                1505                1510
   TTC TCC ATG GAA TGC GTG CTG AAG ATC ATC GCC TTT GGG GTG CTG AAC        4730
   Phe Ser Met Glu Cys Val Leu Lys Ile Ile Ala Phe Gly Val Leu Asn
       1515                1520                1525
   TAT TTC AGA GAT GCC TGG AAT GTC TTT GAC TTT GTC ACT GTG TTG GGA      4778
   Tyr Phe Arg Asp Ala Trp Asn Val Phe Asp Phe Val Thr Val Leu Gly
   1530                1535                1540                1545
   AGT ATT ACT GAT ATT TTA GTA ACA GAG ATT GCG GAA ACG AAC AAT TTC      4826
   Ser Ile Thr Asp Ile Leu Val Thr Glu Ile Ala Glu Thr Asn Asn Phe
                   1550                1555                1560
   ATC AAC CTC AGC TTC CTC CGC CTC TTT CGA GCT GCG CGG CTG ATC AAG      4874
   Ile Asn Leu Ser Phe Leu Arg Leu Phe Arg Ala Ala Arg Leu Ile Lys
               1565                1570                1575
   CTG CTC CGC CAG GGC TAC ACC ATC CGC ATC CTG CTG TGG ACC TTT GTC      4922
   Leu Leu Arg Gln Gly Tyr Thr Ile Arg Ile Leu Leu Trp Thr Phe Val
           1580                1585                1590
   CAG TCC TTC AAG GCC CTG CCC TAC GTG TGT CTG CTC ATT GCC ATG CTG      4970
   Gln Ser Phe Lys Ala Leu Pro Tyr Val Cys Leu Leu Ile Ala Met Leu
       1595                1600                1605
   TTC TTC ATC TAC GCC ATC ATC GGC ATG CAG GTG TTT GGG AAT ATT GCC      5018
   Phe Phe Ile Tyr Ala Ile Ile Gly Met Gln Val Phe Gly Asn Ile Ala
   1610                1615                1620                1625
   CTG GAT GAT GAC ACC AGC ATC AAC CGC CAC AAC AAC TTC CGG ACG TTT      5066
   Leu Asp Asp Asp Thr Ser Ile Asn Arg His Asn Asn Phe Arg Thr Phe
                   1630                1635                1640
   TTG CAA GCC CTG ATG CTG CTG TTC AGG AGC GCC ACG GGG GAG GCC TGG      5114
   Leu Gln Ala Leu Met Leu Leu Phe Arg Ser Ala Thr Gly Glu Ala Trp
               1645                1650                1655
   CAC GAG ATC ATG CTG TCC TGC CTG AGC AAC CAG GCC TGT GAT GAG CAG      5162
   His Glu Ile Met Leu Ser Cys Leu Ser Asn Gln Ala Cys Asp Glu Gln
           1660                1665                1670
   GCC AAT GCC ACC GAG TGT GGA AGT GAC TTT GCC TAC TTC TAC TTC GTC      5210
   Ala Asn Ala Thr Glu Cys Gly Ser Asp Phe Ala Tyr Phe Tyr Phe Val
       1675                1680                1685
   TCC TTC ATC TTC CTG TGC TCC TTT CTG ATG TTG AAC CTC TTT GTG GCT      5258
   Ser Phe Ile Phe Leu Cys Ser Phe Leu Met Leu Asn Leu Phe Val Ala
   1690                1695                1700                1705
   GTG ATC ATG GAC AAT TTT GAG TAC CTC ACG CGG GAC TCT TCC ATC CTA      5306
   Val Ile Met Asp Asn Phe Glu Tyr Leu Thr Arg Asp Ser Ser Ile Leu
                   1710                1715                1720
   GGT CCT CAC CAC TTG GAT GAG TTC ATC CGG GTC TGG GCT GAA TAC GAC      5354
   Gly Pro His His Leu Asp Glu Phe Ile Arg Val Trp Ala Glu Tyr Asp
               1725                1730                1735
   CCG GCT GCG TGT GGG CGC ATC AGT TAC AAT GAC ATG TTT GAG ATG CTG      5402
   Pro Ala Ala Cys Gly Arg Ile Ser Tyr Asn Asp Met Phe Glu Met Leu
           1740                1745                1750
   AAA CAC ATG TCC CCG CCT CTG GGG CTG GGG AAG AAA TGC CCT GCT CGA      5450
   Lys His Met Ser Pro Pro Leu Gly Leu Gly Lys Lys Cys Pro Ala Arg
       1755                1760                1765
   GTT GCT TAC AAG CGC CTG GTT CGC ATG AAC ATG CCC ATC TCC AAC GAG      5498
   Val Ala Tyr Lys Arg Leu Val Arg Met Asn Met Pro Ile Ser Asn Glu
   1770                1775                1780                1785
   GAC ATG ACT GTT CAC TTC ACG TCC ACG CTG ATG GCC CTC ATC CGG ACG      5546
   Asp Met Thr Val His Phe Thr Ser Thr Leu Met Ala Leu Ile Arg Thr
                   1790                1795                1800
   GCA CTG GAG ATC AAG CTG GCC CCA GCT GGG ACA AAG CAG CAT CAG TGT      5594
   Ala Leu Glu Ile Lys Leu Ala Pro Ala Gly Thr Lys Gln His Gln Cys
                  1805                1810                1815
   GAC GCG GAG TTG AGG AAG GAG ATT TCC GTT GTG TGG GCC AAT CTG CCC      5642
   Asp Ala Glu Leu Arg Lys Glu Ile Ser Val Val Trp Ala Asn Leu Pro
             1820                1825                1830
   CAG AAG ACT TTG GAC TTG CTG GTA CCA CCC CAT AAG CCT GAT GAG ATG      5690
   Gln Lys Thr Leu Asp Leu Leu Val Pro Pro His Lys Pro Asp Glu Met
        1835                1840                1845
   ACA GTG GGG AAG GTT TAT GCA GCT CTG ATG ATA TTT GAC TTC TAC AAG      5738
   Thr Val Gly Lys Val Tyr Ala Ala Leu Met Ile Phe Asp Phe Tyr Lys
   1850                1855                1860                1865
   CAG AAC AAA ACC ACC AGA GAC CAG ATG CAG CAG GCT CCT GGA GGC CTC      5786
   Gln Asn Lys Thr Thr Arg Asp Gln Met Gln Gln Ala Pro Gly Gly Leu
                       1870                1875                1880
   TCC CAG ATG GGT CCT GTG TCC CTG TTC CAC CCT CTG AAG GCC ACC CTG      5834
   Ser Gln Met Gly Pro Val Ser Leu Phe His Pro Leu Lys Ala Thr Leu
                  1885                1890                1895
   GAG CAG ACA CAG CCG GCT GTG CTC CGA GGA GCC CGG GTT TTC CTT CGA      5882
   Glu Gln Thr Gln Pro Ala Val Leu Arg Gly Ala Arg Val Phe Leu Arg
             1900                1905                1910
   CAG AAG AGT TCC ACC TCC CTC AGC AAT GGC GGG GCC ATA CAA AAC CAA      5930
   Gln Lys Ser Ser Thr Ser Leu Ser Asn Gly Gly Ala Ile Gln Asn Gln
        1915                1920                1925
   GAG AGT GGC ATC AAA GAG TCT GTC TCC TGG GGC ACT CAA AGG ACC CAG      5978
   Glu Ser Gly Ile Lys Glu Ser Val Ser Trp Gly Thr Gln Arg Thr Gln
   1930                1935                1940                1945
   GAT GCA CCC CAT GAG GCC AGG CCA CCC CTG GAG CGT GGC CAC TCC ACA      6026
   Asp Ala Pro His Glu Ala Arg Pro Pro Leu Glu Arg Gly His Ser Thr
                       1950                1955                1960
   GAG ATC CCT GTG GGG CGG TCA GGA GCA CTG GCT GTG GAC GTT CAG ATG      6074
   Glu Ile Pro Val Gly Arg Ser Gly Ala Leu Ala Val Asp Val Gln Met
                  1965                1970                1975
   CAG AGC ATA ACC CGG AGG GGC CCT GAT GGG GAG CCC CAG CCT GGG CTG      6122
   Gln Ser Ile Thr Arg Arg Gly Pro Asp Gly Glu Pro Gln Pro Gly Leu
             1980                1985                1990
   GAG AGC CAG GGT CGA GCG GCC TCC ATG CCC CGC CTT GCG GCC GAG ACT      6170
   Glu Ser Gln Gly Arg Ala Ala Ser Met Pro Arg Leu Ala Ala Glu Thr
        1995                2000                2005
   CAG CCC GTC ACA GAT GCC AGC CCC ATG AAG CGC TCC ATC TCC ACG CTG      6218
   Gln Pro Val Thr Asp Ala Ser Pro Met Lys Arg Ser Ile Ser Thr Leu
   2010                2015                2020                2025
   GCC CAG CGG CCC CGT GGG ACT CAT CTT TGC AGC ACC ACC CCG GAC CGC      6266
   Ala Gln Arg Pro Arg Gly Thr His Leu Cys Ser Thr Thr Pro Asp Arg
                       2030                2035                2040
   CCA CCC CCT AGC CAG GCG TCG TCG CAC CAC CAC CAC CAC CGC TGC CAC      6314
   Pro Pro Pro Ser Gln Ala Ser Ser His His His His His Arg Cys His
                  2045                2050                2055
   CGC CGC AGG GAC AGG AAG CAG AGG TCC CTG GAG AAG GGG CCC AGC CTG      6362
   Arg Arg Arg Asp Arg Lys Gln Arg Ser Leu Glu Lys Gly Pro Ser Leu
             2060                2065                2070
   TCT GCC GAT ATG GAT GGC GCA CCA AGC AGT GCT GTG GGG CCG GGG CTG      6410
   Ser Ala Asp Met Asp Gly Ala Pro Ser Ser Ala Val Gly Pro Gly Leu
       2075                2080                2085
   CCC CCG GGA GAG GGG CCT ACA GGC TGC CGG CGG GAA CGA GAG CGC CGG      6458
   Pro Pro Gly Glu Gly Pro Thr Gly Cys Arg Arg Glu Arg Glu Arg Arg
   2090                2095                2100                2105
   CAG GAG CGG GGC CGG TCC CAG GAG CGG AGG CAG CCC TCA TCC TCC TCC      6506
   Gln Glu Arg Gly Arg Ser Gln Glu Arg Arg Gln Pro Ser Ser Ser Ser
                   2110                2115                2120
   TCG GAG AAG CAG CGC TTC TAC TCC TGC GAC CGC TTT GGG GGC CGT GAG      6554
   Ser Glu Lys Gln Arg Phe Tyr Ser Cys Asp Arg Phe Gly Gly Arg Glu
               2125                2130                2135
   CCC CCG AAG CCC AAG CCC TCC CTC AGC AGC CAC CCA ACG TCG CCA ACA      6602
   Pro Pro Lys Pro Lys Pro Ser Leu Ser Ser His Pro Thr Ser Pro Thr
           2140                2145                2150
   GCT GGC CAG GAG CCG GGA CCC CAC CCA CAG GCC GGC TCA GCC GTG GGC      6650
   Ala Gly Gln Glu Pro Gly Pro His Pro Gln Ala Gly Ser Ala Val Gly
       2155                2160                2165
   TTT CCG AAC ACA ACG CCC TGC TGC AGA GAG ACC CCC TCA GCC AGC CCC      6698
   Phe Pro Asn Thr Thr Pro Cys Cys Arg Glu Thr Pro Ser Ala Ser Pro
   2170                2175                2180                2185
   TGG CCC CTG GCT CTC GAA TTG GCT CTG ACC CTT ACC TGG GGC AGC GTC      6746
   Trp Pro Leu Ala Leu Glu Leu Ala Leu Thr Leu Thr Trp Gly Ser Val
                   2190                2195                2200
   TGG ACA GTG AGG CCT CTG TCC ACG CCC TGC CTG AGG ACA CGC TCA CTT      6794
   Trp Thr Val Arg Pro Leu Ser Thr Pro Cys Leu Arg Thr Arg Ser Leu
               2205                2210                2215
   TCG AGG AGG CTG TGG CCA CCA ACT CGG GCC GCT CCT CCA GGA CTT CCT      6842
   Ser Arg Arg Leu Trp Pro Pro Thr Arg Ala Ala Pro Pro Gly Leu Pro
           2220                2225                2230
   ACG TGT CCT CCC TGACCTCCCA GTCTCACCCT CTCCGCCGCG TGCCCAACGG          6894
   Thr Cys Pro Pro
       2235
   TTACCACTGC ACCCTGGGAC TCAGCTCGGG TGGCCGAGCA CGGCACAGCT ACCACCACCC    6954
   TGACCAAGAC CACTGGTGCT AGCTGCACCG TGACCGCTCA GACGCCTGCA TGCAGCAGGC    7014
   GTGTGTTCCA GTGGATGAGT TTTATCATCC ACACGGGGCA GTCGGCCCTC GGGGGAGGCC    7074
   TTGCCCACCT TGGTGAGGCT CCTGTGGCCC CTCCCTCCCC CTCCTCCCCT CTTTTACTCT    7134
   AGACGACGAA TAAAGCCCTG TTGCTTGAGT GTACGTACCG C                        7175

### (2) INFORMATION FOR SEQ ID NO:9:

(i) SEQUENCE CHARACTERISTICS:
   (A) LENGTH: 1546 base pairs
   (B) TYPE: nucleic acid
   (C) STRANDEDNESS: double
   (D) TOPOLOGY: linear
(ii) MOLECULE TYPE: DNA (genomic)
(ix) FEATURE:
   (A) NAME/KEY: CDS
   (B) LOCATION: 1..1437
(ix) FEATURE:
   (A) NAME/KEY: 3'UTR
   (B) LOCATION: 1435..1546
(xi) SEQUENCE DESCRIPTION: SEQ ID NO:9:

### (2 ) INFORMATION FOR SEQ ID NO:10 :

(i) SEQUENCE CHARACTERISTICS:
   (A) LENGTH: 1851 base pairs
   (B) TYPE: nucleic acid
   (C) STRANDEDNESS: double
   (D) TOPOLOGY: linear
(ii) MOLECULE TYPE: DNA (genomic)
(ix) FEATURE:
   (A) NAME/KEY: CDS
   (B) LOCATION: 1..1797
   (D) OTHER INFORMATION: /standard_name= "Beta-3"
(ix) FEATURE:
   (A) NAME/KEY: 3'UTR
   (B) LOCATION: 1795..1851
(xi) SEQUENCE DESCRIPTION: SEQ ID NO:10:

### (2) INFORMATION FOR SEQ ID NO:11:

(i) SEQUENCE CHARACTERISTICS:
   (A) LENGTH: 3600 base pairs
   (B) TYPE: nucleic acid
   (C) STRANDEDNESS: double
   (D) TOPOLOGY: linear
(ii) MOLECULE TYPE: DNA (genomic)
(ix) FEATURE:
   (A) NAME/KEY: CDS
   (B) LOCATION: 35..3310
   (D) OTHER INFORMATION: /standard_name= "Alpha-2"
(ix) FEATURE:
   (A) NAME/KEY: 5'UTR
   (B) LOCATION: 1..34
(ix) FEATURE:
   (A) NAME/KEY: 3'UTR
   (B) LOCATION: 3308..3600
(xi) SEQUENCE DESCRIPTION: SEQ ID NO:11:
   GCGGGGGAGG GGGCATTGAT CTTCGATCGC GAAG ATG GCT GCT GGC TGC CTG       52
                                         Met Ala Ala Gly Cys Leu
                                          1               5
   CTG GCC TTG ACT CTG ACA CTT TTC CAA TCT TTG CTC ATC GGC CCC TCG    100
   Leu Ala Leu Thr Leu Thr Leu Phe Gln Ser Leu Leu Ile Gly Pro Ser
               10                  15                  20
   TCG GAG GAG CCG TTC CCT TCG GCC GTC ACT ATC AAA TCA TGG GTG GAT    148
   Ser Glu Glu Pro Phe Pro Ser Ala Val Thr Ile Lys Ser Trp Val Asp
           25                  30                  35
   AAG ATG CAA GAA GAC CTT GTC ACA CTG GCA AAA ACA GCA AGT GGA GTC    196
   Lys Met Gln Glu Asp Leu Val Thr Leu Ala Lys Thr Ala Ser Gly Val
       40                  45                  50
   AAT CAG CTT GTT GAT ATT TAT GAG AAA TAT CAA GAT TTG TAT ACT GTG    244
   Asn Gln Leu Val Asp Ile Tyr Glu Lys Tyr Gln Asp Leu Tyr Thr Val
    55                  60                  65                  70
   GAA CCA AAT AAT GCA CGC CAG CTG GTA GAA ATT GCA GCC AGG GAT ATT      292
   Glu Pro Asn Asn Ala Arg Gln Leu Val Glu Ile Ala Ala Arg Asp Ile
                    75                  80                  85
   GAG AAA CTT CTG AGC AAC AGA TCT AAA GCC CTG GTG AGC CTG GCA TTG      340
   Glu Lys Leu Leu Ser Asn Arg Ser Lys Ala Leu Val Ser Leu Ala Leu
                90                  95                 100
   GAA GCG GAG AAA GTT CAA GCA GCT CAC CAG TGG AGA GAA GAT TTT GCA      388
   Glu Ala Glu Lys Val Gln Ala Ala His Gln Trp Arg Glu Asp Phe Ala
           105                 110                 115
   AGC AAT GAA GTT GTC TAC TAC AAT GCA AAG GAT GAT CTC GAT CCT GAG      436
   Ser Asn Glu Val Val Tyr Tyr Asn Ala Lys Asp Asp Leu Asp Pro Glu
       120                 125                 130
   AAA AAT GAC AGT GAG CCA GGC AGC CAG AGG ATA AAA CCT GTT TTC ATT      484
   Lys Asn Asp Ser Glu Pro Gly Ser Gln Arg Ile Lys Pro Val Phe Ile
   135                 140                 145                 150
   GAA GAT GCT AAT TTT GGA CGA CAA ATA TCT TAT CAG CAC GCA GCA GTC      532
   Glu Asp Ala Asn Phe Gly Arg Gln Ile Ser Tyr Gln His Ala Ala Val
                   155                 160                 165
   CAT ATT CCT ACT GAC ATC TAT GAG GGC TCA ACA ATT GTG TTA AAT GAA      580
   His Ile Pro Thr Asp Ile Tyr Glu Gly Ser Thr Ile Val Leu Asn Glu
               170                 175                 180
   CTC AAC TGG ACA AGT GCC TTA GAT GAA GTT TTC AAA AAG AAT CGC GAG      628
   Leu Asn Trp Thr Ser Ala Leu Asp Glu Val Phe Lys Lys Asn Arg Glu
           185                 190                 195
   GAA GAC CCT TCA TTA TTG TGG CAG GTT TTT GGC AGT GCC ACT GGC CTA      676
   Glu Asp Pro Ser Leu Leu Trp Gln Val Phe Gly Ser Ala Thr Gly Leu
       200                 205                 210
   GCT CGA TAT TAT CCA GCT TCA CCA TGG GTT GAT AAT AGT AGA ACT CCA      724
   Ala Arg Tyr Tyr Pro Ala Ser Pro Trp Val Asp Asn Ser Arg Thr Pro
   215                 220                 225                 230
   AAT AAG ATT GAC CTT TAT GAT GTA CGC AGA AGA CCA TGG TAC ATC CAA      772
   Asn Lys Ile Asp Leu Tyr Asp Val Arg Arg Arg Pro Trp Tyr Ile Gln
                   235                 240                 245
   GGA GCT GCA TCT CCT AAA GAC ATG CTT ATT CTG GTG GAT GTG AGT GGA      820
   Gly Ala Ala Ser Pro Lys Asp Met Leu Ile Leu Val Asp Val Ser Gly
               250                 255                 260
   AGT GTT AGT GGA TTG ACA CTT AAA CTG ATC CGA ACA TCT GTC TCC GAA      868
   Ser Val Ser Gly Leu Thr Leu Lys Leu Ile Arg Thr Ser Val Ser Glu
           265                 270                 275
   ATG TTA GAA ACC CTC TCA GAT GAT GAT TTC GTG AAT GTA GCT TCA TTT         916
   Met Leu Glu Thr Leu Ser Asp Asp Asp Phe Val Asn Val Ala Ser Phe
       280                 285                 290
   AAC AGC AAT GCT CAG GAT GTA AGC TGT TTT CAG CAC CTT GTC CAA GCA         964
   Asn Ser Asn Ala Gln Asp Val Ser Cys Phe Gln His Leu Val Gln Ala
   295                 300                 305                 310
   AAT GTA AGA AAT AAA AAA GTG TTG AAA GAC GCG GTG AAT AAT ATC ACA     1012
   Asn Val Arg Asn Lys Lys Val Leu Lys Asp Ala Val Asn Asn Ile Thr
                   315                 320                 325
   GCC AAA GGA ATT ACA GAT TAT AAG AAG GGC TTT AGT TTT GCT TTT GAA     1060
   Ala Lys Gly Ile Thr Asp Tyr Lys Lys Gly Phe Ser Phe Ala Phe Glu
           330                 335                 340
   CAG CTG CTT AAT TAT AAT GTT TCC AGA GCA AAC TGC AAT AAG ATT ATT      1108
   Gln Leu Leu Asn Tyr Asn Val Ser Arg Ala Asn Cys Asn Lys Ile Ile
           345                 350                 355
   ATG CTA TTC ACG GAT GGA GGA GAA GAG AGA GCC CAG GAG ATA TTT AAC      1156
   Met Leu Phe Thr Asp Gly Gly Glu Glu Arg Ala Gln Glu Ile Phe Asn
       360                 365                 370
   AAA TAC AAT AAA GAT AAA AAA GTA CGT GTA TTC AGG TTT TCA GTT GGT      1204
   Lys Tyr Asn Lys Asp Lys Lys Val Arg Val Phe Arg Phe Ser Val Gly
   375                 380                 385                 390
   CAA CAC AAT TAT GAG AGA GGA CCT ATT CAG TGG ATG GCC TGT GAA AAC      1252
   Gln His Asn Tyr Glu Arg Gly Pro Ile Gln Trp Met Ala Cys Glu Asn
                   395                 400                 405
   AAA GGT TAT TAT TAT GAA ATT CCT TCC ATT GGT GCA ATA AGA ATC AAT      1300
   Lys Gly Tyr Tyr Tyr Glu Ile Pro Ser Ile Gly Ala Ile Arg Ile Asn
           410                 415                 420
   ACT CAG GAA TAT TTG GAT GTT TTG GGA AGA CCA ATG GTT TTA GCA GGA      1348
   Thr Gln Glu Tyr Leu Asp Val Leu Gly Arg Pro Met Val Leu Ala Gly
           425                 430                 435
   GAC AAA GCT AAG CAA GTC CAA TGG ACA AAT GTG TAC CTG GAT GCA TTG      1396
   Asp Lys Ala Lys Gln Val Gln Trp Thr Asn Val Tyr Leu Asp Ala Leu
       440                 445                 450
   GAA CTG GGA CTT GTC ATT ACT GGA ACT CTT CCG GTC TTC AAC ATA ACC      1444
   Glu Leu Gly Leu Val Ile Thr Gly Thr Leu Pro Val Phe Asn Ile Thr
   455                 460                 465                 470
   GGC CAA TTT GAA AAT AAG ACA AAC TTA AAG AAC CAG CTG ATT CTT GGT      1492
   Gly Gln Phe Glu Asn Lys Thr Asn Leu Lys Asn Gln Leu Ile Leu Gly
                   475                 480                 485
   GTG ATG GGA GTA GAT GTG TCT TTG GAA GAT ATT AAA AGA CTG ACA CCA      1540
   Val Met Gly Val Asp Val Ser Leu Glu Asp Ile Lys Arg Leu Thr Pro
           490                 495                 500
   CGT TTT ACA CTG TGC CCC AAT GGG TAT TAC TTT GCA ATC GAT CCT AAT      1588
   Arg Phe Thr Leu Cys Pro Asn Gly Tyr Tyr Phe Ala Ile Asp Pro Asn
           505                 510                 515
   GGT TAT GTT TTA TTA CAT CCA AAT CTT CAG CCA AAG AAC CCC AAA TCT      1636
   Gly Tyr Val Leu Leu His Pro Asn Leu Gln Pro Lys Asn Pro Lys Ser
       520                 525                 530
   CAG GAG CCA GTA ACA TTG GAT TTC CTT GAT GCA GAG TTA GAG AAT GAT      1684
   Gln Glu Pro Val Thr Leu Asp Phe Leu Asp Ala Glu Leu Glu Asn Asp
   535                 540                 545                 550
   ATT AAA GTG GAG ATT CGA AAT AAG ATG ATT GAT GGG GAA AGT GGA GAA      1732
   Ile Lys Val Glu Ile Arg Asn Lys Met Ile Asp Gly Glu Ser Gly Glu
                   555                 560                 565
   AAA ACA TTC AGA ACT CTG GTT AAA TCT CAA GAT GAG AGA TAT ATT GAC      1780
   Lys Thr Phe Arg Thr Leu Val Lys Ser Gln Asp Glu Arg Tyr Ile Asp
           570                 575                 580
   AAA GGA AAC AGG ACA TAC ACA TGG ACA CCT GTC AAT GGC ACA GAT TAC      1828
   Lys Gly Asn Arg Thr Tyr Thr Trp Thr Pro Val Asn Gly Thr Asp Tyr
           585                 590                 595
   AGT TTG GCC TTG GTA TTA CCA ACC TAC AGT TTT TAC TAT ATA AAA GCC      1876
   Ser Leu Ala Leu Val Leu Pro Thr Tyr Ser Phe Tyr Tyr Ile Lys Ala
       600                 605                 610
   AAA CTA GAA GAG ACA ATA ACT CAG GCC AGA TCA AAA AAG GGC AAA ATG      1924
   Lys Leu Glu Glu Thr Ile Thr Gln Ala Arg Ser Lys Lys Gly Lys Met
   615                 620                 625                 630
   AAG GAT TCG GAA ACC CTG AAG CCA GAT AAT TTT GAA GAA TCT GGC TAT      1972
   Lys Asp Ser Glu Thr Leu Lys Pro Asp Asn Phe Glu Glu Ser Gly Tyr
                   635                 640                 645
   ACA TTC ATA GCA CCA AGA GAT TAC TGC AAT GAC CTG AAA ATA TCG GAT      2020
   Thr Phe Ile Ala Pro Arg Asp Tyr Cys Asn Asp Leu Lys Ile Ser Asp
               650                 655                 660
   AAT AAC ACT GAA TTT CTT TTA AAT TTC AAC GAG TTT ATT GAT AGA AAA      2068
   Asn Asn Thr Glu Phe Leu Leu Asn Phe Asn Glu Phe Ile Asp Arg Lys
           665                 670                 675
   ACT CCA AAC AAC CCA TCA TGT AAC GCG GAT TTG ATT AAT AGA GTC TTG      2116
   Thr Pro Asn Asn Pro Ser Cys Asn Ala Asp Leu Ile Asn Arg Val Leu
       680                 685                 690
   CTT GAT GCA GGC TTT ACA AAT GAA CTT GTC CAA AAT TAC TGG AGT AAG      2164
   Leu Asp Ala Gly Phe Thr Asn Glu Leu Val Gln Asn Tyr Trp Ser Lys
   695                 700                 705                 710
   CAG AAA AAT ATC AAG GGA GTG AAA GCA CGA TTT GTT GTG ACT GAT GGT      2212
   Gln Lys Asn Ile Lys Gly Val Lys Ala Arg Phe Val Val Thr Asp Gly
                   715                 720                 725
   GGG ATT ACC AGA GTT TAT CCC AAA GAG GCT GGA GAA AAT TGG CAA GAA      2260
   Gly Ile Thr Arg Val Tyr Pro Lys Glu Ala Gly Glu Asn Trp Gln Glu
               730                 735                 740
   AAC CCA GAG ACA TAT GAG GAC AGC TTC TAT AAA AGG AGC CTA GAT AAT      2308
   Asn Pro Glu Thr Tyr Glu Asp Ser Phe Tyr Lys Arg Ser Leu Asp Asn
           745                 750                 755
   GAT AAC TAT GTT TTC ACT GCT CCC TAC TTT AAC AAA AGT GGA CCT GGT      2356
   Asp Asn Tyr Val Phe Thr Ala Pro Tyr Phe Asn Lys Ser Gly Pro Gly
       760                 765                 770
   GCC TAT GAA TCG GGC ATT ATG GTA AGC AAA GCT GTA GAA ATA TAT ATT      2404
   Ala Tyr Glu Ser Gly Ile Met Val Ser Lys Ala Val Glu Ile Tyr Ile
   775                 780                 785                 790
   CAA GGG AAA CTT CTT AAA CCT GCA GTT GTT GGA ATT AAA ATT GAT GTA      2452
   Gln Gly Lys Leu Leu Lys Pro Ala Val Val Gly Ile Lys Ile Asp Val
                   795                 800                 805
   AAT TCC TGG ATA GAG AAT TTC ACC AAA ACC TCA ATC AGA GAT CCG TGT      2500
   Asn Ser Trp Ile Glu Asn Phe Thr Lys Thr Ser Ile Arg Asp Pro Cys
               810                 815                 820
   GCT GGT CCA GTT TGT GAC TGC AAA AGA AAC AGT GAC GTA ATG GAT TGT      2548
   Ala Gly Pro Val Cys Asp Cys Lys Arg Asn Ser Asp Val Met Asp Cys
           825                 830                 835
   GTG ATT CTG GAT GAT GGT GGG TTT CTT CTG ATG GCA AAT CAT GAT GAT      2596
   Val Ile Leu Asp Asp Gly Gly Phe Leu Leu Met Ala Asn His Asp Asp
       840                 845                 850
   TAT ACT AAT CAG ATT GGA AGA TTT TTT GGA GAG ATT GAT CCC AGC TTG      2644
   Tyr Thr Asn Gln Ile Gly Arg Phe Phe Gly Glu Ile Asp Pro Ser Leu
   855                 860                 865                 870
   ATG AGA CAC CTG GTT AAT ATA TCA GTT TAT GCT TTT AAC AAA TCT TAT      2692
   Met Arg His Leu Val Asn Ile Ser Val Tyr Ala Phe Asn Lys Ser Tyr
                   875                 880                 885
   GAT TAT CAG TCA GTA TGT GAG CCC GGT GCT GCA CCA AAA CAA GGA GCA      2740
   Asp Tyr Gln Ser Val Cys Glu Pro Gly Ala Ala Pro Lys Gln Gly Ala
                 890                       895                       900
   GGA CAT CGC TCA GCA TAT GTG CCA TCA GTA GCA GAC ATA TTA CAA ATT      2788
   Gly His Arg Ser Ala Tyr Val Pro Ser Val Ala Asp Ile Leu Gln Ile
           905                     910                     915
   GGC TGG TGG GCC ACT GCT GCT GCC TGG TCT ATT CTA CAG CAG TTT CTC      2836
   Gly Trp Trp Ala Thr Ala Ala Ala Trp Ser Ile Leu Gln Gln Phe Leu
       920                     925                     930
   TTG AGT TTG ACC TTT CCA CGA CTC CTT GAG GCA GTT GAG ATG GAG GAT      2884
   Leu Ser Leu Thr Phe Pro Arg Leu Leu Glu Ala Val Glu Met Glu Asp
   935                     940                     945                 950
   GAT GAC TTC ACG GCC TCC CTG TCC AAG CAG AGC TGC ATT ACT GAA CAA      2932
   Asp Asp Phe Thr Ala Ser Leu Ser Lys Gln Ser Cys Ile Thr Glu Gln
                   955                     960                     965
   ACC CAG TAT TTC TTC GAT AAC GAC AGT AAA TCA TTC AGT GGT GTA TTA      2980
   Thr Gln Tyr Phe Phe Asp Asn Asp Ser Lys Ser Phe Ser Gly Val Leu
               970                     975                     980
   GAC TGT GGA AAC TGT TCC AGA ATC TTT CAT GGA GAA AAG CTT ATG AAC      3028
   Asp Cys Gly Asn Cys Ser Arg Ile Phe His Gly Glu Lys Leu Met Asn
           985                     990                     995
   ACC AAC TTA ATA TTC ATA ATG GTT GAG AGC AAA GGG ACA TGT CCA TGT      3076
   Thr Asn Leu Ile Phe Ile Met Val Glu Ser Lys Gly Thr Cys Pro Cys
       1000                    1005                    1010
   GAC ACA CGA CTG CTC ATA CAA GCG GAG CAG ACT TCT GAC GGT CCA AAT      3124
   Asp Thr Arg Leu Leu Ile Gln Ala Glu Gln Thr Ser Asp Gly Pro Asn
   1015                    1020                    1025                1030
   CCT TGT GAC ATG GTT AAG CAA CCT AGA TAC CGA AAA GGG CCT GAT GTC      3172
   Pro Cys Asp Met Val Lys Gln Pro Arg Tyr Arg Lys Gly Pro Asp Val
                   1035                    1040                    1045
   TGC TTT GAT AAC AAT GTC TTG GAG GAT TAT ACT GAC TGT GGT GGT GTT      3220
   Cys Phe Asp Asn Asn Val Leu Glu Asp Tyr Thr Asp Cys Gly Gly Val
               1050                    1055                    1060
   TCT GGA TTA AAT CCC TCC CTG TGG TAT ATC ATT GGA ATC CAG TTT CTA      3268
   Ser Gly Leu Asn Pro Ser Leu Trp Tyr Ile Ile Gly Ile Gln Phe Leu
           1065                    1070                    1075
   CTA CTT TGG CTG GTA TCT GGC AGC ACA CAC CGG CTG TTA TGACCTTCTA       3317
   Leu Leu Trp Leu Val Ser Gly Ser Thr His Arg Leu Leu
       1080                    1085                    1090
   AAAACCAAAT CTGCATAGTT AAACTCCAGA CCCTGCCAAA ACATGAGCCC TGCCCTCAAT    3377
   TACAGTAACG TAGGGTCAGC TATAAAATCA GACAAACATT AGCTGGGCCT GTTCCATGGC    3437
   ATAACACTAA GGCGCAGACT CCTAAGGCAC CCACTGGCTG CATGTCAGGG TGTCAGATCC    3497
   TTAAACGTGT GTGAATGCTG CATCATCTAT GTGTAACATC AAAGCAAAAT CCTATACGTG    3557
   TCCTCTATTG GAAAATTTGG GCGTTTGTTG TTGCATTGTT GGT                      3600

### (2) INFORMATION FOR SEQ ID NO:12:

(i) SEQUENCE CHARACTERISTICS:
   (A) LENGTH: 323 base pairs
   (B) TYPE: nucleic acid
   (C) STRANDEDNESS: double
   (D) TOPOLOGY: linear
(ii) MOLECULE TYPE: DNA (genomic)

### (2) INFORMATION FOR SEQ ID NO:13:

(i) SEQUENCE CHARACTERISTICS:
   (A) LENGTH: 57 base pairs
   (B) TYPE: nucleic acid
   (C) STRANDEDNESS: double
   (D) TOPOLOGY: linear
(ii) MOLECULE TYPE: DNA (genomic)
(xi) SEQUENCE DESCRIPTION: SEQ ID NO:13:
   CCTATTGGTG TAGGTATACC AACAATTAAT TTAAGAAAAA GGAGACCCAA TATCCAG     57

### (2) INFORMATION FOR SEQ ID NO:14:

(i) SEQUENCE CHARACTERISTICS:
   (A) LENGTH: 180 base pairs
   (B) TYPE: nucleic acid
   (C) STRANDEDNESS: double
   (D) TOPOLOGY: linear
(ii) MOLECULE TYPE: DNA (genomic)
(ix) FEATURE:
   (A) NAME/KEY: CDS
   (B) LOCATION: 1..132
(xi) SEQUENCE DESCRIPTION: SEQ ID NO:14:
   TGG TCC TTT GCC TGC GCC TGT GCC GCC TTC ATC CTC CTC TTT CTC GGC        48
   Trp Ser Phe Ala Cys Ala Cys Ala Ala Phe Ile Leu Leu Phe Leu Gly
     1               5                  10                  15
   GGT CTC GCC CTC CTG CTG TTC TCC CTG CCT CGA ATG CCC CGG AAC CCA        96
   Gly Leu Ala Leu Leu Leu Phe Ser Leu Pro Arg Met Pro Arg Asn Pro
                20                  25                  30
   TGG GAG TCC TGC ATG GAT GCT GAG CCC GAG CAC TAACCCTCCT GCGGCCCTAG     149
   Trp Glu Ser Cys Met Asp Ala Glu Pro Glu His
            35                  40
   CGACCCTCAG GCTTCTTCCC AGGAAGCGGG G                                   180

### (2) INFORMATION FOR SEQ ID NO:15:

(i) SEQUENCE CHARACTERISTICS:
   (A) LENGTH: 22 base pairs
   (B) TYPE: nucleic acid
   (C) STRANDEDNESS: single
   (D) TOPOLOGY: linear
(ii) MOLECULE TYPE: Other nucleic acid;
   (A) DESCRIPTION: Oligonucleotide
(xi) SEQUENCE DESCRIPTION: SEQ ID NO:15:
   AATTCGGTAC GTACACTCGA GC                                              22

### (2) INFORMATION FOR SEQ ID NO:16:

(i) SEQUENCE CHARACTERISTICS:
   (A) LENGTH: 18 base pairs
   (B) TYPE: nucleic acid
   (C) STRANDEDNESS: single
   (D) TOPOLOGY: linear
(ii) MOLECULE TYPE: Other nucleic acid;
   (A) DESCRIPTION: Oligonucleotide
(xi) SEQUENCE DESCRIPTION: SEQ ID NO:16:
   GCTCGAGTGT ACGTACCG                                                   18

### (2) INFORMATION FOR SEQ ID NO:17:

(i) SEQUENCE CHARACTERISTICS:
   (A) LENGTH: 20 base pairs
   (B) TYPE: nucleic acid
   (C) STRANDEDNESS: single
   (D) TOPOLOGY: linear
(ii) MOLECULE TYPE: Other nucleic acid;
   (A) DESCRIPTION: Oligonucleotide
(xi) SEQUENCE DESCRIPTION: SEQ ID NO:17:
   CCATGGTACC TTCGTTGACG 20

### (2) INFORMATION FOR SEQ ID NO:18:

(i) SEQUENCE CHARACTERISTICS:
   (A) LENGTH: 24 base pairs
   (B) TYPE: nucleic acid
   (C) STRANDEDNESS: single
   (D) TOPOLOGY: linear
(ii) MOLECULE TYPE: Other nucleic acid;
   (A) DESCRIPTION: Oligonucleotide
(xi) SEQUENCE DESCRIPTION: SEQ ID NO:18:
   AATTCGTCAA CGAAGGTACC ATGG 24

### (2) INFORMATION FOR SEQ ID NO:19:

(i) SEQUENCE CHARACTERISTICS:
   (A) LENGTH: 249 base pairs
   (B) TYPE: nucleic acid
   (C) STRANDEDNESS: double
   (D) TOPOLOGY: linear
(ii) MOLECULE TYPE: cDNA
(xi) SEQUENCE DESCRIPTION: SEQ ID NO:19:
   CGGTACGTAC ACTCGAGCGA CTGTGTCATG ATGTTTATTA GTGATGACAG TGAGGTGAGG    60
   CAGGGGCTTG TGGAGCATGC TCTGTAGGTC ACACACTAGA GCCATAAGGC AAGAGTAGGC   120
   GGGGAGACAG GTCCTCTGTG CCCTGTCTCT CCCCATCTAA CCCTAACCTA ACAAGCGGTA   180
   GTTATGAGTC AGGGAACAAC GTCTGGAGCC CCGTCCTCCA AAGATGTTTG AGGGACAAGA   240
   ACAGAAATG                                                           249

### (2) INFORMATION FOR SEQ ID NO:20:

(i) SEQUENCE CHARACTERISTICS:
   (A) LENGTH: 402 base pairs
   (B) TYPE: nucleic acid
   (C) STRANDEDNESS: double
   (D) TOPOLOGY: linear
(ii) MOLECULE TYPE: cDNA
(xi) SEQUENCE DESCRIPTION: SEQ ID NO:20:
   CCACCTAGCA CGGGTTCGTT CCCCTCTCCC GGCCTGGCCC GGGCTCCCCG GTGGCCGCCG    60
   CCCCCTCGCC GCCCATCTCT GGACTCAGAC GTCTCCCTGG AGGAGGACCG GGAGAGTGCC   120
   CGGCGTGAAG TAGAGAGCCA GGCTCAGCAG CAGCTCGAAA GGGCCAAGCA CAAACCTGTG   180
   GCATTTGCGG TGAGGACCAA TGTCAGCTAC TGTGGCGTAC TGGATGAGGA GTGCCCAGTC   240
   CAGGGCTCTG GAGTCAACTT TGAGGCCAAA GATTTTCTGC ACATTAAAGA GAAGTACAGC   300
   AATGACTGGT GGATCGGGCG GCTAGTGAAA GAGGGCGGGG ACATCGCCTT CATCCCCAGC   360
   CCCCAGTGCC TGGTGAGCAT CCGCTCAAAC AGGAGCAGAA GG                      402

### (2) INFORMATION FOR SEQ ID NO:21:

(i) SEQUENCE CHARACTERISTICS:
   (A) LENGTH: 28 base pairs
   (B) TYPE: nucleic acid
   (C) STRANDEDNESS: single
   (D) TOPOLOGY: linear
(ii) MOLECULE TYPE: Other nucleic acid;
   (A) DESCRIPTION: Oligonucleotide
(xi) SEQUENCE DESCRIPTION: SEQ ID NO:21:
   CTCAGTACCA TCTCTGATAC CAGCCCCA 28

### (2) INFORMATION FOR SEQ ID NO:22:

(i) SEQUENCE CHARACTERISTICS:
   (A) LENGTH: 7635 base pairs
   (B) TYPE: nucleic acid
   (C) STRANDEDNESS: double
   (D) TOPOLOGY: linear
(ii) MOLECULE TYPE: Genomic DNA
(iii) HYPOTHETICAL: NO
(iv) ANTISENSE: NO
(v) FRAGMENT TYPE:
(vi) ORIGINAL SOURCE:
(ix) FEATURE:
   (A) NAME/KEY: Coding Sequence
   (B) LOCATION: 511...6996
   (D) OTHER INFORMATION:

   (A) NAME/KEY: 5'UTR
   (B) LOCATION: 1...510
   (D) OTHER INFORMATION:

   (A) NAME/KEY: 3'UTR
   (B) LOCATION: 6994...7635
   (D) OTHER INFORMATION:
(xi) SEQUENCE DESCRIPTION: SEQ ID NO:22:
   GGGCGAGCGC CTCCGTCCCC GGATGTGAGC TCCGGCTGCC CGCGGTCCCG AGCCAGCGGC     60
   GCGCGGGCGG CGGCGGCGGG CACCGGGCAC CGCGGCGGGC GGGCAGACGG GCGGGCATGG    120
   GGGGAGCGCC GAGCGGCCCC GGCGGCCGGG CCGGCATCAC CGCGGCGTCT CTCCGCTAGA    180
   GGAGGGGACA AGCCAGTTCT CCTTTGCAGC AAAAAATTAC ATGTATATAT TATTAAGATA    240
   ATATATACAT TGGATTTTAT TTTTTTAAAA AGTTTATTTT GCTCCATTTT TGAAAAAGAG    300
   AGAGCTTGGG TGGCGAGCGG TTTTTTTTTA AAATCAATTA TCCTTATTTT CTGTTATTTG    360
   TCCCCGTCCC TCCCCACCCC CCTGCTGAAG CGAGAATAAG GGCAGGGACC GCGGCTCCTA    420
   CCTCTTGGTG ATCCCCTTCC CCATTCCGCC CCCGCCCCAA CGCCCAGCAC AGTGCCCTGC    480
   ACACAGTAGT CGCTCAATAA ATGTTCGTGG ATG ATG ATG ATG ATG ATG ATG AAA     534
                                    Met Met Met Met Met Met Met Lys
                                    1               5
   AAA ATG CAG CAT CAA CGG CAG CAG CAA GCG GAC CAC GCG AAC GAG GCA     582
   Lys Met Gln His Gln Arg Gln Gln Gln Ala Asp His Ala Asn Glu Ala
       10                  15                  20
   AAC TAT GCA AGA GGC ACC AGA CTT CCT CTT TCT GGT GAA GGA CCA ACT     630
   Asn Tyr Ala Arg Gly Thr Arg Leu Pro Leu Ser Gly Glu Gly Pro Thr
   25                  30                  35                  40
   TCT CAG CCG AAT AGC TCC AAG CAA ACT GTC CTG TCT TGG CAA GCT GCA     678
   Ser Gln Pro Asn Ser Ser Lys Gln Thr Val Leu Ser Trp Gln Ala Ala
                   45                  50                  55
   ATC GAT GCT GCT AGA CAG GCC AAG GCT GCC CAA ACT ATG AGC ACC TCT     726
   Ile Asp Ala Ala Arg Gln Ala Lys Ala Ala Gln Thr Met Ser Thr Ser
               60                  65                  70
   GCA CCC CCA CCT GTA GGA TCT CTC TCC CAA AGA AAA CGT CAG CAA TAC     774
   Ala Pro Pro Pro Val Gly Ser Leu Ser Gln Arg Lys Arg Gln Gln Tyr
           75                  80                  85
   GCC AAG AGC AAA AAA CAG GGT AAC TCG TCC AAC AGC CGA CCT GCC CGC     822
   Ala Lys Ser Lys Lys Gln Gly Asn Ser Ser Asn Ser Arg Pro Ala Arg
       90                  95                  100
   GCC CTT TTC TGT TTA TCA CTC AAT AAC CCC ATC CGA AGA GCC TGC ATT     870
   Ala Leu Phe Cys Leu Ser Leu Asn Asn Pro Ile Arg Arg Ala Cys Ile
   105                 110                 115                 120
   AGT ATA GTG GAA TGG AAA CCA TTT GAC ATA TTT ATA TTA TTG GCT ATT     918
   Ser Ile Val Glu Trp Lys Pro Phe Asp Ile Phe Ile Leu Leu Ala Ile
                   125                 130                 135
   TTT GCC AAT TGT GTG GCC TTA GCT ATT TAC ATC CCA TTC CCT GAA GAT     966
   Phe Ala Asn Cys Val Ala Leu Ala Ile Tyr Ile Pro Phe Pro Glu Asp
               140                 145                 150
   GAT TCT AAT TCA ACA AAT CAT AAC TTG GAA AAA GTA GAA TAT GCC TTC    1014
   Asp Ser Asn Ser Thr Asn His Asn Leu Glu Lys Val Glu Tyr Ala Phe
           155                 160                 165
   CTG ATT ATT TTT ACA GTC GAG ACA TTT TTG AAG ATT ATA GCG TAT GGA      1062
   Leu Ile Ile Phe Thr Val Glu Thr Phe Leu Lys Ile Ile Ala Tyr Gly
       170                 175                 180
   TTA TTG CTA CAT CCT AAT GCT TAT GTT AGG AAT GGA TGG AAT TTA CTG      1110
   Leu Leu Leu His Pro Asn Ala Tyr Val Arg Asn Gly Trp Asn Leu Leu
   185                 190                 195                 200
   GAT TTT GTT ATA GTA ATA GTA GGA TTG TTT AGT GTA ATT TTG GAA CAA      1158
   Asp Phe Val Ile Val Ile Val Gly Leu Phe Ser Val Ile Leu Glu Gln
                   205                 210                 215
   TTA ACC AAA GAA ACA GAA GGC GGG AAC CAC TCA AGC GGC AAA TCT GGA      1206
   Leu Thr Lys Glu Thr Glu Gly Gly Asn His Ser Ser Gly Lys Ser Gly
               220                 225                 230
   GGC TTT GAT GTC AAA GCC CTC CGT GCC TTT CGA GTG TTG CGA CCA CTT      1254
   Gly Phe Asp Val Lys Ala Leu Arg Ala Phe Arg Val Leu Arg Pro Leu
           235                 240                 245
   CGA CTA GTG TCA GGA GTG CCC AGT TTA CAA GTT GTC CTG AAC TCC ATT      1302
   Arg Leu Val Ser Gly Val Pro Ser Leu Gln Val Val Leu Asn Ser Ile
       250                 255                 260
   ATA AAA GCC ATG GTT CCC CTC CTT CAC ATA GCC CTT TTG GTA TTA TTT      1350
   Ile Lys Ala Met Val Pro Leu Leu His Ile Ala Leu Leu Val Leu Phe
   265                 270                 275                 280
   GTA ATC ATA ATC TAT GCT ATT ATA GGA TTG GAA CTT TTT ATT GGA AAA      1398
   Val Ile Ile Ile Tyr Ala Ile Ile Gly Leu Glu Leu Phe Ile Gly Lys
                   285                 290                 295
   ATG CAC AAA ACA TGT TTT TTT GCT GAC TCA GAT ATC GTA GCT GAA GAG      1446
   Met His Lys Thr Cys Phe Phe Ala Asp Ser Asp Ile Val Ala Glu Glu
               300                 305                 310
   GAC CCA GCT CCA TGT GCG TTC TCA GGG AAT GGA CGC CAG TGT ACT GCC      1494
   Asp Pro Ala Pro Cys Ala Phe Ser Gly Asn Gly Arg Gln Cys Thr Ala
           315                 320                 325
   AAT GGC ACG GAA TGT AGG AGT GGC TGG GTT GGC CCG AAC GGA GGC ATC      1542
   Asn Gly Thr Glu Cys Arg Ser Gly Trp Val Gly Pro Asn Gly Gly Ile
       330                 335                 340
   ACC AAC TTT GAT AAC TTT GCC TTT GCC ATG CTT ACT GTG TTT CAG TGC      1590
   Thr Asn Phe Asp Asn Phe Ala Phe Ala Met Leu Thr Val Phe Gln Cys
   345                 350                 355                 360
   ATC ACC ATG GAG GGC TGG ACA GAC GTG CTC TAC TGG GTA AAT GAT GCG      1638
   Ile Thr Met Glu Gly Trp Thr Asp Val Leu Tyr Trp Val Asn Asp Ala
                   365                 370                 375
   ATA GGA TGG GAA TGG CCA TGG GTG TAT TTT GTT AGT CTG ATC ATC CTT      1686
   Ile Gly Trp Glu Trp Pro Trp Val Tyr Phe Val Ser Leu Ile Ile Leu
               380                 385                 390
   GGC TCA TTT TTC GTC CTT AAC CTG GTT CTT GGT GTC CTT AGT GGA GAA      1734
   Gly Ser Phe Phe Val Leu Asn Leu Val Leu Gly Val Leu Ser Gly Glu
           395                 400                 405
   TTC TCA AAG GAA AGA GAG AAG GCA AAA GCA CGG GGA GAT TTC CAG AAG      1782
   Phe Ser Lys Glu Arg Glu Lys Ala Lys Ala Arg Gly Asp Phe Gln Lys
       410                 415                 420
   CTC CGG GAG AAG CAG CAG CTG GAG GAG GAT CTA AAG GGC TAC TTG GAT      1830
   Leu Arg Glu Lys Gln Gln Leu Glu Glu Asp Leu Lys Gly Tyr Leu Asp
   425                 430                 435                 440
   TGG ATC ACC CAA GCT GAG GAC ATC GAT CCG GAG AAT GAG GAA GAA GGA     1878
   Trp Ile Thr Gln Ala Glu Asp Ile Asp Pro Glu Asn Glu Glu Glu Gly
                   445                 450                 455
   GGA GAG GAA GGC AAA CGA AAT ACT AGC ATG CCC ACC AGC GAG ACT GAG     1926
   Gly Glu Glu Gly Lys Arg Asn Thr Ser Met Pro Thr Ser Glu Thr Glu
               460                 465                 470
   TCT GTG AAC ACA GAG AAC GTC AGC GGT GAA GGC GAG AAC CGA GGC TGC     1974
   Ser Val Asn Thr Glu Asn Val Ser Gly Glu Gly Glu Asn Arg Gly Cys
           475                 480                 485
   TGT GGA AGT CTC TGT CAA GCC ATC TCA AAA TCC AAA CTC AGC CGA CGC     2022
   Cys Gly Ser Leu Cys Gln Ala Ile Ser Lys Ser Lys Leu Ser Arg Arg
       490                 495                 500
   TGG CGT CGC TGG AAC CGA TTC AAT CGC AGA AGA TGT AGG GCC GCC GTG     2070
   Trp Arg Arg Trp Asn Arg Phe Asn Arg Arg Arg Cys Arg Ala Ala Val
   505                 510                 515                 520
   AAG TCT GTC ACG TTT TAC TGG CTG GTT ATC GTC CTG GTG TTT CTG AAC     2118
   Lys Ser Val Thr Phe Tyr Trp Leu Val Ile Val Leu Val Phe Leu Asn
                   525                 530                 535
   ACC TTA ACC ATT TCC TCT GAG CAC TAC AAT CAG CCA GAT TGG TTG ACA     2166
   Thr Leu Thr Ile Ser Ser Glu His Tyr Asn Gln Pro Asp Trp Leu Thr
               540                 545                 550
   CAG ATT CAA GAT ATT GCC AAC AAA GTC CTC TTG GCT CTG TTC ACC TGC     2214
   Gln Ile Gln Asp Ile Ala Asn Lys Val Leu Leu Ala Leu Phe Thr Cys
           555                 560                 565
   GAG ATG CTG GTA AAA ATG TAC AGC TTG GGC CTC CAA GCA TAT TTC GTC     2262
   Glu Met Leu Val Lys Met Tyr Ser Leu Gly Leu Gln Ala Tyr Phe Val
       570                 575                 580
   TCT CTT TTC AAC CGG TTT GAT TGC TTC GTG GTG TGT GGT GGA ATC ACT     2310
   Ser Leu Phe Asn Arg Phe Asp Cys Phe Val Val Cys Gly Gly Ile Thr
   585                 590                 595                 600
   GAG ACG ATC TTG GTG GAA CTG GAA ATC ATG TCT CCC CTG GGG ATC TCT     2358
   Glu Thr Ile Leu Val Glu Leu Glu Ile Met Ser Pro Leu Gly Ile Ser
                   605                 610                 615
   GTG TTT CGG TGT GTG CGC CTC TTA AGA ATC TTC AAA GTG ACC AGG CAC     2406
   Val Phe Arg Cys Val Arg Leu Leu Arg Ile Phe Lys Val Thr Arg His
               620                 625                 630
   TGG ACT TCC CTG AGC AAC TTA GTG GCA TCC TTA TTA AAC TCC ATG AAG     2454
   Trp Thr Ser Leu Ser Asn Leu Val Ala Ser Leu Leu Asn Ser Met Lys
           635                 640                 645
   TCC ATC GCT TCG CTG TTG CTT CTG CTT TTT CTC TTC ATT ATC ATC TTT     2502
   Ser Ile Ala Ser Leu Leu Leu Leu Leu Phe Leu Phe Ile Ile Ile Phe
       650                 655                 660
   TCC TTG CTT GGG ATG CAG CTG TTT GGC GGC AAG TTT AAT TTT GAT GAA     2550
   Ser Leu Leu Gly Met Gln Leu Phe Gly Gly Lys Phe Asn Phe Asp Glu
   665                 670                 675                 680
   ACG CAA ACC AAG CGG AGC ACC TTT GAC AAT TTC CCT CAA GCA CTT CTC     2598
   Thr Gln Thr Lys Arg Ser Thr Phe Asp Asn Phe Pro Gln Ala Leu Leu
                   685                 690                 695
   ACA GTG TTC CAG ATC CTG ACA GGC GAA GAC TGG AAT GCT GTG ATG TAC     2646
   Thr Val Phe Gln Ile Leu Thr Gly Glu Asp Trp Asn Ala Val Met Tyr
               700                 705                 710
   GAT GGC ATC ATG GCT TAC GGG GGC CCA TCC TCT TCA GGA ATG ATC GTC     2694
   Asp Gly Ile Met Ala Tyr Gly Gly Pro Ser Ser Ser Gly Met Ile Val
           715                     720                     725
   TGC ATC TAC TTC ATC ATC CTC TTC ATT TGT GGT AAC TAT ATT CTA CTG     2742
   Cys Ile Tyr Phe Ile Ile Leu Phe Ile Cys Gly Asn Tyr Ile Leu Leu
       730                     735                     740
   AAT GTC TTC TTG GCC ATC GCT GTA GAC AAT TTG GCT GAT GCT GAA AGT     2790
   Asn Val Phe Leu Ala Ile Ala Val Asp Asn Leu Ala Asp Ala Glu Ser
   745                 750                 755                 760
   CTG AAC ACT GCT CAG AAA GAA GAA GCG GAA GAA AAG GAG AGG AAA AAG     2838
   Leu Asn Thr Ala Gln Lys Glu Glu Ala Glu Glu Lys Glu Arg Lys Lys
                   765                     770                 775
   ATT GCC AGA AAA GAG AGC CTA GAA AAT AAA AAG AAC AAC AAA CCA GAA     2886
   Ile Ala Arg Lys Glu Ser Leu Glu Asn Lys Lys Asn Asn Lys Pro Glu
               780                 785                 790
   GTC AAC CAG ATA GCC AAC AGT GAC AAC AAG GTT ACA ATT GAT GAC TAT     2934
   Val Asn Gln Ile Ala Asn Ser Asp Asn Lys Val Thr Ile Asp Asp Tyr
           795                 800                 805
   AGA GAA GAG GAT GAA GAC AAG GAC CCC TAT CCG CCT TGC GAT GTG CCA     2982
   Arg Glu Glu Asp Glu Asp Lys Asp Pro Tyr Pro Pro Cys Asp Val Pro
       810                 815                 820
   GTA GGG GAA GAG GAA GAG GAA GAG GAG GAG GAT GAA CCT GAG GTT CCT     3030
   Val Gly Glu Glu Glu Glu Glu Glu Glu Glu Asp Glu Pro Glu Val Pro
   825                 830                 835                 840
   GCC GGA CCC CGT CCT CGA AGG ATC TCG GAG TTG AAC ATG AAG GAA AAA     3078
   Ala Gly Pro Arg Pro Arg Arg Ile Ser Glu Leu Asn Met Lys Glu Lys
                   845                 850                 855
   ATT GCC CCC ATC CCT GAA GGG AGC GCT TTC TTC ATT CTT AGC AAG ACC     3126
   Ile Ala Pro Ile Pro Glu Gly Ser Ala Phe Phe Ile Leu Ser Lys Thr
               860                 865                 870
   AAC CCG ATC CGC GTA GGC TGC CAC AAG CTC ATC AAC CAC CAC ATC TTC     3174
   Asn Pro Ile Arg Val Gly Cys His Lys Leu Ile Asn His His Ile Phe
           875                 880                 885
   ACC AAC CTC ATC CTT GTC TTC ATC ATG CTG AGC AGT GCT GCC CTG GCC     3222
   Thr Asn Leu Ile Leu Val Phe Ile Met Leu Ser Ser Ala Ala Leu Ala
       890                 895                 900
   GCA GAG GAC CCC ATC CGC AGC CAC TCC TTC CGG AAC ACG ATA CTG GGT     3270
   Ala Glu Asp Pro Ile Arg Ser His Ser Phe Arg Asn Thr Ile Leu Gly
   905                 910                 915                 920
   TAC TTT GAC TAT GCC TTC ACA GCC ATC TTT ACT GTT GAG ATC CTG TTG     3318
   Tyr Phe Asp Tyr Ala Phe Thr Ala Ile Phe Thr Val Glu Ile Leu Leu
                   925                 930                 935
   AAG ATG ACA ACT TTT GGA GCT TTC CTC CAC AAA GGG GCC TTC TGC AGG     3366
   Lys Met Thr Thr Phe Gly Ala Phe Leu His Lys Gly Ala Phe Cys Arg
               940                 945                 950
   AAC TAC TTC AAT TTG CTG GAT ATG CTG GTG GTT GGG GTG TCT CTG GTG     3414
   Asn Tyr Phe Asn Leu Leu Asp Met Leu Val Val Gly Val Ser Leu Val
           955                 960                 965
   TCA TTT GGG ATT CAA TCC AGT GCC ATC TCC GTT GTG AAG ATT CTG AGG     3462
   Ser Phe Gly Ile Gln Ser Ser Ala Ile Ser Val Val Lys Ile Leu Arg
       970                 975                 980
   GTC TTA AGG GTC CTG CGT CCC CTC AGG GCC ATC AAC AGA GCA AAA GGA     3510
   Val Leu Arg Val Leu Arg Pro Leu Arg Ala Ile Asn Arg Ala Lys Gly
   985                 990                 995                 1000
   CTT AAG CAC GTG GTC CAG TGC GTC TTC GTG GCC ATC CGG ACC ATC GGC     3558
   Leu Lys His Val Val Gln Cys Val Phe Val Ala Ile Arg Thr Ile Gly
                   1005                1010                1015
   AAC ATC ATG ATC GTC ACC ACC CTC CTG CAG TTC ATG TTT GCC TGT ATC     3606
   Asn Ile Met Ile Val Thr Thr Leu Leu Gln Phe Met Phe Ala Cys Ile
               1020                1025                1030
   GGG GTC CAG TTG TTC AAG GGG AAG TTC TAT CGC TGT ACG GAT GAA GCC     3654
   Gly Val Gln Leu Phe Lys Gly Lys Phe Tyr Arg Cys Thr Asp Glu Ala
           1035                1040                1045
   AAA AGT AAC CCT GAA GAA TGC AGG GGA CTT TTC ATC CTC TAC AAG GAT     3702
   Lys Ser Asn Pro Glu Glu Cys Arg Gly Leu Phe Ile Leu Tyr Lys Asp
       1050                1055                1060
   GGG GAT GTT GAC AGT CCT GTG GTC CGT GAA CGG ATC TGG CAA AAC AGT     3750
   Gly Asp Val Asp Ser Pro Val Val Arg Glu Arg Ile Trp Gln Asn Ser
   1065                1070                1075                1080
   GAT TTC AAC TTC GAC AAC GTC CTC TCT GCT ATG ATG GCG CTC TTC ACA     3798
   Asp Phe Asn Phe Asp Asn Val Leu Ser Ala Met Met Ala Leu Phe Thr
                   1085                1090                1095
   GTC TCC ACG TTT GAG GGC TGG CCT GCG TTG CTG TAT AAA GCC ATC GAC     3846
   Val Ser Thr Phe Glu Gly Trp Pro Ala Leu Leu Tyr Lys Ala Ile Asp
               1100                1105                1110
   TCG AAT GGA GAG AAC ATC GGC CCA ATC TAC AAC CAC CGC GTG GAG ATC     3894
   Ser Asn Gly Glu Asn Ile Gly Pro Ile Tyr Asn His Arg Val Glu Ile
           1115                1120                1125
   TCC ATC TTC TTC ATC ATC TAC ATC ATC ATT GTA GCT TTC TTC ATG ATG     3942
   Ser Ile Phe Phe Ile Ile Tyr Ile Ile Ile Val Ala Phe Phe Met Met
       1130                1135                1140
   AAC ATC TTT GTG GGC TTT GTC ATC GTT ACA TTT CAG GAA CAA GGA GAA     3990
   Asn Ile Phe Val Gly Phe Val Ile Val Thr Phe Gln Glu Gln Gly Glu
   1145                1150                1155                1160
   AAA GAG TAT AAG AAC TGT GAG CTG GAC AAA AAT CAG CGT CAG TGT GTT     4038
   Lys Glu Tyr Lys Asn Cys Glu Leu Asp Lys Asn Gln Arg Gln Cys Val
                   1165                1170                1175
   GAA TAC GCC TTG AAA GCA CGT CCC TTG CGG AGA TAC ATC CCC AAA AAC     4086
   Glu Tyr Ala Leu Lys Ala Arg Pro Leu Arg Arg Tyr Ile Pro Lys Asn
               1180                1185                1190
   CCC TAC CAG TAC AAG TTC TGG TAC GTG GTG AAC TCT TCG CCT TTC GAA     4134
   Pro Tyr Gln Tyr Lys Phe Trp Tyr Val Val Asn Ser Ser Pro Phe Glu
           1195                1200                1205
   TAC ATG ATG TTT GTC CTC ATC ATG CTC AAC ACA CTC TGC TTG GCC ATG     4182
   Tyr Met Met Phe Val Leu Ile Met Leu Asn Thr Leu Cys Leu Ala Met
       1210                1215                1220
   CAG CAC TAC GAG CAG TCC AAG ATG TTC AAT GAT GCC ATG GAC ATT CTG     4230
   Gln His Tyr Glu Gln Ser Lys Met Phe Asn Asp Ala Met Asp Ile Leu
   1225                1230                1235                1240
   AAC ATG GTC TTC ACC GGG GTG TTC ACC GTC GAG ATG GTT TTG AAA GTC     4278
   Asn Met Val Phe Thr Gly Val Phe Thr Val Glu Met Val Leu Lys Val
                   1245                1250                1255
   ATC GCA TTT AAG CCT AAG GGG TAT TTT AGT GAC GCC TGG AAC ACG TTT     4326
   Ile Ala Phe Lys Pro Lys Gly Tyr Phe Ser Asp Ala Trp Asn Thr Phe
               1260                1265                1270
   GAC TCC CTC ATC GTA ATC GGC AGC ATT ATA GAC GTG GCC CTC AGC GAA     4374
   Asp Ser Leu Ile Val Ile Gly Ser Ile Ile Asp Val Ala Leu Ser Glu
           1275                1280                1285
   GCA GAC CCA ACT GAA AGT GAA AAT GTC CCT GTC CCA ACT GCT ACA CCT     4422
   Ala Asp Pro Thr Glu Ser Glu Asn Val Pro Val Pro Thr Ala Thr Pro
       1290                1295                1300
   GGG AAC TCT GAA GAG AGC AAT AGA ATC TCC ATC ACC TTT TTC CGT CTT     4470
   Gly Asn Ser Glu Glu Ser Asn Arg Ile Ser Ile Thr Phe Phe Arg Leu
   1305                1310                1315                1320
   TTC CGA GTG ATG CGA TTG GTG AAG CTT CTC AGC AGG GGG GAA GGC ATC     4518
   Phe Arg Val Met Arg Leu Val Lys Leu Leu Ser Arg Gly Glu Gly Ile
                   1325                1330                1335
   CGG ACA TTG CTG TGG ACT TTT ATT AAG TTC TTT CAG GCG CTC CCG TAT     4566
   Arg Thr Leu Leu Trp Thr Phe Ile Lys Phe Phe Gln Ala Leu Pro Tyr
               1340                1345                1350
   GTG GCC CTC CTC ATA GCC ATG CTG TTC TTC ATC TAT GCG GTC ATT GGC     4614
   Val Ala Leu Leu Ile Ala Met Leu Phe Phe Ile Tyr Ala Val Ile Gly
           1355                1360                1365
   ATG CAG ATG TTT GGG AAA GTT GCC ATG AGA GAT AAC AAC CAG ATC AAT     4662
   Met Gln Met Phe Gly Lys Val Ala Met Arg Asp Asn Asn Gln Ile Asn
       1370                1375                1380
   AGG AAC AAT AAC TTC CAG ACG TTT CCC CAG GCG GTG CTG CTG CTC TTC     4710
   Arg Asn Asn Asn Phe Gln Thr Phe Pro Gln Ala Val Leu Leu Leu Phe
   1385                1390                1395                1400
   AGG TGT GCA ACA GGT GAG GCC TGG CAG GAG ATC ATG CTG GCC TGT CTC     4758
   Arg Cys Ala Thr Gly Glu Ala Trp Gln Glu Ile Met Leu Ala Cys Leu
                   1405                1410                1415
   CCA GGG AAG CTC TGT GAC CCT GAG TCA GAT TAC AAC CCC GGG GAG GAG     4806
   Pro Gly Lys Leu Cys Asp Pro Glu Ser Asp Tyr Asn Pro Gly Glu Glu
               1420                1425                1430
   CAT ACA TGT GGG AGC AAC TTT GCC ATT GTC TAT TTC ATC AGT TTT TAC     4854
   His Thr Cys Gly Ser Asn Phe Ala Ile Val Tyr Phe Ile Ser Phe Tyr
           1435                1440                1445
   ATG CTC TGT GCA TTT CTG ATC ATC AAT CTG TTT GTG GCT GTC ATC ATG     4902
   Met Leu Cys Ala Phe Leu Ile Ile Asn Leu Phe Val Ala Val Ile Met
       1450                1455                1460
   GAT AAT TTC GAC TAT CTG ACC CGG GAC TGG TCT ATT TTG GGG CCT CAC     4950
   Asp Asn Phe Asp Tyr Leu Thr Arg Asp Trp Ser Ile Leu Gly Pro His
   1465                1470                1475                1480
   CAT TTA GAT GAA TTC AAA AGA ATA TGG TCA GAA TAT GAC CCT GAG GCA     4998
   His Leu Asp Glu Phe Lys Arg Ile Trp Ser Glu Tyr Asp Pro Glu Ala
                   1485                1490                1495
   AAG GGA AGG ATA AAA CAC CTT GAT GTG GTC ACT CTG CTT CGA CGC ATC     5046
   Lys Gly Arg Ile Lys His Leu Asp Val Val Thr Leu Leu Arg Arg Ile
               1500                1505                1510
   CAG CCT CCC CTG GGG TTT GGG AAG TTA TGT CCA CAC AGG GTA GCG TGC     5094
   Gln Pro Pro Leu Gly Phe Gly Lys Leu Cys Pro His Arg Val Ala Cys
           1515                1520                1525
   AAG AGA TTA GTT GCC ATG AAC ATG CCT CTC AAC AGT GAC GGG ACA GTC     5142
   Lys Arg Leu Val Ala Met Asn Met Pro Leu Asn Ser Asp Gly Thr Val
       1530                1535                1540
   ATG TTT AAT GCA ACC CTG TTT GCT TTG GTT CGA ACG GCT CTT AAG ATC     5190
   Met Phe Asn Ala Thr Leu Phe Ala Leu Val Arg Thr Ala Leu Lys Ile
   1545                1550                1555                1560
   AAG ACC GAA GGG AAC CTG GAG CAA GCT AAT GAA GAA CTT CGG GCT GTG     5238
   Lys Thr Glu Gly Asn Leu Glu Gln Ala Asn Glu Glu Leu Arg Ala Val
                   1565                1570                1575
   ATA AAG AAA ATT TGG AAG AAA ACC AGC ATG AAA TTA CTT GAC CAA GTT     5286
   Ile Lys Lys Ile Trp Lys Lys Thr Ser Met Lys Leu Leu Asp Gln Val
               1580                1585                1590
   GTC CCT CCA GCT GGT GAT GAT GAG GTA ACC GTG GGG AAG TTC TAT GCC     5334
   Val Pro Pro Ala Gly Asp Asp Glu Val Thr Val Gly Lys Phe Tyr Ala
           1595                1600                1605
   ACT TTC CTG ATA CAG GAC TAC TTT AGG AAA TTC AAG AAA CGG AAA GAA     5382
   Thr Phe Leu Ile Gln Asp Tyr Phe Arg Lys Phe Lys Lys Arg Lys Glu
       1610                1615                1620
   CAA GGA CTG GTG GGA AAG TAC CCT GCG AAG AAC ACC ACA ATT GCC CTA     5430
   Gln Gly Leu Val Gly Lys Tyr Pro Ala Lys Asn Thr Thr Ile Ala Leu
   1625                1630                1635                1640
   CAG GCG GGA TTA AGG ACA CTG CAT GAC ATT GGG CCA GAA ATC CGG CGT     5478
   Gln Ala Gly Leu Arg Thr Leu His Asp Ile Gly Pro Glu Ile Arg Arg
                   1645                1650                1655
   GCT ATA TCG TGT GAT TTG CAA GAT GAC GAG CCT GAG GAA ACA AAA CGA     5526
   Ala Ile Ser Cys Asp Leu Gln Asp Asp Glu Pro Glu Glu Thr Lys Arg
               1660                1665                1670
   GAA GAA GAA GAT GAT GTG TTC AAA AGA AAT GGT GCC CTG CTT GGA AAC     5574
   Glu Glu Glu Asp Asp Val Phe Lys Arg Asn Gly Ala Leu Leu Gly Asn
           1675                1680                1685
   CAT GTC AAT CAT GTT AAT AGT GAT AGG AGA GAT TCC CTT CAG CAG ACC     5622
   His Val Asn His Val Asn Ser Asp Arg Arg Asp Ser Leu Gln Gln Thr
       1690                1695                1700
   AAT ACC ACC CAC CGT CCC CTG CAT GTC CAA AGG CCT TCA ATT CCA CCT     5670
   Asn Thr Thr His Arg Pro Leu His Val Gln Arg Pro Ser Ile Pro Pro
   1705                1710                1715                1720
   GCA AGT GAT ACT GAG AAA CCG CTG TTT CCT CCA GCA GGA AAT TCG GTG     5718
   Ala Ser Asp Thr Glu Lys Pro Leu Phe Pro Pro Ala Gly Asn Ser Val
                   1725                1730                1735
   TGT CAT AAC CAT CAT AAC CAT AAT TCC ATA GGA AAG CAA GTT CCC ACC     5766
   Cys His Asn His His Asn His Asn Ser Ile Gly Lys Gln Val Pro Thr
               1740                1745                1750
   TCA ACA AAT GCC AAT CTC AAT AAT GCC AAT ATG TCC AAA GCT GCC CAT     5814
   Ser Thr Asn Ala Asn Leu Asn Asn Ala Asn Met Ser Lys Ala Ala His
           1755                1760                1765
   GGA AAG CGG CCC AGC ATT GGG AAC CTT GAG CAT GTG TCT GAA AAT GGG     5862
   Gly Lys Arg Pro Ser Ile Gly Asn Leu Glu His Val Ser Glu Asn Gly
       1770                1775                1780
   CAT CAT TCT TCC CAC AAG CAT GAC CGG GAG CCT CAG AGA AGG TCC AGT     5910
   His His Ser Ser His Lys His Asp Arg Glu Pro Gln Arg Arg Ser Ser
   1785                1790                1795                1800
   GTG AAA AGA ACC CGC TAT TAT GAA ACT TAC ATT AGG TCC GAC TCA GGA     5958
   Val Lys Arg Thr Arg Tyr Tyr Glu Thr Tyr Ile Arg Ser Asp Ser Gly
                   1805                1810                1815
   GAT GAA CAG CTC CCA ACT ATT TGC CGG GAA GAC CCA GAG ATA CAT GGC     6006
   Asp Glu Gln Leu Pro Thr Ile Cys Arg Glu Asp Pro Glu Ile His Gly
               1820                1825                1830
   TAT TTC AGG GAC CCC CAC TGC TTG GGG GAG CAG GAG TAT TTC AGT AGT     6054
   Tyr Phe Arg Asp Pro His Cys Leu Gly Glu Gln Glu Tyr Phe Ser Ser
           1835                1840                1845
   GAG GAA TGC TAC GAG GAT GAC AGC TCG CCC ACC TGG AGC AGG CAA AAC     6102
   Glu Glu Cys Tyr Glu Asp Asp Ser Ser Pro Thr Trp Ser Arg Gln Asn
       1850                1855                1860
   TAT GGC TAC TAC AGC AGA TAC CCA GGC AGA AAC ATC GAC TCT GAG AGG     6150
   Tyr Gly Tyr Tyr Ser Arg Tyr Pro Gly Arg Asn Ile Asp Ser Glu Arg
   1865                1870                1875                1880
   CCC CGA GGC TAC CAT CAT CCC CAA GGA TTC TTG GAG GAC GAT GAC TCG     6198
   Pro Arg Gly Tyr His His Pro Gln Gly Phe Leu Glu Asp Asp Asp Ser
                   1885                1890                1895
   CCC GTT TGC TAT GAT TCA CGG AGA TCT CCA AGG AGA CGC CTA CTA CCT     6246
   Pro Val Cys Tyr Asp Ser Arg Arg Ser Pro Arg Arg Arg Leu Leu Pro
               1900                1905                1910
   CCC ACC CCA GCA TCC CAC CGG AGA TCC TCC TTC AAC TTT GAG TGC CTG     6294
   Pro Thr Pro Ala Ser His Arg Arg Ser Ser Phe Asn Phe Glu Cys Leu
           1915                1920                1925
   CGC CGG CAG AGC AGC CAG GAA GAG GTC CCG TCG TCT CCC ATC TTC CCC     6342
   Arg Arg Gln Ser Ser Gln Glu Glu Val Pro Ser Ser Pro Ile Phe Pro
       1930                1935                1940
   CAT CGC ACG GCC CTG CCT CTG CAT CTA ATG CAG CAA CAG ATC ATG GCA     6390
   His Arg Thr Ala Leu Pro Leu His Leu Met Gln Gln Gln Ile Met Ala
   1945                1950                1955                1960
   GTT GCC GGC CTA GAT TCA AGT AAA GCC CAG AAG TAC TCA CCG AGT CAC     6438
   Val Ala Gly Leu Asp Ser Ser Lys Ala Gln Lys Tyr Ser Pro Ser His
                   1965                1970                1975
   TCG ACC CGG TCG TGG GCC ACC CCT CCA GCA ACC CCT CCC TAC CGG GAC     6486
   Ser Thr Arg Ser Trp Ala Thr Pro Pro Ala Thr Pro Pro Tyr Arg Asp
               1980                1985                1990
   TGG ACA CCG TGC TAC ACC CCC CTG ATC CAA GTG GAG CAG TCA GAG GCC     6534
   Trp Thr Pro Cys Tyr Thr Pro Leu Ile Gln Val Glu Gln Ser Glu Ala
           1995                2000                2005
   CTG GAC CAG GTG AAC GGC AGC CTG CCG TCC CTG CAC CGC AGC TCC TGG     6582
   Leu Asp Gln Val Asn Gly Ser Leu Pro Ser Leu His Arg Ser Ser Trp
       2010                2015                2020
   TAC ACA GAC GAG CCC GAC ATC TCC TAC CGG ACT TTC ACA CCA GCC AGC     6630
   Tyr Thr Asp Glu Pro Asp Ile Ser Tyr Arg Thr Phe Thr Pro Ala Ser
   2025                2030                2035                2040
   CTG ACT GTC CCC AGC AGC TTC CGG AAC AAA AAC AGC GAC AAG CAG AGG     6678
   Leu Thr Val Pro Ser Ser Phe Arg Asn Lys Asn Ser Asp Lys Gln Arg
                   2045                2050                2055
   AGT GCG GAC AGC TTG GTG GAG GCA GTC CTG ATA TCC GAA GGC TTG GGA     6726
   Ser Ala Asp Ser Leu Val Glu Ala Val Leu Ile Ser Glu Gly Leu Gly
               2060                2065                2070
   CGC TAT GCA AGG GAC CCA AAA TTT GTG TCA GCA ACA AAA CAC GAA ATC     6774
   Arg Tyr Ala Arg Asp Pro Lys Phe Val Ser Ala Thr Lys His Glu Ile
           2075                2080                2085
   GCT GAT GCC TGT GAC CTC ACC ATC GAC GAG ATG GAG AGT GCA GCC AGC     6822
   Ala Asp Ala Cys Asp Leu Thr Ile Asp Glu Met Glu Ser Ala Ala Ser
       2090                2095                2100
   ACC CTG CTT AAT GGG AAC GTG CGT CCC CGA GCC AAC GGG GAT GTG GGC     6870
   Thr Leu Leu Asn Gly Asn Val Arg Pro Arg Ala Asn Gly Asp Val Gly
   2105                2110                2115                2120
   CCC CTC TCA CAC CGG CAG GAC TAT GAG CTA CAG GAC TTT GGT CCT GGC    6918
   Pro Leu Ser His Arg Gln Asp Tyr Glu Leu Gln Asp Phe Gly Pro Gly
                   2125                2130                2135
   TAC AGC GAC GAA GAG CCA GAC CCT GGG AGG GAT GAG GAG GAC CTG GCG    6966
   Tyr Ser Asp Glu Glu Pro Asp Pro Gly Arg Asp Glu Glu Asp Leu Ala
               2140                2145                2150
   GAT GAA ATG ATA TGC ATC ACC ACC TTG TAG CCCCCAGCGA GGGGCAGACT GGCT 7020
   Asp Glu Met Ile Cys Ile Thr Thr Leu  *
           2155                2160
   CTGGCCTCAG GTGGGGCGCA GGAGAGCCAG GGGAAAAGTG CCTCATAGTT AGGAAAGTTT    7080
   AGGCACTAGT TGGGAGTAAT ATTCAATTAA TTAGACTTTT GTATAAGAGA TGTCATGCCT    7140
   CAAGAAAGCC ATAAACCTGG TAGGAACAGG TCCCAAGCGG TTGAGCCTGG CAGAGTACCA    7200
   TGCGCTCGGC CCCAGCTGCA GGAAACAGCA GGCCCCGCCC TCTCACAGAG GATGGGTGAG    7260
   GAGGCCAGAC CTGCCCTGCC CCATTGTCCA GATGGGCACT GCTGTGGAGT CTGCTTCTCC    7320
   CATGTACCAG GGCACCAGGC CCACCCAACT GAAGGCATGG CGGCGGGGTG CAGGGGAAAG    7380
   TTAAAGGTGA TGACGATCAT CACACCTGTG TCGTTACCTC AGCCATCGGT CTAGCATATC    7440
   AGTCACTGGG CCCAACATAT CCATTTTTAA ACCCTTTCCC CCAAATACAC TGCGTCCTGG    7500
   TTCCTGTTTA GCTGTTCTGA AATACGGTGT GTAAGTAAGT CAGAACCCAG CTACCAGTGA    7560
   TTATTGCGAG GGCAATGGGA CCTCATAAAT AAGGTTTTCT GTGATGTGAC GCCAGTTTAC    7620
   ATAAGAGAAT ATCAC                                                     7635

## Claims

1. An isolated nucleic acid fragment that encodes an α₁-subunit of a human calcium channel having an amino acid sequence set forth in SEQ ID No. 7 or SEQ ID No. 8.

2. The nucleic acid fragment of claim 1 having the sequence of nucleotides set forth in SEQ ID No. 7 or SEQ ID No. 8.

3. The nucleic acid fragment of claim 1 or 2, wherein the sequence of nucleotides comprises nucleotides 144 to 7160 set forth in SEQ ID Neo. 7.

4. The nucleic acid fragment of claim 1 or 2, wherein the sequence of nucleotides comprises nucleotides 144 to 6854 set forth in SEQ ID No. 8.

5. A eukaryotic cell, comprising heterologous nucleic acid, wherein the heterologous nucleic acid comprises at least one of the nucleic acid fragments as claimed in any preceding claim.

6. The eukaryotic cell of claim 5, wherein at least one nucleic acid fragment encodes an α_{1B}-type α₁₋ subunit of a human calcium channel

7. The eukaryotic cell of claim 5 which has a functional heterologous calcium channel that contains at least one subunit encoded by the nucleic acid fragments of claims 1, 2 or 3.

8. The eukaryotic cell of claims 5, 6 or 7 selected from HEK 293 cells, Chinese hamster ovary cells, African green monkey cells, and mouse L cells.

9. A eukaryotic cell with a functional, heterologous calcium channel, produced by a process comprising introducing into a cell at least one RNA transcript which is encoded by a nucleic acidfragment as claimed in any one of claims 1, 2 or 3 , wherein the heterologous calcium channel thus produced includes at least one human calcium channel subunit encoded by the introduced RNA.

10. The eukaryotic cell of claim 9 which is an amphibian oöcyte.

11. A method for identifying a compound that modulates the activity of a calcium channel, comprising;
suspending a eukaryotic cell of any of claims 9 to 10 which has a functional, heterologous calcium channel, in a solution containing said compound and a calcium channel selective ion:
depolarizing the cell membrane of said cell; and
detecting the current flowing into said cell,
wherein:
the current that is detected is different from that produced by depolarizing the same or a substantially identical cell in the presence of the same calcium channel selective ion but in the absence of said compound.

12. The method of claim 11, wherein prior to the depolarization step the cell is maintained at a holding potential which substantially inactivates calcium channels which are endogenous to said cell.

13. The method of claim 11, wherein the cell is an HEK cell and the subunits of the heterologous calcium channel are encoded by heterologous DNA.

14. A substantially pure α₁-subunit of a human calcium channel encoded by the nucleic acid of claim 1.

15. The nucleic acid fragment of claim 1, wherein the human calcium channel is a human neural calcium channel, a human skeletal muscle calcium channel or a human aortic calcium channel.

## Patentansprüche

1. Isoliertes Nukleinsäurefragment, welches für eine α₁-Untereinheit eines humanen Calciumkanals mit einer in SEQ-ID-Nr. 7 oder SEQ-ID-Nr. 8 angegebenen Aminosäuresequenz kodiert.

2. Nukleinsäurefragment nach Anspruch 1 mit der in SEQ-ID-Nr. 7 oder SEQ-ID-Nr. 8 angegebenen Sequenz von Nukleotiden.

3. Nukleinsäurefragment nach Anspruch 1 oder 2, wobei die Sequenz von Nukleotiden die in SEQ-ID-Nr. 7 angegebenen Nukleotide 144 bis 7160 umfaßt.

4. Nukleinsäurefragment nach Anspruch 1 oder 2, wobei die Sequenz von Nukleotiden die in SEQ-ID-Nr. 8 angegebenen Nukleotide 144 bis 6854 umfaßt.

5. Eukaryotische Zelle, umfassend eine heterologe Nukleinsäure, wobei die heterologe Nukleinsäure mindestens eines der Nukleinsäurefragmente nach irgendeinem der vorherigen Ansprüche umfaßt.

6. Eukaryotische Zelle nach Anspruch 5, wobei mindestens ein Nukleinsäurefragment für eine α₁-Untereinheit vom α_{1B}-Typ eines humanen Calciumkanals kodiert.

7. Eukaryotische Zelle nach Anspruch 5, welche einen funktionellen heterologen Calciumkanal aufweist, der mindestens eine Untereinheit enthält, die von den Nukleinsäurefragmenten der Ansprüche 1, 2 oder 3 kodiert wird.

8. Eukaryotische Zelle nach den Ansprüchen 5, 6 oder 7, ausgewählt aus HEK 293-Zeilen, Eierstockzellen des Chinesischen Hamsters, Zellen der Afrikanischen Grünen Meerkatze und Maus-L-Zellen.

9. Eukaryotische Zelle mit einem funktionellen heterologen Calciumkanal, hergestellt durch ein Verfahren, welches das Einführen mindestens eines RNA-Transkripts, das von einem Nukleinsäurefragment nach irgendeinem der Ansprüche 1, 2 oder 3 kodiert wird, in eine Zelle umfaßt, wobei der so hergestellte heterologe Calciumkanal mindestens eine humane Calciumkanal-Untereinheit einschließt, die von der eingeführten RNA kodiert wird.

10. Eukaryotische Zelle nach Anspruch 9, welche ein Amphibien-Oozyt ist.

11. Verfahren zur Identifizierung einer Verbindung, welche die Aktivität eines Calciumkanals moduliert, umfassend:
Suspendieren einer eukaryotischen Zelle nach irgendeinem der Ansprüche 9 bis 10, welche einen funktionellen heterologen Calciumkanal aufweist, in einer Lösung, welche die Verbindung und ein calciumkanal-selektives lon enthält;
Depolarisieren der Zellmembran der Zelle; und
Nachweisen des in die Zelle fließenden Stroms, wobei
der nachgewiesene Strom sich von demjenigen unterscheidet, welcher durch Depolarisieren derselben oder einer im wesentlichen identischen Zelle in Anwesenheit desselben calciumkanal-selektiven lons, jedoch in Abwesenheit der Verbindung, erzeugt wird.

12. Verfahren nach Anspruch 11, wobei vor dem Depolarisierungsschritt die Zelle bei einem Haltepotential gehalten wird, welches im wesentlichen Calciumkanäle inaktiviert, die für die Zelle endogen sind.

13. Verfahren nach Anspruch 11, wobei die Zelle eine HEK-Zelle ist und die Untereinheiten des heterologen Calciumkanals von heterologer DNA kodiert werden.

14. Im wesentlichen reine α₁-Untereinheit eines humanen Calciumkanals, kodiert von der Nukleinsäure nach Anspruch 1.

15. Nukleinsäurefragment nach Anspruch 1, wobei der humane Calciumkanal ein humaner Nerven-Calciumkanal, ein humaner Skelettmuskel-Calciumkanal oder ein humaner Aorta-Calciumkanal ist.

## Revendications

1. Fragment d'acide nucléique isolé qui code pour une sous-unité α₁ d'un canal calcique humain ayant une séquence d'acides aminés définie dans SEQ ID N°7 ou SEQ ID N°8.

2. Fragment d'acide nucléique selon la revendication 1 ayant la séquence de nucléotides définie dans SEQ ID N°7 ou SEQ ID N°8.

3. Fragment d'acide nucléique selon la revendication 1 ou 2, dans lequel la séquence de nucléotides comprend les nucléotides 144 à 7160 définis dans SEQ ID N°7.

4. Fragment d'acide nucléique selon la revendication 1 ou 2, dans lequel la séquence de nucléotides comprend les nucléotides 144 à 6854 définis dans SEQ ID N°8.

5. Cellule eucaryote, comprenant un acide nucléique hétérologue, dans laquelle l'acide nucléique hétérologue comprend au moins un des fragments d'acide nucléique selon l'une quelconque des revendications précédentes.

6. Cellule eucaryote selon la revendication 5, dans laquelle au moins un fragment d'acide nucléique code pour une sous-unité α₁ de type α_{1B} d' un canal calcique humain.

7. Cellule eucaryote selon la revendication 5, qui a un canal calcique hétérologue fonctionnel qui contient au moins une sous-unité codée par les fragments d'acide nucléique selon les revendications 1, 2 ou 3.

8. Cellule eucaryote selon les revendications 5, 6 ou 7 choisie parmi les cellules HEK 293, les cellules ovariennes de hamster chinois, les cellules du singe vert d'Afrique et les cellules L de souris.

9. Cellule eucaryote avec un canal calcique hétérologue fonctionnel, produite selon un procédé comprenant l'introduction dans une cellule d'au moins un ARN transcrit qui est codé par un fragment d'acide nucléique selon l'une quelconque des revendications 1,2 ou 3, dans laquelle le canal calcique hétérologue ainsi produit comprend au moins une sous-unité de canal calcique humain codée par l'ARN introduit.

10. Cellule eucaryote selon la revendication 9 qui est un ovocyte amphibie.

11. Procédé d'identification d'un composé qui module l'activité d'un canal calcique, comprenant :
■ la suspension d'une cellule eucaryote selon les revendications 9 ou 10 qui a un canal calcique hétérologue fonctionnel, dans une solution contenant ledit composé et un ion sélectif du canal calcique ;
■ la dépolarisation de la membrane cellulaire de ladite cellule ; et
■ la détection du courant parcourant ladite cellule,
dans lequel:
■ le courant détecté est différent du courant produit par la dépolarisation de la même cellule ou d'une cellule essentiellement identique en présence du même ion sélectif du canal calcique mais en l'absence dudit composé.

12. Procédé selon la revendication 11, dans lequel avant l'étape de dépolarisation, la cellule a été maintenue à un potentiel de maintien, qui inactive substantiellement les canaux calciques endogènes à ladite cellule.

13. Procédé selon la revendication 11, dans lequel la cellule est une cellule HEK et les sous-unités du canal calcique hétérologue sont codées par l'ADN hétérologue.

14. Sous-unité α₁ essentiellement pure d'un canal calcique humain codée par l'acide nucléique selon la revendication 1.

15. Fragment d'acide nucléique selon la revendication 1, dans lequel le canal calcique humain est un canal calcique neural humain, un canal calcique du muscle squelettique ou un canal calcique de l'aorte humaine.
